# EUROPEAN PATENT APPLICATION

(11) **EP 4 631 959 A2**
(43) Date of publication of application: **15.10.2025**
(21) Application number: 25193071.5
(22) Date of filing: 19.05.2023
(51) Int. Cl.: C07K 5/117

(54) **NOVEL MINIGASTRIN-DERIVED CHOLECYSTOKININ 2 RECEPTOR BINDING MOLECULES FOR IMAGING AND TARGETED RADIOTHERAPY**

(30) Priority: 20.09.2022 EP 22196630
(62) Divisional of application: 23727562.3
(71) Applicant: Technische Universität München, in Vertretung des Freistaates Bayern, 80333 München (DE); Universität Augsburg, in Vertretung des Freistaates Bayern, 86159 Augsburg (DE)
(72) Inventor: GÜNTHER, Thomas, 87656 Germaringen (DE); HOLZLEITNER, Nadine, 80992 Munich (DE); WESTER, Hans-Jürgen, 85301 Schweitenkirchen (DE); LAPA, Constantin, 60314 Frankfurt am Main (DE)
(74) Representative: Vossius & Partner Patentanwälte Rechtsanwälte mbB

(57) **Abstract**

The present invention relates to a conjugate compound binding to Cholecystokinin 2 receptor (CCK-2R) that is represented by formula (IV)

R^{L}-dap(R^{SiFA})-R^{GABA}-L-R^{H} (IV)

wherein:
R^{L}
is DOTA or DOTAGA, optionally containing a chelated nonradioactive or radioactive cation; dap(R^{SiFA}) is a diaminopropionic acid (dap) group carrying a silicon-based fluoride acceptor moiety R^{SiFA};
R^{GABA}
is present or absent, and, if present, is one or more y-aminobutryic acid (GABA), preferably (GABA)₁₋₃;
L
is a linking moiety comprising at least 5, preferably at least 6 α- or γ-glu and most preferably 6 to 9 α- or γ-glu, whereby γ-glu is preferred over α-glu; and
R^{H}
is or comprises Ala-Tyr-Gly-Trp-Nle-Asp-Phe-NH₂

## Description

The present invention relates to a tetrapeptide binding to Cholecystokinin 2 receptor (CCK-2R), wherein the tetrapeptide is represented by formula (I) Xaa1-Xaa2-Xaa3-Xaa4 (formula (I)) or salt thereof, wherein Xaa1 is Trp, (β-(3-benzothienyl)-alanine, Trp, wherein one or more of the H atoms of the 1*H-*indol-3-yl are replaced by a substituent that is for each H atom independently selected from a C₁ to C₃ alkyl, -OH, -SH, -F, and -Cl, Phe, 1-Nal, 2-Nal, Tyr or p-Amino-Phe, Xaa2 is an N-methyl amino acid, wherein the N-methyl group is at the alpha carbon, and preferably an N-methyl amino acid containing an aliphatic side chain, wherein the N-methyl group is at the alpha carbon, Xaa3 is an amino acid with an acidic side chain at neutral pH, and Xaa4 is an amino acid with an aromatic side chain or an amide thereof.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Medullary thyroid cancer (MTC), a neuroendocrine neoplasm, is reported to account for 1-2% of all thyroid cancers in the United States [1]. In general, thyroid cancer constitutes for approximately 3% of all new cancer cases in the United States, which is why the occurrence of MTC is considered as rare [2]. Despite improvement in detection of MTC via different screening methods and biopsy technologies, mean survival time remains modest (8.6 ± 7.6 years), especially when metastasis has already started [3-7].

Among MTC patients, approximately 70% and 30% suffer from the sporadic and hereditary/familial type, respectively [8]. For the diagnosis of hereditary MTC, detection of receptor tyrosine kinase (RET) proto-oncogene mutations via molecular analysis of the DNA is applied. By this, patients at risk for developing MTC can be regularly examined, which enables an early detection of MTC lesions [9,10].

Diagnosis of sporadic MTC is usually made after fine needle aspiration. Given its neuroendocrine origin, production of calcitonin is a characteristic feature of this tumor. Injection of calcium or the synthetic gastrin analogue pentagastrin stimulates the secretion of calcitonin, especially from MTC cells [9,11]. Thus, rising calcitonin levels are a strong indicator for MTC. However, biochemical testing only indicates the presence of a tumor/metastasis, whereas the localization is often impossible [12]. As a supplement to these screening methods, better and earlier localization of tumors and metastases can be achieved via molecular imaging using positron emission tomography/computed tomography (PET/CT) [13,14].

The recently published EANM practice guidelines for MTC imaging recommend ⁶⁸Ga-labeled somatostatin analogues, [¹⁸F]fluorodeoxyglucose ([¹⁸F]FDG) and [¹⁸F]fluorodihydroxyphenylalanine ([¹⁸F]F-DOPA) [15]. In a retrospective analysis, [¹⁸F]F-DOPA PET/CT displayed enhanced detection rates (72%) in patients with recurrent MTC compared to [¹⁸F]FDG PET/CT (17%) or PET/CT performed with ⁶⁸Ga-labeled somatostatin analogues (33%) [16]. Interestingly, [¹⁸F]F-DOPA PET reveals good sensitivity in primary tumors (86%) but only moderate (57%) to low (6%) sensitivity in lymph node (LN) and distant metastases, respectively [17]. Moreover, as reported in a recent meta-analysis of the literature, the per-patient detection rate of [¹⁸F]F-DOPA PET or PET/CT is only 66% (95% confidence interval, 58-74%) in patients with suspected recurrent MTC [18].

For patients with metastatic disease, treatment options are still limited. Neither external beam radiation, nor conventional chemotherapy have shown sufficient therapeutic efficiency and are therefore not recommended [1,19,20]. Unlike common forms of thyroid cancer, MTC originates from malignant de-differentiation of parafollicular cells. As these cells do not accumulate iodine, radioactive iodine therapy is not a treatment option [21,22]. For this reason, total thyroidectomy and central compartment neck dissection are still performed most frequently in case of MTC [1,23]. However in general, 10-year survival rate for patients with local and distant metastases was 76% and 40%, respectively [6]. Tyrosine kinase inhibitors such as selpercatinib, vandetanib or cabozantinib have been shown to prolong (mainly progression-free) survival [1]. However, therapy is associated with a variety of relevant side effects such as hypertension, renal toxicity and hepatotoxicity, amongst others [1,24].

Over the last decades, the field of nuclear medicine emerged as a powerful alternative due to its non-invasive imaging and therapy opportunities [13,14]. As a supplement to the screening methods mentioned earlier, localization of tumors and metastases could be thus determined via molecular imaging. In the 1990s, diagnosis of MTC was first accomplished via somatostatin receptor scintigraphy (SRS), applying Somatostatin receptor subtype 2 (sstr2)-specific ligands. Although decent improvement in diagnosis, lots of MTC-negative scans were still reported, especially in patients with metastatic spreading [25]. For this reason, new options for MTC targeting, particularly for progressed disease and metastasis were warranted.

As was reported by *Reubi et al.* in 1997, MTC overexpresses cholecystokinin receptors A (CCK-A) and B (CCK-B), with the latter also known as gastrin receptor or CCK-2 receptor (CCK-2R) [26]. In 2006, *Gotthardt et al.* showed that CCK-2R expression is extensively higher in MTC-derived metastases than sstr2 expression (94% to 40%, respectively) [27]. Ever since, the development of CCK-2R-specific tracers that are based on the endogenous ligands CCK or minigastrin, increased [28,29].

In view of the above there is an ongoing need for further options to diagnose and treat CCK-2R-expressing cancer, such as MTC. This need is addressed by the present invention.

Accordingly, the present invention relates in a first aspect to a tetrapeptide binding to Cholecystokinin 2 receptor (CCK-2R), wherein the tetrapeptide is represented by formula (I) Xaa1-Xaa2-Xaa3-Xaa4 (formula (I)) or salt thereof, wherein Xaa1 is Trp, (β-(3-benzothienyl)-alanine, Trp, wherein one or more of the H atoms of the 1*H*-indol-3-yl are replaced by a substituent that is for each H atom independently selected from a C₁ to C₃ alkyl, -OH, -SH, -F, and -Cl, Phe, 1-Nal, 2-Nal, Tyr or p-Amino-Phe, Xaa2 is an N-methyl amino acid, wherein the N-methyl group is at the alpha carbon, and preferably an N-methyl amino acid containing an aliphatic side chain, wherein the N-methyl group is at the alpha carbon, Xaa3 is an amino acid with an acidic side chain at neutral pH, and Xaa4 is an amino acid with an aromatic side chain or an amide thereof.

The term "tetrapeptide" as used herein relates to a peptide consisting of 4 amino acids that are covalently connected via peptide bonds.

The salt of the tetrapeptide is preferably a pharmaceutically acceptable salt.

The first amino acid is designated Xaa1 and can be Trp, (β-(3-benzothienyl)-alanine, Trp, wherein one or more of the H atoms of the 1*H*-indol-3-yl are replaced by a substituent that is for each H atom independently selected from a C₁ to C₃ alkyl, -OH, -SH, -F, and -Cl, Phe, 1-Nal, 2-Nal, Tyr or p-Amino-Phe. The one or more of the H atoms are preferably 1 or 2 H atoms and is preferably 1 H atom. Among the substituents a C₁ to C₃ alkyl is preferred and the C₁ to C₃ alkyl is preferably ethyl or methyl and most preferably methyl.

The second amino acid is designated Xaa2 and can be an N-methyl amino acid, wherein the N-methyl group is at the alpha carbon, and preferably an N-methyl amino acid containing an aliphatic side chain, wherein the N-methyl group is at the alpha carbon. The N-methyl amino acid is preferably derived from the 22 proteinaceous amino acids or proteinogenic amino acids (Alanine, Arginine, Asparagine, Aspartic Acid, Cysteine, Glutamic acid, Glutamine, Glycine, Histidine, Isoleucine, Leucine, Lysine, Methionine, Phenylalanine, Proline, Serine, Threonine, Tryptophan, Tyrosine, Valine, Selenocysteine and Pyrrolysine (noting that Pyrrolysine is not used in human protein synthesis)). Based on these proteinaceous amino acids or proteinogenic amino acids an N-methyl amino acid, wherein the N-methyl group is at the alpha carbon, can be obtained by replacing the NH₂ group at the alpha C atom by a N-methyl group. Among the 22 proteinaceous amino acids or proteinogenic amino acids alanine, isoleucine, leucine, proline, valine and methionine have an aliphatic side chain. Amino acids having an aliphatic side chain are also referred to herein as aliphatic amino acids. Aliphatic amino acids are nonpolar and hydrophobic.

In organic chemistry, the alpha carbon (Cα) refers to the first carbon atom that is attached to a functional group - such as a COOH group in the case of the 22 proteinaceous amino acids or proteinogenic amino acids. Hence, such amino acids are also called alpha-amino acids. The second carbon atom is called the beta carbon (Cβ) and the naming system continues in Greek alphabetical order.

The third amino acid is designated Xaa3 and can be an amino acid with an acidic side chain at neutral pH. The amino acid Xaa3 is preferably an alpha-amino acid. Among the 22 proteinaceous amino acids or proteinogenic amino acids two amino acids have acidic side chains at neutral pH, aspartate (Asp) and glutamic acid or glutamate (Glu).

The fourth amino acid is designated Xaa4 and can be an amino acid with an aromatic side chain or an amide thereof. Among the 22 proteinaceous amino acids or proteinogenic amino acids phenylalanine, tryptophan and tyrosine are amino acids with an aromatic side chain. Although histidine contains an aromatic ring, its basic properties cause it to be predominantly classified in the art and also herein as a polar amino acid or an amino acid containing an aliphatic side chain. In the case of amide of an amino acid with an aromatic side chain the normal C-terminus of the tetrapeptide is -NH₂. This amide is also referred to herein as Xaa4-NH2. The C-terminus of the tetrapeptide Xaa1-Xaa2-Xaa3-Xaa4 (formula (I)) is preferably an amide. In this case the tetrapeptide can be represented as Xaa1-Xaa2-Xaa3-Xaa4-NH2.

The tetrapeptide of the invention binds to Cholecystokinin 2 receptor (CCK-2R). CCK-2R is also known as cholecystokinin B receptor. The protein is a G protein-coupled receptor for gastrin and cholecystokinin (CCK), regulatory peptides of the brain and gastrointestinal tract. This protein is a type B gastrin receptor, which has a high affinity for both sulfated and nonsulfated CCK analogs and is found principally in the central nervous system and the gastrointestinal tract. A misspliced transcript variant including an intron has been observed in cells from colorectal, pancreatic tumors and thyroid cancer (in particular medullary thyroid cancer (MTC)).

The tetrapeptide preferably specifically binds to CCK-2R. Specific binding designates that the binding molecule essentially does not or does not bind to other proteins or peptides than CCK-2R. In particular, it is preferred that the binding molecule is not capable to bind to other cholecystokinin receptors than CCK-2R. The tetrapeptide only is, for example, suitable for research purposes. For example, the tetrapeptide can be used in immunoassays to capture CCK-2R, such as an ELISA or Western Blot. Immunoassays are biochemical tests that can measure the presence or concentration of the CCK-2R in a sample (e.g. a solution). As will be further explained herein below, the tetrapeptide is particularly suitable for medical and diagnostic applications for disease being associated with the expression of CCK-2R.

As can be taken from the appended examples the tetrapeptide of the first aspect of the invention is identical to or closely resembles the four N-terminal amino acids of the prior art CCK-R2 binder minigastrin with seven amino acids, i.e. Trp-(N-Me)Nle-Asp-1-Nal motif. The finding that only the four N-terminal amino acids *H*-Trp-(*N*-Me)Nle-Asp-1-Nal-NH₂ of minigastrin are necessary for high CCK-2R affinity was surprising and advantageously results in a smaller binder of CCK-R2. It is of note that a number of prior art CCK-R2 binders, including F11N consist of seven amino acids. The four N-terminal amino acids of F11N are closely related to *H*-Trp-Nle-Asp-Phe-NH₂ but did not show high CCK-2R affinity. It is therefore believed that in particular the (N-Me) amino acid at position Xaa2 in the tetrapeptide is important to ensure CCK-R2 binding. To the best knowledge of the inventors no peptidic high-affinity CCK-2R ligand that consists of only four amino acids is known from the prior art. It is furthermore shown in the appended examples that high CCK-2R affinity of the tetrapeptide is retained when it is connected to a linker moiety, such as a PEG chain, together with a chelator capable of complexing a radioactive or nonradioactive cation. For this reason, the tetrapeptide can not only be used alone but can also be a key element in the design of further CCK-2R binding compounds that are of diagnostic or medical value.

In accordance with a preferred embodiment of the first aspect of the invention Xaa1 is Trp.

It is revealed by the appended examples that Trp at position Xaa1 shows the highest binding affinity to CCK-2R. β-(3-benzothienyl)-alanine and Trp, wherein one or more of the H atoms of the 1*H*-indol-3-yl are replaced by a substituent that is for each H atom independently selected from a C₁ to C₃ alkyl, -OH, -SH, -F, and -CI closely resembles Trp and are therefore also supposed to work.

Also in accordance with a preferred embodiment of the first aspect of the invention Xaa2 is (N-Me)Nle, (N-Me)Met, (N-Me)lle, (N-Me)Leu, (N-Me)Val, (N-Me)Gly, (N-Me)Ala, (N-Me)Glu, is preferably (N-Me)Nle or (N-Me)Met and is most preferably (N-Me)Nle.

The appended examples revealed by the tests with tetrapeptides being identical to or closely resembling the four N-terminal amino acids of minigastrin that (N-Me)Nle at position Xaa2 shows the highest binding affinity to CCK-2R. Since (N-Me)lle, (N-Me)Leu, (N-Me)Val, (N-Me)Gly, (N-Me)Ala, (N-Me)Glu resemble and (N-Me)Met closely resembles (N-Me)Nle it is believed that in particular these alternatives can be used instead of (N-Me)Nle.

In accordance with a further preferred embodiment of the first aspect of the invention Xaa3 is Asp, isoaspartate, Glu or isoglutamine and is preferably Asp.

The appended examples furthermore revealed by tests with various tetrapeptides being identical to or closely resembling the four N-terminal amino acids of minigastrin that Asp at position Xaa3 shows the highest binding affinity to CCK-2R. Since isoaspartate, Glu or isoglutamine closely resemble Asp it is believed that in particular these alternatives can be used instead of Asp. Among the alternatives Glu is preferred since it is a proteinaceous amino acid or proteinogenic amino acid.

In accordance with a yet further preferred embodiment of the first aspect of the invention Xaa4 is 1-Nal, Phe, Trp, Tyr, 2-Nal or Amino-Phe, is preferably 1-Nal, Phe, Tyr or an amide of any one of the foregoing, and is most preferably 1-Nal or Tyr or an amide of 1-Nal or Tyr.

The appended examples also revealed by the tests with the tetrapeptides being identical to or closely resembling the four N-terminal amino acids of minigastrin that 1-Nal or Tyr at position Xaa4 shows the highest binding affinity to CCK-2R. Since Phe, Trp, 2-Nal and Amino-Phe closely resemble 1-Nal or Tyr it is believed that in particular these alternatives can be used instead of 1-Nal or Tyr.

1-Nal, Phe, Trp, Tyr, or 2-Nal and *para*-Amino-Phe as well as the amides thereof are all amino acids wherein with the side chain an aromatic ring can be found with H atoms as substituents. Hence, Xaa4 can also be a derivative of 1-Nal, Phe, Trp, Tyr, or 2-Nal, p-Amino-Phe or an amide thereof, wherein one or more of the H atoms of the aromatic ring in the side chain are replaced by a substituent independently selected for each H atom from C₁ to C₃ alkyl, -OH, -SH, -F, and -Cl. The one or more of the H atoms are preferably 1 or 2 H atoms and is preferably 1 H atom. Among the substituents a C₁ to C₃ alkyl is preferred and the C₁ to C₃ alkyl is preferably ethyl or methyl and most preferably methyl.

In accordance with another preferred embodiment of the first aspect of the invention the tetrapeptide has a CCK-2R binding affinity depicted as half-maximal inhibitory concentration (IC₅₀) of 100 nM or less, preferably 20 nM or less and most preferably 7.5 nM or less.

It is shown in Table 2 of the appended examples that the tetrapeptide being identical to the four N-terminal amino acids of minigastrin displays an IC₅₀ of 6.4 ± 1.0 nM for CCK-2R. The tetrapeptides *H-*Trp-(*N*-Me)Nle-Asp-Phe-NH₂ and *H*-Trp-(N-Me)Nle-Asp-Tyr-NH₂ with an IC₅₀ of 4.5 ± 0.6 nM and 5.7 ± 0.5 nM for CCK-2R were even slightly better. The tetrapeptide *H*-Trp-(*N*-Me)Nle-Asp-Trp-NH₂ still maintained an IC₅₀ of 18.8 ± 0.5 nM and the tetrapeptide *H*-Trp-(*N*-Me)Nle-Asp-2-Nal-NH₂ an IC₅₀ of 102 ± 9 nM.

These particular tested peptides fall under the ambit of the above preferred embodiment and show that the IC₅₀ values of this preferred embodiment are technically supported by exemplified tetrapeptides.

The present invention relates in a second aspect to a conjugate comprising within a single molecule: (a) the tetrapeptide of the first aspect of the invention, and (b) one or more chelating groups capable of complexing a nonradioactive or radioactive cation or one or more chelating groups containing a chelated nonradioactive or radioactive cation.

The one or more chelating groups is preferably one or two chelating groups and most preferably one chelating group.

A chelating group capable of complexing a nonradioactive or radioactive cation refers to the state wherein the chelating group is not complexed with a nonradioactive or radioactive cation and can still be complexed with a nonradioactive or radioactive cation. In this case a nonradioactive or radioactive cation is not a part of the conjugate of the second aspect of the invention.

On the other hand, in a chelating group containing a chelated nonradioactive or radioactive cation a nonradioactive or radioactive cation is complexed with the chelating group, so that the nonradioactive or radioactive cation is a part of the conjugate of the second aspect of the invention.

Diverse chelating agents from which suitable chelating groups can be derived are well known in the art and can be used in the context of the present invention. Metal- or cation-chelating agents, e.g. macrocyclic or acyclic compounds, which are suitable to act as a chelating group, are available from a number of manufacturers. It will be understood that numerous chelating agents can be used in an off-the-shelf manner by a skilled person without further ado. It will further be understood that the suitability of the chelating group to form a chelate with a given cation requires the chelating group to be able to provide a chelated ligand in a chelate complex comprising the cation under consideration, but does not require the chelating group to provide the only ligand of the cation in the chelate complex. Thus, if the chelating group contains a chelated radioactive or nonradioactive cation, the cation may be a complex cation, e.g. a metal ion carrying an additional coordinated ligand other than the chelating group, such as an oxo ligand.

For example, the chelating group may comprise at least one of (i) a macrocyclic ring structure with 8 to 20 ring atoms of which 2 or more, preferably 3 or more, are selected from oxygen atoms and nitrogen atoms; and (ii) an acyclic, open chain chelating structure with 8 to 20 main chain atoms of which 2 or more, preferably 3 or more are heteroatoms selected from oxygen atoms and nitrogen atoms.

In accordance with a preferred embodiment of the second aspect of the invention, the chelating group is a group which can be derived from a chelating agent selected from bis(carboxymethyl)-1,4,8,11-tetraazabicyclo[6.6.2]hexadecane (CBTE2a), cyclohexyl-1,2-diaminetetraacetic acid (CDTA), 4-(1,4,8,11-tetraazacyclotetradec-1-yl)-methylbenzoic acid (CPTA), N'-[5-[acetyl(hydroxy)amino]pentyl]-N-[5-[[4-[5-aminopentyl-(hydroxy)amino]-4-oxobutanoyl]amino]pentyl]-N-hydroxybutandiamide (DFO), 4,11-bis(carboxymethyl)-1,4,8,11-tetraazabicyclo[6.6.2]hexadecan (DO2A), 1,4,7,10-tetraazacyclododecan-N,N',N",N‴-tetraacetic acid (DOTA), 2-[1,4,7,10-tetraazacyclododecane-4,7,10-triacetic acid]-pentanedioic acid (DOTAGA or DOTA-GA), N,N'-dipyridoxylethylendiamine-N,N'-diacetate-5,5'-bis(phosphat) (DPDP), diethylenetriaminepentaacetic acid (DTPA), ethylenediamine-N,N'-tetraacetic acid (EDTA), ethyleneglykol-O,O-bis(2-aminoethyl)-N,N,N',N'-tetraacetic acid (EGTA), N,N-bis(hydroxybenzyl)-ethylenediamine-N,N'-diacetic acid (HBED), hydroxyethyldiaminetriacetic acid (HEDTA), 1-(p-nitrobenzyl)-1,4,7,10-tetraazacyclodecan-4,7,10-triacetate (HP-DOA3), 6-hydrazinyl-N-methylpyridine-3-carboxamide (HYNIC), 1,4,7-triazacyclononan-1-succinic acid-4,7-diacetic acid (NODASA), 1-(1-carboxy-3-carboxypropyl)-4,7-(carboxy)-1,4,7-triazacyclononane (NODAGA), 1,4,7-triazacyclononanetriacetic acid (NOTA), 4,11-bis(carboxymethyl)-1,4,8,11-tetraaza-bicyclo[6.6.2]hexadecane (TE2A), 1,4,8,11-tetraazacyclododecane-1,4,8,11-tetraacetic acid (TETA), terpyridine-bis(methyleneamine) tetraacetic acid (TMT), 1,4,7,10-tetraazacyclotridecan-N,N',N",N‴-tetraacetic acid (TRITA), and triethylenetetraaminehexaacetic acid (TTHA), *N,N'*-bis[(6-carboxy-2-pyridin)methyl]-4,13-diaza-18-crown-6 (H₂macropa), 4-amino-4-{2-[(3-hydroxy-1,6-dimethyl-4-oxo-1,4-dihydro-pyridin-2-ylmethyl)-carbamoyl]-ethyl} heptanedioic acid bis-[(3-hydroxy-1,6-dimethyl-4-oxo-1,4-dihydro-pyridin- 2-ylmethyl)-amide] (THP), 1,4,7-triazacyclononane-1,4,7-tris[methylene(2-carboxyethyl)phosphinic acid (TRAP), 2-(4,7,10-tris(2-amino-2-oxoethyl)-1,4,7,10-tetraazacyclododecan-1-yl)acetic acid (DO3AM), and 1,4,7,10-tetraazacyclododecane-1,4,7, 10-tetrakis[methylene(2-carboxyethylphosphinic acid)] (DOTPI), S-2-(4-isothiocyanatobenzyl)-1,4,7,10-tetraazacyclododecane tetraacetic acid, mercaptoacetyl-triserine (mas₃), hydrazinonicotinic acid (HYNIC) and the N4 chelator. The chelating group is typically provided using a functional group of one of the chelating agents referred to above, e.g. a carboxyl group, to form a bond, e.g. an amide bond, with the remainder of the conjugate which contains the chelating group.

More preferred are DOTA, DOTAGA, DOTAM, DO3AM, NOTA and NODAGA.

As will be understood by the skilled reader, a chelating group in a conjugate of the invention can be conveniently derived from the chelating agents listed above by either using a functional group contained in the chelating agent, such as a carboxylic acid group, an amide group, an amino group, a hydroxy group, or a thiol function to provide a coupling group, e.g. selected from -C(O)-, -NH-, -S- and -O-, which attaches the chelating group to the remainder of the compound. Preferably, a carboxylic acid group is used to provide a coupling group -C(O)- (a carbonyl) to form an amide bond. The coupling group may be covalently linked to a further, complementary coupling group comprised in conjugates, such as the tetrapeptide itself, so that the two coupling groups combine to form a binding unit, such as an amide bond -C(O)-NH-.

Alternatively, as will be understood by the skilled reader, a chelating group in a conjugate of the invention can be conveniently derived from the chelating agents listed above by the introduction of an additional functional group or the introduction of additional groups having a functional group able to form a chemical bond to the other parts of the conjugate, preferably the tetrapeptide or a linker between the chelating group and the tetrapeptide, such as a chelator modified with an additional residue with an isothiocyanate that can link to an amine. As will be understood by the skilled reader, other conjugation strategies, typically summarized as "bioconjugation strategies" can also be used to link a chelating group in a conjugate of the invention.

In accordance with a further preferred embodiment of the second aspect of the invention the chelating group is a group, which can be derived from DOTA or DOTAGA with or without a chelated nonradioactive or radioactive cation, wherein DOTA and DOTAGA are preferably bound with one of its carboxylic groups via an amide bond to the remainder of the conjugate.

The amine can either be an amine within the tetrapeptide (e.g. the N-terminal amine group or an amine in the side chain of an amino acid) or an amine within a linker linking the chelating group and the tetrapeptide.

In accordance with another preferred embodiment of the second aspect of the invention the radioactive or nonradioactive cation is selected from the cations of ⁴³Sc, ⁴⁴Sc, ⁴⁷Sc, ⁵¹Cr, ^{52m}Mn, ⁵⁵Co, ⁵⁷Co, ⁵⁸Co, ⁵²Fe, ⁵⁶Ni, ⁵⁷Ni, ⁶²Cu, ⁶⁴Cu, ⁶⁷Cu, ⁶⁶Ga, ⁶⁸Ga, ⁶⁷Ga, ⁸⁹Zr, ⁹⁰Y, ⁸⁶Y, ^{94m}Tc, ^{99m}Tc, ⁹⁷Ru, ¹⁰⁵Rh, ¹⁰⁹Pd, ¹¹¹Ag, ¹¹⁰In, ¹¹¹In, ^{113m}In, ^{114m}In, ^{117m}Sn, ¹²¹Sn, ¹²⁷Te, ¹⁴²Pr, ¹⁴³Pr, ¹⁴⁷Nd, ¹⁴⁹Gd, ¹⁴⁹Pm, ¹⁵¹Pm, ¹⁴⁹Tb, ¹⁵²Tb, ¹⁵⁵Tb, ¹⁵³Sm, ¹⁵⁶Eu, ¹⁵⁷Gd, ¹⁵⁵Tb, ¹⁶¹Tb, ¹⁶⁴Tb, ¹⁶¹Ho, ¹⁶⁶Ho, ¹⁵⁷Dy, ¹⁶⁵Dy, ¹⁶⁶Dy, ¹⁶⁰Er, ¹⁶⁵Er, ¹⁶⁹Er, ¹⁷¹Er, ¹⁶⁶Yb, ¹⁶⁹Yb, ¹⁷⁵Yb, ¹⁶⁷Tm, ¹⁷²Tm, ¹⁷⁷Lu, ¹⁸⁶Re, ^{186g}Re, ¹⁸⁸Re, ¹⁸⁸W, ¹⁹¹Pt, ^{195m}Pt, ¹⁹⁴Ir, ¹⁹⁷Hg, ¹⁹⁸Au, ¹⁹⁹Au, ²¹²Pb, ²⁰³Pb, ²¹¹At, ²¹²Bi, ²¹³Bi, ²²³Ra, ²²⁴Ra, ²²⁵Ac, ²²⁶Th and ²²⁷Th, and from cations of nonradioactive isotopes thereof, or is a cationic molecule comprising ¹⁸F or ¹⁹F, such as ¹⁸F-[AlF]²⁺ or ¹⁸F-[ScF]²⁺.

Among this group of cations the cation is preferably selected from the radioactive cations of ⁶⁸Ga, ⁹⁰Y, and ¹⁷⁷Lu and from nonradioactive cationsof Ga, Y or Lu.

⁶⁸Ga is frequently used as a tracer for pharmaceutical molecules. ⁹⁰Y plays a significant role in the treatment of hepatocellular carcinoma (HCC), leukaemia, and lymphoma, although it has the potential to treat a range of tumors. ¹⁷⁷Lu is used in the art for radiolabeling of pharmaceutical molecules, aimed either as an anti-cancer therapy or for scintigraphy (medical imaging).

In accordance with another preferred embodiment of the second aspect of the invention the conjugate further comprises within the single molecule: (c) a silicon-based fluoride acceptor moiety R^{SiFA} selected from: (i) a silicon-based fluoride acceptor moiety which comprises a silicon atom and a fluorine atom, wherein the fluorine atom is linked via a covalent bond directly to the silicon atom, and which moiety can be labeled with ¹⁸F by isotopic exchange of ¹⁹F by ¹⁸F or which is labeled with ¹⁸F; (ii) a silicon-based fluoride acceptor moiety which comprises a silicon atom and a hydroxy group, wherein the hydroxy group is linked via a covalent bond directly to the silicon atom, and which moiety can be labeled with ¹⁸F by nucleophilic substitution of OH by ¹⁸F; and (iii) a silicon-based fluoride acceptor moiety which comprises a silicon atom and a hydrogen atom, wherein the hydrogen atom is linked via a covalent bond directly to the silicon atom, and which moiety can be labeled with ¹⁸F by nucleophilic substitution of H by ¹⁸F.

The use of a silicon-based fluoride acceptor moiety R^{SiFA}, also briefly referred to as a silicon fluoride acceptor moiety, represents an attractive approach for introducing an ¹⁸F label. Silicon fluoride acceptor moieties are described, for example, in Lindner et al., Bioconjugate Chemistry 25, 738-749 (2014). It has been demonstrated in the literature that ¹⁸F-labeled compounds using silicon-based fluoride acceptors can be produced by ¹⁸F-for-¹⁹F isotopic exchange reactions, but also by ¹⁸F-for-OH and even ¹⁸F-for-H substitution reactions, e.g. in Mu L et al., Angew Chem Int Ed Engl. 2008;47(26):4922-5 and Höhne A et al., Bioconjug Chem. 2008 Sep;19(9):1871-9, respectively. However, in order to preserve the silicon-fluoride bond, silicon-based fluoride acceptors frequently rely on sterically demanding groups, e.g. two tert-butyl groups at the Si-F and Si-¹⁸F silicon group, e.g. as Si(*tert*-butyl)₂X, where X is F or ¹⁸F. This in turn renders silicon fluoride acceptors highly hydrophobic. In terms of binding to the target molecule, in particular to the target protein which is CCK-2R, the hydrophobic moiety provided by the silicon-based fluoride acceptor may be exploited for the purpose of establishing interactions of the radio-diagnostic or -therapeutic compound with the hydrophobic pocket described in Zhang et al., Journal of the American Chemical Society 132, 12711-12716 (2010). Yet, prior to binding, the higher degree of lipophilicity introduced into the molecule poses a severe problem with respect to the development of radiopharmaceuticals with suitable in vivo biodistribution, i.e. low unspecific binding in non-target tissue.

In accordance with a more preferred embodiment of the second aspect of the invention, the silicon-based fluoride acceptor moiety R^{SiFA} contains a group of the following formula (S-1): 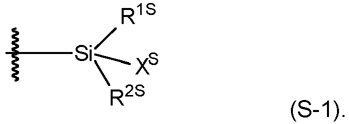 wherein
X^{S} is F, OH or H, preferably F;
R^{1S} and R^{2S} are independently a linear or branched C₃ to C₁₀ alkyl group, preferably R^{1S} and R^{2S} are independently selected from isopropyl and *tert*-butyl, and more preferably R^{1S} and R^{2S} are *tert*-butyl. The waved line marks the bond which attaches the group to the remainder of the conjugate compound.

More preferably, the moiety R^{SiFA} contains a group of the formula (S-2): 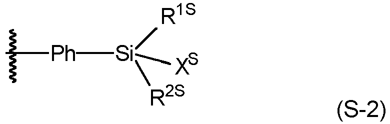 wherein
X^{S} is F, OH or H, preferably F;
R^{1S} and R^{2S} are independently a linear or branched C₃ to C₁₀ alkyl group, preferably R^{1S} and R^{2S} are independently selected from isopropyl and *tert*-butyl, and more preferably R^{1S} and R^{2S} are *tert*-butyl; Ph is a phenylene group (-C₆H₄-), wherein one or more, such as one, two, or three of the hydrogen atoms may be replaced by an organic functional group as a substituent. The waved line marks the bond which attaches the group to the remainder of the conjugate compound.

It is still further preferred that the silicon-based fluoride acceptor moiety R^{SiFA} has a structure represented by one of the following formulae (S-3) and (S-4), or by one of the following formulae (S-5) to (S-7).

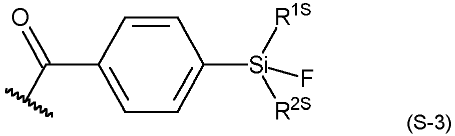

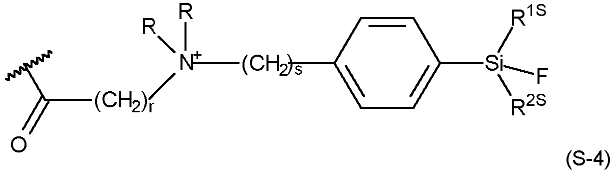

In the moiety of formula (S-3), R^{1S} and R^{2S} are independently from each other a linear or branched C3 to C10 alkyl group, preferably R^{1S} and R^{2S} are selected from isopropyl and tert-butyl, and more preferably R^{1S} and R^{2S} are tert-butyl. The waved line marks a bond which attaches the group to the remainder of the compound.

In the moiety of formula (S-4), r is 1, 2 or 3, preferably 1, s in -(CH₂)ₛ- is an integer of 1 to 6 and is preferably 1,
the groups R are, independently, H or C1 to C6 alkyl, preferably H or C1 to C2 alkyl, and are more preferably both methyl, and
R^{1S} and R^{2S} are independently from each other a linear or branched C3 to C10 alkyl group, preferably R^{1S} and R^{2S} are selected from isopropyl and tert-butyl, and more preferably R^{1S} and R^{2S} are tert-butyl. The waved line marks a bond which attaches the group to the remainder of the compound. As exemplary counterions for the positively charged quaternary ammonium group indicated in formula (S-4) which carries two substituents R, reference can be made to trifluoro acetate anions, acetate anions, or to halogenide anions such as chloride ions.

As will be understood by the skilled reader, the fluorine atom indicated in formulae (S-3) and (S-4) may be a ¹⁸F atom, or a ¹⁹F atom which can be exchanged to provide ¹⁸F by isotopic exchange of ¹⁹F by ¹⁸F.

Furthermore, the moiety of formula (S-3) is preferably a moiety of formula (S-3a) (also referred to as "SiFA" herein), and the moiety of formula (S-4) is preferably a moiety of formula (S-4a) (also referred to as "SiFAlin" herein): 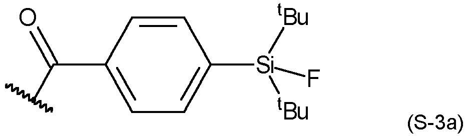 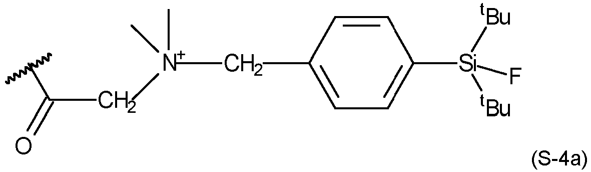 wherein ^{t}Bu indicates a tert-butyl group. The waved line marks a bond which attaches the group to the remainder of the compound. As noted above with respect to (S-4), exemplary counterions for the positively charged quaternary ammonium group indicated in formula (S-4a) which carries two methyl substituents are trifluoro acetate anions, acetate anions, or halogenide anions such as chloride ions. 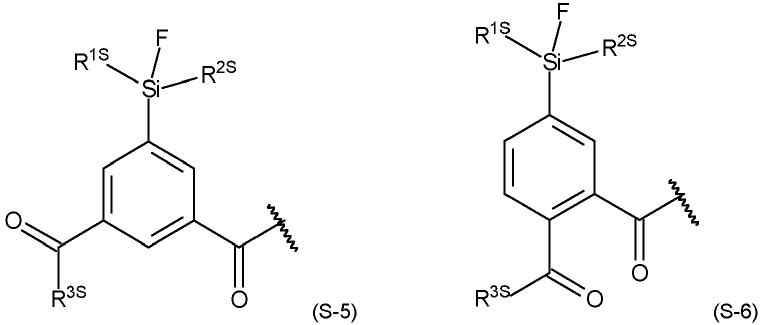 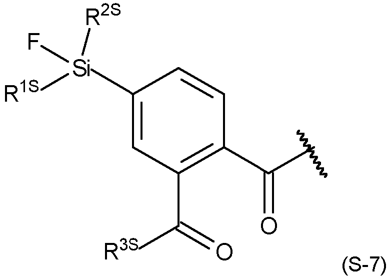 wherein:
R^{1S} and R^{2S} are independently a linear or branched C₃ to C₁₀ alkyl group, preferably R^{1S} and R^{2S} are independently selected from isopropyl and tert-butyl, and more preferably R^{1S} and R^{2S} are tert-butyl; and
R^{3S} is selected from
   (i) -OH or -O⁻,
   (ii) a sugar moiety or an amino sugar moiety,
   (iii) an amino acid moiety or an oligopeptide moiety,
   (iv) a PEG moiety;
and from combinations of two or more of (ii), (iii) and (iv).

The waved line marks a bond which attaches the group to the remainder of the conjugate compound.

It will be understood that, if R^{3S} represents -O⁻, the substituent -C(=O)R^{3S} of the aromatic ring shown in the above formulae is a deprotonated carboxylate group -C(=O)O⁻.

Among formulas (S-5), (S-6) and (S-7), formula (S-5) is preferred.

The amino acid moiety as R^{3S} is preferably derived from a hydrophilic amino acid that comprises, in addition to an amino group and a carboxyl group, a further basic or acidic functional group, or wherein the oligopeptide moiety as R^{3S} is derived from an oligopeptide with at least one hydrophilic amino acid that comprises, in addition to an amino group and a carboxyl group, a further basic or acidic functional group.

The oligopeptide moiety as R^{3S} is preferably a linear or branched moiety comprising 2 to 10, preferably 2 to 5, and more preferably 2 or 3 amino acid moieties.

The hydrophilic amino acid is preferably selected from lysine and glutamic acid.

The PEG moiety as R^{3S} is preferably a moiety of the formula -NH-(CH₂-CH₂-O)_{X}-R^{P1}, wherein the nitrogen atom providing an open bond forms an amide bond -NH-C(O)- with the carbon atom to which R^{3S} is attached, X is an integer of 2 to 10, preferably 4 to 10, and is more preferably 8, and R^{P1} is selected from -CH₂-COOH and -CH₂-CH₂-COOH.

The sugar moiety or amino sugar moiety as R^{3S} is preferably a residue derived from 6-amino-6-deoxy-D-galactopyranose and corresponding tautomers thereof.

R^{3S} is more preferably selected from -OH, and -O⁻. A silicon-based fluoride acceptor moiety of formula (S-5) wherein R^{1S} and R^{2S} represent tert-butyl groups and R^{3S} represents -OH or -O- is also referred to as "SiFA-ipa" herein.

In accordance with a preferred embodiment of the second aspect of the invention the conjugate is represented by formula (III) 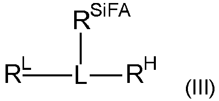 wherein:
R^{L} is one or more chelating groups as defined herein above, wherein chelating group optionally contains a chelated nonradioactive or radioactive cation optionally contains a chelated nonradioactive or radioactive cation;
L is a linking moiety;
R^{H} is the tetrapeptide of the first aspect of the invention, and
R^{SiFA} is present or absent, and, if present, is a silicon-based fluoride acceptor moiety as defined herein above.

It is to be understood that the linking moiety can be any moiety that is capable of connecting the chelating groups and the tetrapeptide.

The one or more chelating groups is preferably one chelating group.

In accordance with a preferred embodiment of the second aspect of the invention the linking moiety comprises D/L-diaminopropionic acid (D/L-Dap), D/L-diaminobutyric acid (D/L-Dab), D/L-ornithine (D/L-Orn) or D/L-lysine (D/L-Lys), wherein, if present, the SiFA is conjugated to D/L-Dap, D/L-Dab, D/L-Orn or D/L-Lys using the -NH₂ groups contained in these amino acids to provide a coupling group -NH-wherein the bond to one hydrogen atom in the -NH₂ group is replaced by a bond to the SiFA, wherein D/L-Dap, D/L-Dab, D/L-Orn or D/L-Lys are preferably placed such in the linking moiety that D/L-Dap, D/L-Dab, D/L- D/L-Orn or Lys are directly connected to R^{L} and/or R^{SiFA}.

Hence, the linking moieties according to this preferred embodiment can serve or serve as the site for the connection R^{SiFA} to the conjugate. Among the options D/L-Dap, D/L-Dab, D/L-Orn or D/L-Lys the option D/L-Dap is most preferred.

In accordance with a further preferred embodiment of the second aspect of the invention the linking moiety comprises polyethylenglycol PEG, preferably (PEG)₃₋₁₁ optionally together with poly-L/D-hydroxyproline (L/D-Hyp), preferably (Hyp)₁₋₈, and/or α- or γ-glu, preferably γ-glu, and wherein the linker most preferably comprises y-glu-(Hyp)₆-y-glu-(PEG)₃ or y-glu-(PEG)₄-y-glu-(PEG)₃.

Hence, the moieties according to this preferred embodiment are preferably used together with the linking moieties according to the before described preferred embodiment that can serve or serve as the site for the connection R^{SiFA} to the conjugate. L/D-Hyp is preferably L-Hyp.

The linking moieties according to this preferred embodiment do not only connect one or more chelating groups and the tetrapeptide but also provide a distance between the one or more chelating groups and the tetrapeptide. It is shown in the appended examples that adding a distance between the sterically demanding chelators such as DOTA and the tetrapeptide by a linker according favors high CCK-2R affinity.

In accordance with a further preferred embodiment of the second aspect of the invention the conjugate is represented by DOTA-γ-glu-(PEG)₃-Trp-(N-Me)Nle-Asp-1-Nal-NH₂, DOTA-dap(SiFA)-γ-glu-(PEG)₇-γ-glu-(PEG)₃-Trp-(N-Me)Nle-Asp-1-Nal-NH₂, DOTA-dap(SiFA)-y-glu-(Hyp)₆-y-glu-(PEG)₃-Trp-(N-Me)Nle-Asp-1-Nal-NH₂, or DOTA-dap(SiFAlin)-y-glu-(PEG)₄-y-glu-(PEG)₃-Trp-(N-Me)Nle-Asp-1-Nal-NH₂, wherein the DOTA chelator optionally contains a chelated nonradioactive or radioactive cation as described herein above, preferably a cation of natLu, natGa, 177Lu or 68Ga.

Among the abobe four conjugates DOTA-dap(SiFA)-y-glu-(Hyp)₆-y-glu-(PEG)₃-Trp-(N-Me)Nle-Asp-1-Nal-NH₂ is most preferred. his embodiment is directed to the best performers with and without a silicon-based fluoride acceptor moiety as illustrated in the appended examples and it is believed that they are very good candidates for clinical applications.

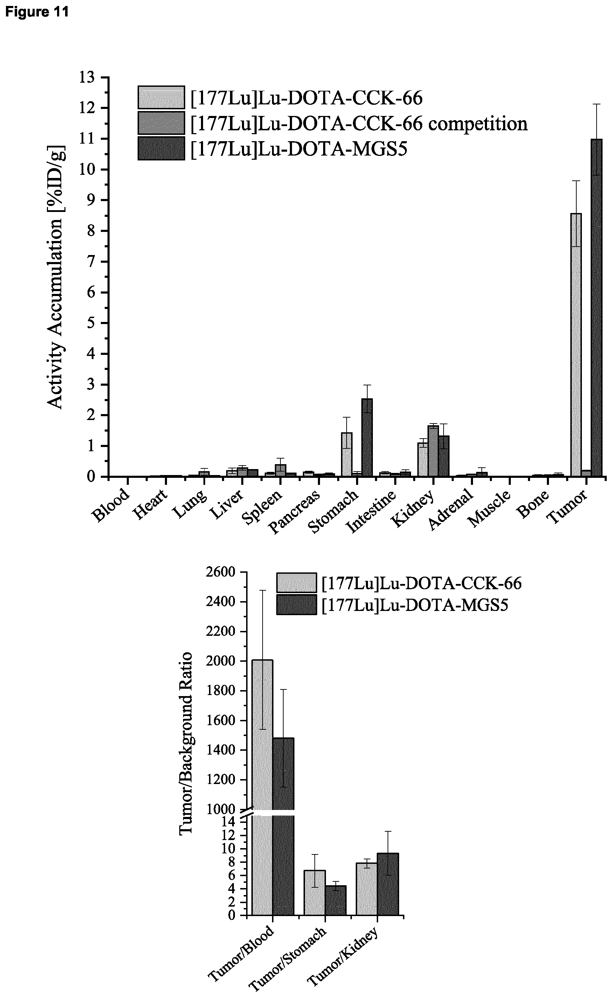

DOTA-γ-glu-(PEG)₃-Trp-(N-Me)Nle-Asp-1-Nal-NH₂ consists of DOTA as chelator, γ-glu-(PEG)₃ as linker and the tetrapeptide -(N-Me)Nle-Asp-1-Nal-NH₂. The DOTA can be complexed with a nonradioactive or radioactive cation as described herein above, such as a cation of natLu, natGa, 177Lu or 68Ga. For therapeutic and diagnostic applications as described herein DOTA is preferably complexed with a nonradioactive or radioactive cation as described herein above. DOTA-γ-glu-(PEG)₃-Trp-(N-Me)Nle-Asp-1-Nal-NH₂ is referred to in the examples as DOTA-CCK-66 and the examples show that DOTA-CCK-66 is particularly suitable as theranostic agent. Upon administration to a tumor mouse it becomes specifically enriched in the tumor with background in other tissues; see **Fig 11****.** DOTA-CCK-66, revealed improved *in vivo* data compared to the clinically applied compound, [⁶⁸Ga]Ga-/[¹⁷⁷Lu]Lu-DOTA-MGS5. Tumor / background ratios are comparable or even enhanced for the former, which can be attributed to the tetrapeptidic design in combination with the PEG linkers.

DOTA-dap(SiFA)-γ-glu-(PEG)₇-γ-glu-(PEG)₃-Trp-(N-Me)Nle-Asp-1-Nal-NH₂ consists of DOTA as chelator, dap as the site for connecting the SiFA moiety (i.e. a moiety of formula (S-3a) as shown above), -glu-(PEG)₇-γ-glu-(PEG)₃ as linker and the tetrapeptide Trp-(N-Me)Nle-Asp-1-Nal-NH₂. DOTA-dap(SiFA)-γ-glu-(PEG)₇-γ-glu-(PEG)₃-Trp-(N-Me)Nle-Asp-1-Nal-NH₂ is referred to in the examples as DOTA-rhCCK-70. Also DOTA-rhCCK-70 revealed high activity levels in the tumor at 24h after administration to a tumor mouse. The DOTA chelator may contain a chelated nonradioactive or radioactive cation as described herein above, preferably a cation of natLu, natGa, 177Lu or 68Ga.

DOTA-dap(SiFA)-y-glu-(Hyp)₆-y-glu-(PEG)₃-Trp-(N-Me)Nle-Asp-1-Nal-NH₂ consists of DOTA as chelator, dap as the site for connecting the SiFA moiety (i.e. a moiety of formula (S-3a) as shown above), -y-glu-(Hyp)₆-y-glu-(PEG)₃- as linker and the tetrapeptide Trp-(N-Me)Nle-Asp-1-Nal-NH₂. DOTA-dap(SiFA)-y-glu-(Hyp)₆-y-glu-(PEG)₃-Trp-(N-Me)Nle-Asp-1-Nal is referred to in the examples as DOTA-rhCCK-84. In a number of test compounds as illustrated in the examples lipophilicity and CCK-2R affinity was addressed *via* a poly-L-hydroxyproline linker. Among these tested compounds DOTA-rhCCK-84 advantageously displayed the highest hydrophilicity (log*D*_{7.4} = -2.14 ± 0.06) and CCK-2R affinity (*IC*₅₀ = 7.87 ± 0.3). Also DOTA-rhCCK-84 revealed one of the highest activity levels in the tumor at 24 h after administration to a tumor mouse, while activity levels in the kidneys were low. The DOTA chelator may contain a chelated nonradioactive or radioactive cation as described herein above, preferably a cation of natLu, natGa, 177Lu or 68Ga.

DOTA-dap(SiFAlin)-y-glu-(PEG)₄-y-glu-(PEG)₃-Trp-(N-Me)Nle-Asp-1-Nal-NH₂ consists of DOTA as chelator, dap as the site for connecting the SiFAlin moiety (i.e. a moiety of formula (S-4a) as shown above), -y-glu-(PEG)₄-y-glu-(PEG)₃-as linker and the tetrapeptide Trp-(N-Me)Nle-Asp-1-Nal-NH₂. DOTA-dap(SiFAlin)-y-glu-(PEG)₄-y-glu-(PEG)₃-Trp-(N-Me)Nle-Asp-1-Nal is referred to in the examples as DOTA-rhCCK-91. DOTA-rhCCK-91 advantageously displayed high CCK-2R affinity when labeled either with [^{nat}Lu]lutetium (*IC*₅₀ = 8.56 ± 0.7) or [^{nat}Ga]gallium (*IC*₅₀ = 8.24 ± 1.6). The DOTA chelator group may contain a chelated nonradioactive or radioactive cation as described herein above, preferably a cation of natLu, natGa, 177Lu or 68Ga.

The present invention relates in a third aspect to the use of the tetrapeptide or the conjugate of the invention in the *in vitro* or *ex vivo* diagnosis of cancer.

The present invention relates in a related fourth aspect to the use of the tetrapeptide or the conjugate of the invention for detecting *in vitro* or *ex vivo* the presence of Cholecystokinin 2 receptor (CCK-2R) in a sample, wherein the sample has preferably been obtained from a cancer patient or subject being suspected to have cancer.

The present invention relates in a fifth aspect to an *in vitro* or *ex vivo* method for detecting the presence of Cholecystokinin 2 receptor (CCK-2R) in a sample, wherein the sample has preferably been obtained from a cancer patient or a subject being suspected to have a cancer, the method comprising (a) contacting the tetrapeptide or the conjugate of the invention with the sample, and (b) detecting the binding of the tetrapeptide or the conjugate of the invention to CCK-2R.

The *in vitro* or *ex vivo* diagnosis of cancer is not practiced on the human or animal body but is instead usually carried out on the basis of a sample that has been obtained from a subject to be diagnosed for cancer.

The sample can be a body fluid or a tissue sample. The body fluid is preferably a blood sample selected from whole bold, serum or plasma. The tissue sample is preferably from the tissue that is supposed to be cancerous, such as breast, lung, liver or colon tissue.

The present invention relates in a sixth aspect to a pharmaceutical composition or a diagnostic composition comprising the tetrapeptide or the conjugate of the invention.

The term "composition" as used herein refers to a composition comprising at least the tetrapeptide or the conjugate of the invention which are also collectively referred in the following as compounds.

With respect to all pharmaceutical and diagnostic applications as described herein it is to be understood that the chelator in these aspects of the invention is preferably complexed with a radioactive cation. The nature of the radioactive cation can be selected based on the planned pharmaceutical or diagnostic applications. For instance, for diagnostic applications a radioactive cation with a relatively short half-life and a relatively weak radiation that can still be detected is generally desired while for therapeutic applications a radioactive cation with a relatively long half-life and a relatively strong radiation is generally desired in order to destroy the tumor cells.

In accordance with the present invention, the term "pharmaceutical composition" relates to a composition for administration to a patient, preferably a human patient that is to be administered for treating or preventing a disease. The pharmaceutical composition of the invention comprises the compounds of the invention as recited above. It may, optionally, comprise further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well-known conventional methods. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day.

In accordance with the present invention, the term "diagnostic composition" relates to a composition for administration to a patient, preferably a human patient that is to be administered for diagnosing a disease. The diagnostic composition of the invention comprises the compounds of the invention recited above. It may, optionally, comprise the same further molecules as the pharmaceutical composition and may also be in the same form as the pharmaceutical composition. The above preferred ingredients and formulations of the pharmaceutical composition apply *mutatis mutandis* to the diagnostic composition.

The present invention relates in a seventh aspect to the tetrapeptide or the conjugate or the pharmaceutical composition of the invention for treating or preventing cancer.

The present invention relates in an eighth aspect to the tetrapeptide or the conjugate or the pharmaceutical composition of the invention for use in a method of diagnosis *in vivo* a cancer in a subject.

The subject as referred to herein is preferably a mammal and most preferably human.

Cancer is an abnormal malignant new growth of tissue that possesses no physiological function and arises from uncontrolled usually rapid cellular proliferation. The cancer is preferably selected from the group consisting of breast cancer, ovarian cancer, endometrial cancer, vaginal cancer, vulva cancer, bladder cancer, salivary gland cancer, pancreatic cancer, thyroid cancer, kidney cancer, lung cancer, cancer concerning the upper gastrointestinal tract, colon cancer, colorectal cancer, prostate cancer, squamous-cell carcinoma of the head and neck, cervical cancer, glioblastomas, malignant ascites, lymphomas and leukemia. Among this list of cancers are preferred colorectal cancer, pancreatic cancer and thyroid cancer are preferred since in these cancer types abnormal CCK-2R expression was already found. Thyroid cancer in particular medullary thyroid cancer (MTC) are most preferred in view of the illustration of this cancer type by the appended examples.

The cancer is preferably a solid cancer. A solid cancer is an abnormal mass of tissue that usually does not contain cysts or liquid areas by contrast to a liquid cancer.

The present invention relates in a ninth aspect to a conjugate compound binding to Cholecystokinin 2 receptor (CCK-2R) that is represented by formula (IV) R^{L}-dap(R^{SiFA})-R^{GABA}-L-R^{H} (IV), wherein:
- R^{L}: is DOTA or DOTAGA, optionally containing a chelated nonradioactive or radioactive cation;
- dap(R^{SiFA}): is a dap group carrying a silicon-based fluoride acceptor moiety R^{SiFA};
- R^{GABA}: is present or absent, and, if present, is one or more γ-aminobutryic acid (GABA), preferably (GABA)₁₋₃;
- L: is a linking moiety comprising at least 5, preferably at least 6 α- or γ-glu and most preferably 6 to 9 α- or γ-glu, whereby γ-glu is preferred over α-glu; and
- R^{H}: is or comprises Ala-Tyr-Gly-Trp-Nle-Asp-Phe-NH₂.

The above definitions and preferred embodiments of the first to eight aspects of the invention apply *mutatis mutandis* to the ninth aspect and the further aspects of the invention as will follow herein below, as far as these definitions and preferred embodiments are amenable for combination with the ninth aspect and the further aspects of the invention as will follow herein below.

As can be taken form the appended examples several chelator-minigastrin- silicon-based fluoride acceptor conjugates comprising a poly-glutamate linker were synthesized and evaluated *in vitro* ^{nat/177}Lu-labeled and also compared with the prior art reference ligands ^{nat/177}Lu-DOTA-PP-F11N and ^{nat/177}Lu-CP04 (^{nat/177}Lu-DOTA-(glu)₆-Ala-Tyr-Gly-Trp-Met-Asp-Phe-NH₂).

It was surprisingly found that the highest CCK-2R affinity was observed when this group was separated by at least five or six α-/γ-glu moieties, whereby further six α-/γ-glu moieties did not provide a further improvement. It is believed that this highest CCK-2R affinity is due to the separation of the dap(R^{SiFA}) and the chelator moiety from the CCK-2R binding sequence (Ala-Tyr-Gly-Trp1-Nle-Asp-Phe-NH₂). γ-glu is preferred over α-glu as the respective peptide backbone is distinctly prolonged by using γ-glu instead of α-glu. In addition, also different chelators were tested and the best results with respect to CCK-2R affinity were obtained by using DOTA or DOTAGA. DOTA and DOTAGA are preferably bound with one of its carboxylic groups via an amide bond to the remainder of the conjugate. Yet further different compounds and places for introducing the silicon-based fluoride acceptor moiety R^{SiFA} into the conjugates were tested and it was found that dap is the best compound for introducing the silicon-based fluoride acceptor moiety and that the best place of dap is between the chelator and linker and, if present, GABA. As will be understood by the skilled reader, the dap (diaminopropanoic acid) acts as a linking unit in the compounds of formula (IV) between R^{L} and L (if R^{GABA} is absent) or between R^{L} and R^{GABA} (if R^{GABA} is present), and can additionally carry a silicon-based fluoride acceptor moiety R^{SiFA} due to the three functional groups provided by dap.

The one or more γ-aminobutryic acid (GABA) can be optionally present in order to reduce the negative charges of the γ-glu by γ-aminobutyric acid (GABA) moieties. It is at the same time possible to retain the linker length but with a less amount of negative charges, which resulted in the four compounds. Due to the addition of the one or more γ-aminobutryic acid (GABA) the kidney is capable of reducing the amount of conjugate that can be found in the kidney thereby enriching the specific accumulation of the conjugate in the tumor, if the amounts in the kidney should be a concern (noting that also without GABA the conjugates of the ninth aspect specifically accumulate in the tumor upon administration).

Hence, the ninth aspect of the invention is directed to particular preferred Cholecystokinin 2 receptor binding molecules that are useful for imaging and targeted radiotherapy. Tests with chelator-minigastrin-SiFA conjugates revealed that the conjugates of the ninth aspect are advantageously these with the highest CCK-2R affinity.

As mentioned, the above definitions and preferred embodiments of the first to eight aspects of the invention apply *mutatis mutandis* to the ninth aspect and the further aspects of the invention as will follow herein below, as far as these definitions and preferred embodiments are amenable for combination with the ninth aspect and the further aspects of the invention as will follow herein below.

It follows that also in accordance with a preferred embodiment of the ninth aspect of the invention the radioactive or nonradioactive cation of the chelating group is selected from the cations of ⁴³Sc, ⁴⁴Sc, ⁴⁷Sc, ⁵¹Cr, ^{52m}Mn, ⁵⁵Co, ⁵⁷Co, ⁵⁸Co, ⁵²Fe, ⁵⁶Ni, ⁵⁷Ni, ⁶²Cu, ⁶⁴Cu, ⁶⁷Cu, ⁶⁶Ga, ⁶⁸Ga, ⁶⁷Ga, ⁸⁹Zr, ⁹⁰Y, ⁸⁶Y, ^{94m}Tc, ^{99m}Tc, ⁹⁷Ru, ¹⁰⁵Rh, ¹⁰⁹Pd, ¹¹¹Ag, ^{110m}In, ¹¹¹In, ^{113m}In, ^{114m}In, ^{117m}Sn, ¹²¹Sn, ¹²⁷Te, ¹⁴²Pr, ¹⁴³Pr, ¹⁴⁷Nd, ¹⁴⁹Gd, ¹⁴⁹Pm, ¹⁵¹Pm, ¹⁴⁹Tb, ¹⁵²Tb, ¹⁵⁵Tb, ¹⁵³Sm, ¹⁵⁶Eu, ¹⁵⁷Gd, ¹⁵⁵Tb, ¹⁶¹Tb, ¹⁶⁴Tb, ¹⁶¹Ho, ¹⁶⁶Ho, ¹⁵⁷Dy, ¹⁶⁵Dy, ¹⁶⁶Dy, ¹⁶⁰Er, ¹⁶⁵Er, ¹⁶⁹Er, ¹⁷¹Er, ¹⁶⁶Yb, ¹⁶⁹Yb, ¹⁷⁵Yb, ¹⁶⁷Tm, ¹⁷²Tm, ¹⁷⁷Lu, ¹⁸⁶Re, ^{186g}Re, ¹⁸⁸Re, ¹⁸⁸W, ¹⁹¹Pt, ^{195m}Pt, ¹⁹⁴Ir, ¹⁹⁷Hg, ¹⁹⁸Au, ¹⁹⁹Au, ²¹²Pb, ²⁰³Pb, ²¹¹At, ²¹²Bi, ²¹³Bi, ²²³Ra, ²²⁴Ra, ²²⁵Ac, ²²⁶Th and ²²⁷Th, and from cations of nonradioactive isotopes thereof, or is a cationic molecule comprising ¹⁸F or ¹⁹F, such as ¹⁸F-[AlF]²⁺, and is preferably selected from a cation of ⁶⁸Ga, ⁹⁰Y, or ¹⁷⁷Lu and from cations of nonradioactive isotopes of Ga, Y or Lu.

Further details and preferred embodiments of the radioactive or nonradioactive cation are described herein above in connection with the second aspect of the invention. These details and preferred embodiments apply *mutatis mutandis* to the ninth aspect of the invention.

It furthermore follows that also in accordance with a preferred embodiment of the ninth aspect of the invention the silicon-based fluoride acceptor moiety R^{SiFA} is as defined herein above in connection with the second aspects of the invention.

In particular, the silicon-based fluoride acceptor moiety is preferably selected from:
(i) a silicon-based fluoride acceptor moiety which comprises a silicon atom and a fluorine atom, wherein the fluorine atom is linked via a covalent bond directly to the silicon atom, and which moiety can be labeled with ¹⁸F by isotopic exchange of ¹⁹F by ¹⁸F or which is labeled with ¹⁸F;
(ii) a silicon-based fluoride acceptor moiety which comprises a silicon atom and a hydroxy group, wherein the hydroxy group is linked via a covalent bond directly to the silicon atom, and which moiety can be labeled with ¹⁸F by nucleophilic substitution of OH by ¹⁸F; and
(iii) a silicon-based fluoride acceptor moiety which comprises a silicon atom and a hydrogen atom, wherein the hydrogen atom is linked via a covalent bond directly to the silicon atom, and which moiety can be labeled with ¹⁸F by nucleophilic substitution of H by ¹⁸F.

More preferably the silicon-based fluoride acceptor moiety R^{SiFA} contains a group of the formula (S-1) as defined above: 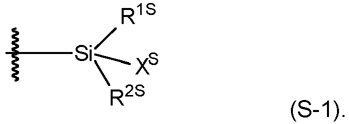 wherein
X^{S} is F, OH or H, preferably F;
R^{1S} and R^{2S} are independently a linear or branched C₃ to C₁₀ alkyl group, preferably R^{1S} and R^{2S} are independently selected from isopropyl and tert-butyl, and more preferably R^{1S} and R^{2S} are tert-butyl.

More preferably, the moiety R^{SiFA} contains a group of the formula (S-2) as defined above: 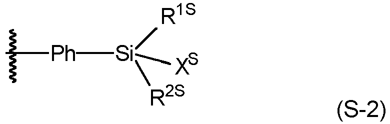 wherein
X^{S} is F, OH or H, preferably F;
R^{1S} and R^{2S} are independently a linear or branched C₃ to C₁₀ alkyl group, preferably R^{1S} and R^{2S} are independently selected from isopropyl and tert-butyl, and more preferably R^{1S} and R^{2S} are tert-butyl; Ph is a phenylene group (-C₆H₄-), wherein one or more, such as one, two, or three of the hydrogen atoms may be replaced by an organic functional group as a substituent.

It is still further preferred that the silicon-based fluoride acceptor moiety R^{SiFA} has a structure represented by one of the formulae (S-3) and (S-4), or by one of the formulae (S-5) to (S-7) as defined above.

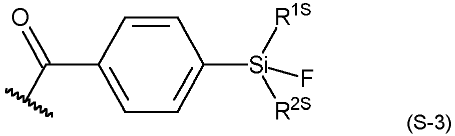

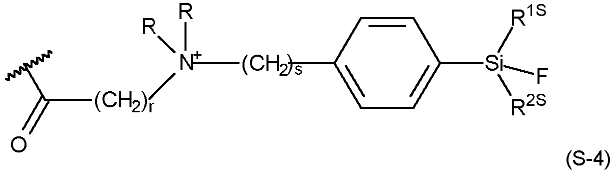

In the moiety of formula (S-3), R^{1S} and R^{2S} are independently from each other a linear or branched C3 to C10 alkyl group, preferably R^{1S} and R^{2S} are selected from isopropyl and tert-butyl, and more preferably R^{1S} and R^{2S} are tert-butyl.

In the moiety of formula (S-4), r is 1, 2 or 3, preferably 1, s in -(CH₂)ₛ- is an integer of 1 to 6 and is preferably 1,
the groups R are, independently, H or C1 to C6 alkyl, preferably H or C1 to C2 alkyl, and are more preferably both methyl, and
R^{1S} and R^{2S} are independently from each other a linear or branched C3 to C10 alkyl group, preferably R^{1S} and R^{2S} are selected from isopropyl and tert-butyl, and more preferably R^{1S} and R^{2S} are tert-butyl.

Furthermore, the moiety of formula (S-3) is preferably a moiety of formula (S-3a) (also referred to as "SiFA" herein), and the moiety of formula (S-4) is preferably a moiety of formula (S-4a) (also referred to as "SiFAlin" herein): 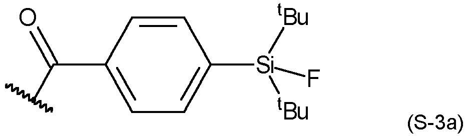 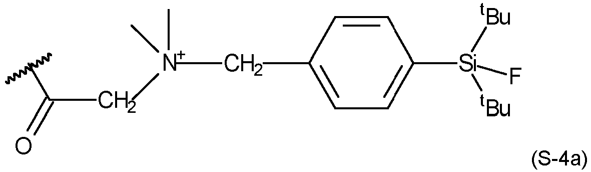 wherein ^{t}Bu indicates a tert-butyl group. 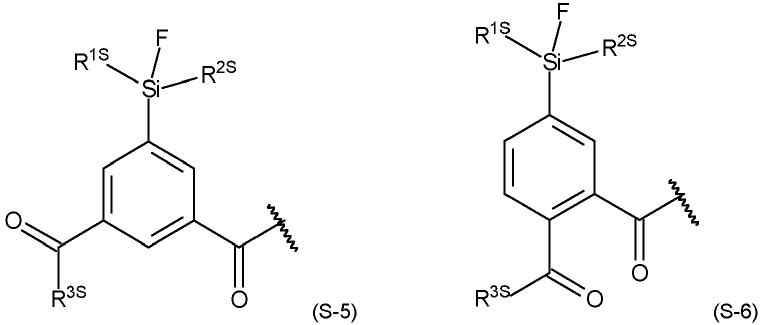 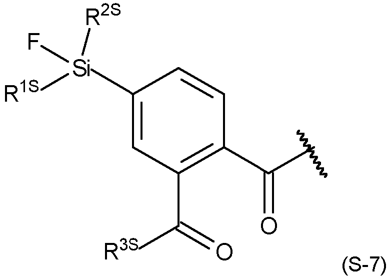 wherein:
R^{1S} and R^{2S} are independently a linear or branched C₃ to C₁₀ alkyl group, preferably R^{1S} and R^{2S} are independently selected from isopropyl and tert-butyl, and more preferably R^{1S} and R^{2S} are tert-butyl; and
R^{3S} is selected from
   (i) -OH or -O⁻,
   (ii) a sugar moiety or an amino sugar moiety,
   (iii) an amino acid moiety or an oligopeptide moiety,
   (iv) a PEG moiety;
and from combinations of two or more of (ii), (iii) and (iv).

The waved line marks a bond which attaches the group to the remainder of the conjugate compound.

Among formulas (S-5), (S-6) and (S-7), formula (S-5) is preferred.

Further details and preferred embodiments on the silicon-based fluoride acceptor moiety R^{SiFA} and of the groups and moieties of formulae (S-1), (S-2), (S-3), (S-3a), (S-4), (S-4a), (S-5), (S-6) and (S-7) are described herein above in connection with the second aspect of the invention. These details and preferred embodiments apply *mutatis mutandis* to the ninth aspect of the invention.

In accordance with a preferred embodiment of the ninth aspect of the invention the conjugate is represented by
DOTAGA-dap(SiFA)-(γ-glu)₆-Ala-Tyr-Gly-Trp-Nle-Asp-Phe-NH₂,
DOTAGA-dap(SiFA)-(α-glu)₈- Ala-Tyr-Gly-Trp-Nle-Asp-Phe-NH₂,
DOTA-dap(SiFA)-(γ-glu)₈- Ala-Tyr-Gly-Trp-Nle-Asp-Phe-NH₂.

The DOTA of DOTAGA chelator optionally contains a chelated nonradioactive or radioactive cation as described herein above.

The above preferred embodiment is directed to the three best performers among the several chelator-minigastrin-SiFA conjugates that were produced and tested in the appended examples.

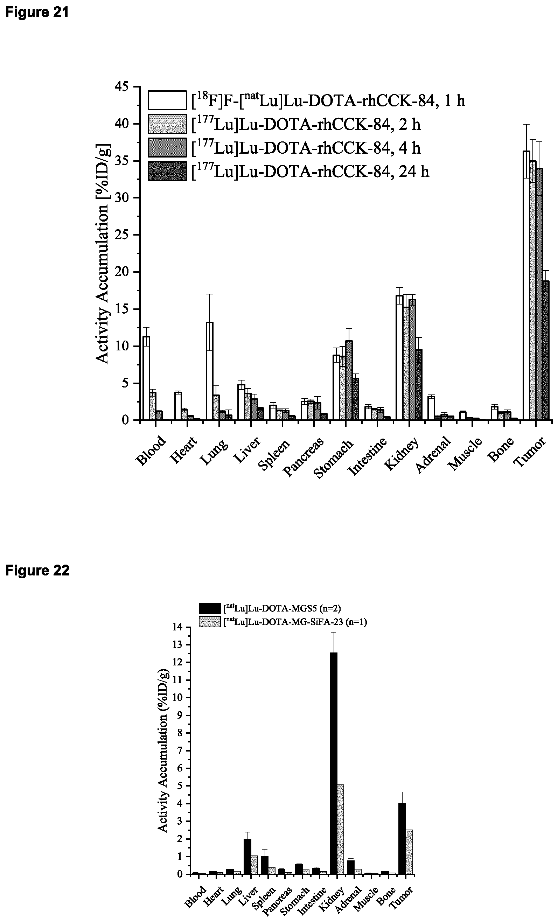

DOTAGA-dap(SiFA)-(γ-glu)₆-Ala-Tyr-Gly-Trp-Nle-Asp-Phe-NH₂ and DOTAGA-dap(SiFA)-(α-glu)₈- Ala-Tyr-Gly-Trp-Nle-Asp-Phe-NH₂ correspond to the compounds DOTAGA-MG-SiFA-19 and DOTAGA-MG-SiFA-21 as referred to in the examples, respectively. **Fig. 21** shows an improved tumor accumulation after 24 h as compared to the prior art reference DOTA-PP-F11N and significant amounts accumulate in the tumor already after 1 h. In connection with DOTA-dap(SiFA)-(γ-glu)₈- Ala-Tyr-Gly-Trp-Nle-Asp-Phe-NH₂ it was unexpectedly found that a substitution of DOTAGA by DOTA in DOTAGA-MG-SiFA-23 led to a massively increased CCK-2R affinity (DOTA-MG-SiFA-23: 4.71 ± 0.62 nM), which surpassed even the prior art reference ligands, see **Fig. 22****.**

DOTA-MG-SiFA-23 revealed the highest activity levels in the tumor at 24 h after administration to tumor mice, which activity levels were more than 8-fold higher than those of the prior art reference DOTA-PP-F11N. Also both DOTAGA-MG-SiFA compounds showed three- to sixfold increased activity levels in the tumor at after administration to tumor mice despite their lower CCK2R affinity as compared to the prior art reference DOTA-PP-F11N which is supposed to be due to decelerated activity clearance of the two DOTAGA-MG-SiFA as compared to the prior art reference.

Hence, the data in the examples prove that the three best performers are particularly useful for imaging and targeted radiotherapy.

In accordance with a preferred embodiment of the ninth aspect of the invention the conjugate has a CCK-2R binding affinity depicted as half-maximal inhibitory concentration (IC₅₀) of 100 nM or less, and preferably 75 nM or less.

DOTAGA-MG-SiFA-19, DOTAGA-MG-SiFA-21 and DOTA-MG-SiFA-23 display a half-maximal inhibitory concentration (IC₅₀) for the binding to CCK-2R of 7.54 ± 0.26 nM, 55.3 ± 7.8 and 4.71 ± 0.62 nM, respectively.

Hence, the IC50s of these three conjugates technically support that the conjugates of the ninth display an IC₅₀ of 100 nM or less, and preferably 75 nM or less.

Also the medical and the diagnostic applications as described herein above as third to eight aspects of the invention are amenable for and meant for combination with the conjugates for the ninth aspect.

Hence, further aspects of the invention relate to:
Use of the conjugate of the ninth aspect in the *in vitro* or *ex vivo* diagnosis of cancer.

Use of the conjugate of the ninth aspect for detecting *in vitro* or *ex vivo* the presence of Cholecystokinin 2 receptor (CCK-2R) in a sample, wherein the sample has preferably been obtained from a cancer patient or subject being suspected to have cancer.

An *in vitro* or *ex vivo* method for detecting the presence of Cholecystokinin 2 receptor (CCK-2R) in a sample, wherein the sample has preferably been obtained from a cancer patient or a subject being suspected to have a cancer, the method comprising (a) contacting the conjugate of the ninth aspect with the sample, and (b) detecting the binding of the conjugate of the ninth aspect to CCK-2R.

A pharmaceutical composition or a diagnostic composition comprising the conjugate of the ninth aspect.

The conjugate of the ninth aspect or the pharmaceutical composition comprising the conjugate of the ninth aspect for treating or preventing cancer.

The conjugate of the ninth aspect or the diagnostic composition conjugate of the ninth aspect for use in a method of diagnosis *in vivo* a cancer in a subject.

Further details and preferred embodiments on the medical and the diagnostic applications are described herein above in connection with the third to eighth aspect of the invention. These details and preferred embodiments apply *mutatis mutandis* to the above further aspect of the invention that use the conjugate of the ninth aspect.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show.

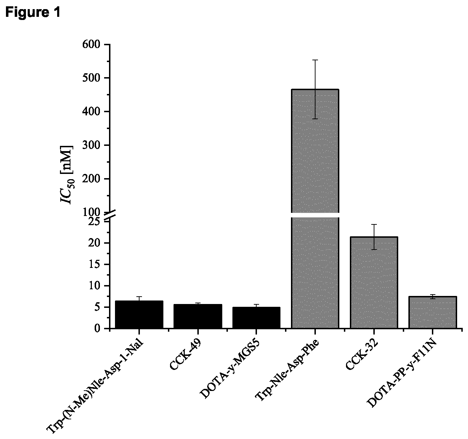

**Fig. 1****:** Schematic representation of the CCK-2R affinities (depicted as half-maximal inhibitory concentration, *IC*₅₀) of the CCK-2R-targeted ligands (*H*-Trp-(*N*-Me)Nle-Asp-1-Nal-NH₂, CCK-49, [^{nat}Lu]Lu-DOTA-γ-MGS5, *H*-Trp-Nle-Asp-Phe-NH₂, CCK-32 and [^{nat}Lu]Lu-DOTA-PP-γ-F11N). DOTA-MGS5 analogues presented in black and DOTA-PP-F11N analogues in grey.

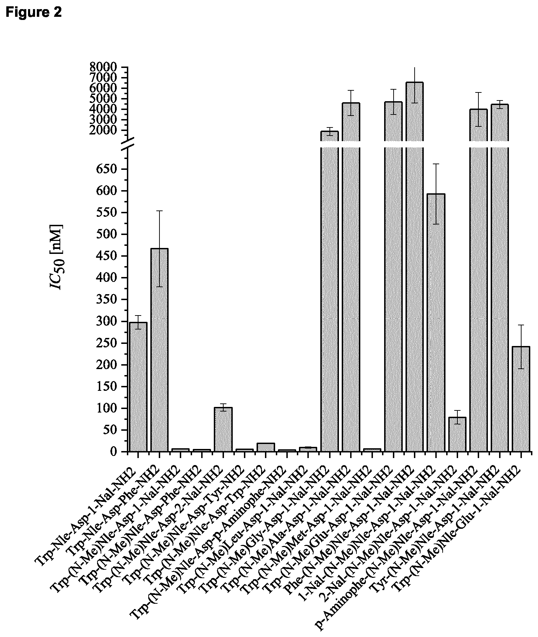

**Fig. 2****:** Schematic representation of the CCK-2R affinities (depicted as half-maximal inhibitory concentration, *IC*₅₀) of the CCK-2R-targeted ligands with modified tetrapeptidic binding motif.

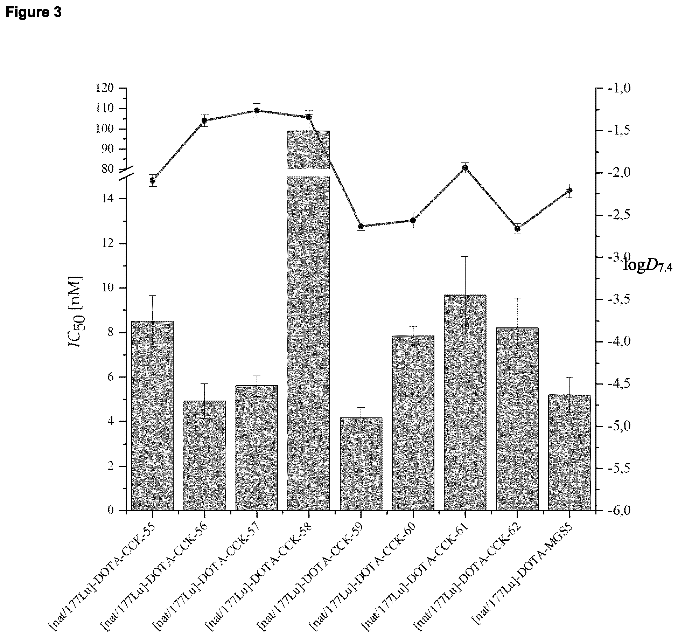

**Fig. 3****:** Schematic representation of the CCK-2R affinities (depicted as half-maximal inhibitory concentration, *IC*₅₀, in bars) and lipophilicities (depicted as n-octanol/PBS distribution coefficients, pH = 7.4, log*D*_{7.4}, in dots) of the glycine scan compounds (DOTA-CCK-55 to -62) compared to the reference (DOTA-MGS5).

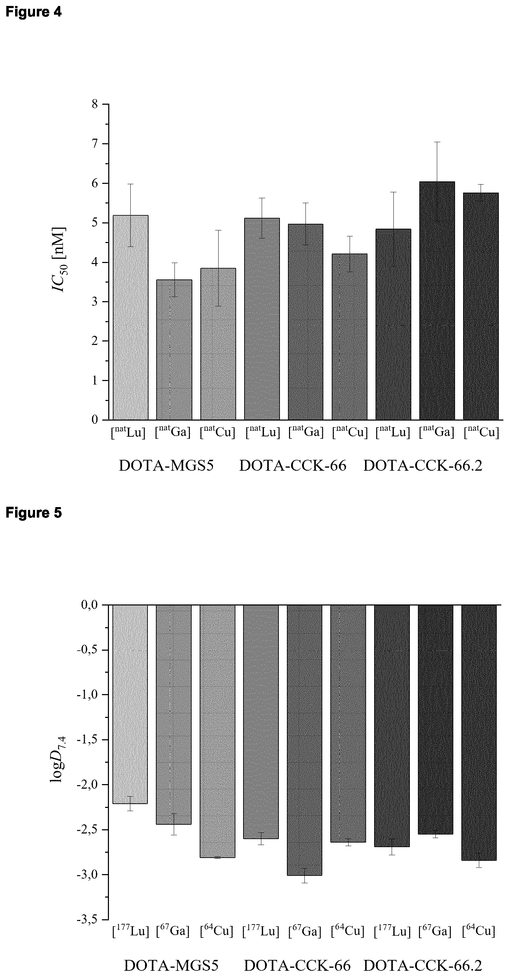

**Fig. 4****:** Schematic representation of the CCK-2R affinity (depicted as half-maximal inhibitory concentration, IC₅₀) of ^{nat}Lu-, ^{nat}Ga- and ^{nat}Cu-labeled DOTA-CCK-66 and -66.2 in comparison with DOTA-MGS5.
**Fig. 5****:** Schematic representation of the lipophilicity (depicted as n-octanol/PBS distribution coefficients, pH = 7.4, log*D*_{7.4}) of DOTA-CCK-66, -66.2 and DOTA-MGS5. The examined compounds were complexated either with [¹⁷⁷Lu]lutetium, [⁶⁷Ga]gallium and [⁶⁴Cu]copper.

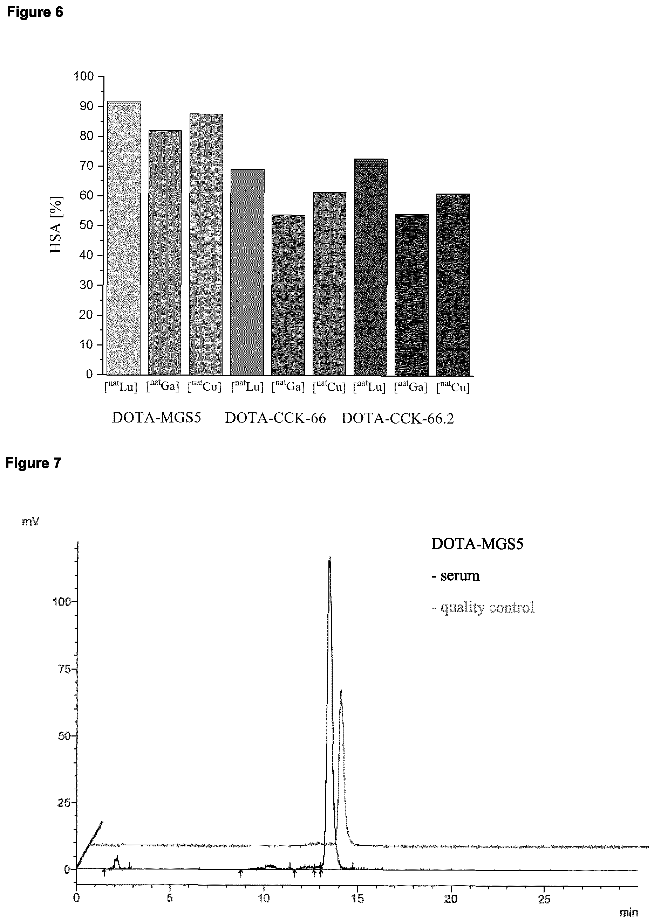

**Fig. *6*****:** *HSA binding (%) of DOTA-CCK-66,* -66.2 *and DOTA-MGS5 complexated either with [^{nat}Lu]lutetium, [^{nat}Ga]gallium or [^{nat}Cu]copper.*

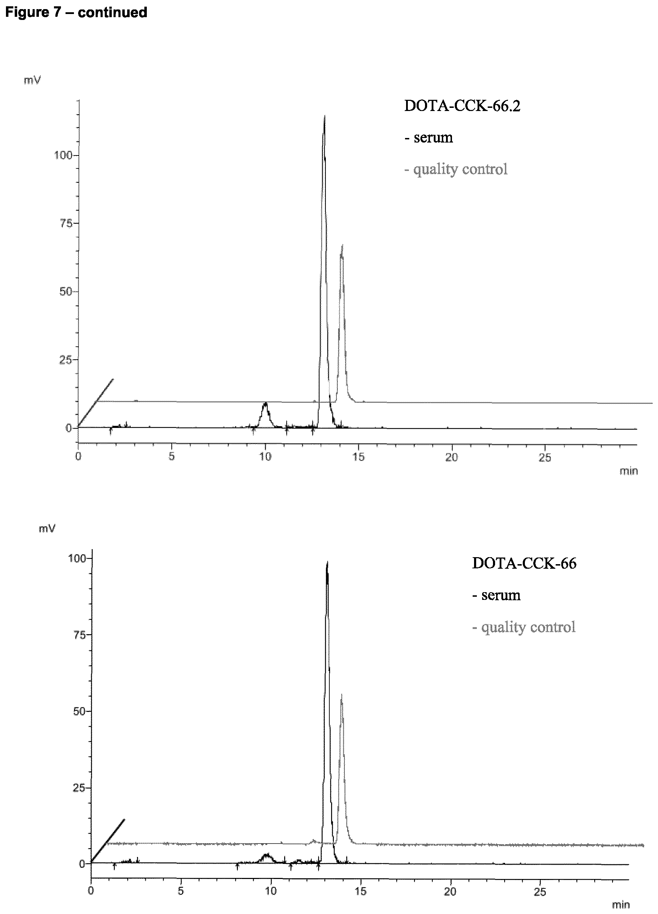

**Fig. 7****:** Representative RP-HPLC chromatograms (10→30% MeCN in H₂O with 0.1% TFA for 5 min and 30→60% MeCN in H₂O with 0.1% TFA for 15 min) of ¹⁷⁷Lu-labeled DOTA-MGS5, -CCK-66 and -CCK-66.2 after incubation in human serum at 37 °C for 72 ± 2 h (black) in comparison with the quality control chromatogram of the compounds directly after ¹⁷⁷Lu-labeling (grey).

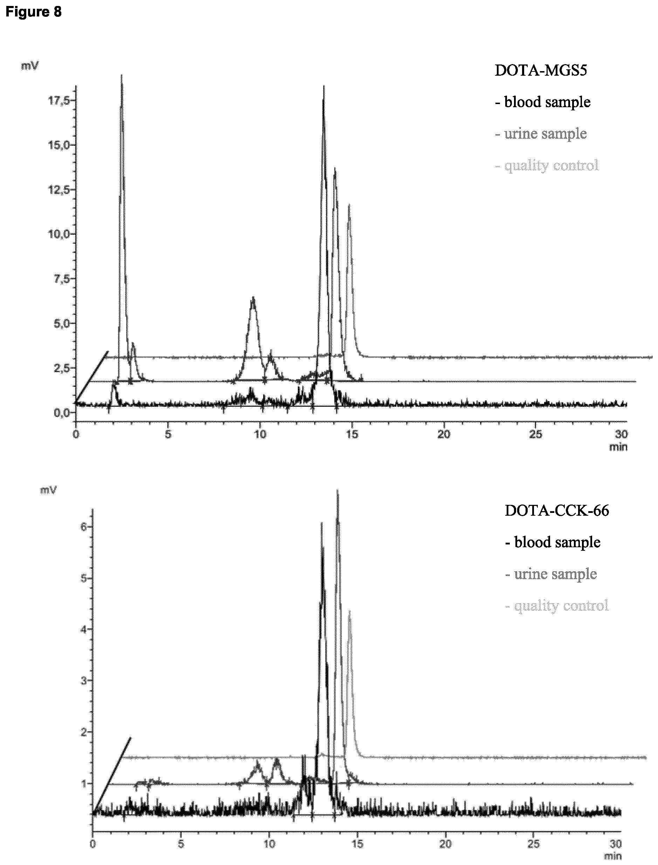

**Fig. 8****:** Representative RP-HPLC chromatograms (10→30% MeCN in H₂O with 0.1% TFA, 5 min and 30→60% MeCN in H₂O with 0.1% TFA, 15 min) of the amount of intact compound of [¹⁷⁷Lu]Lu-DOTA-MGS5 and [¹⁷⁷Lu]Lu-DOTA-CCK-66 (1 nmol, 35 MBq each) in murine serum (black) and urine (dark grey) at 30 min p.i. Reference chromatograms of the compounds are depicted in light grey.

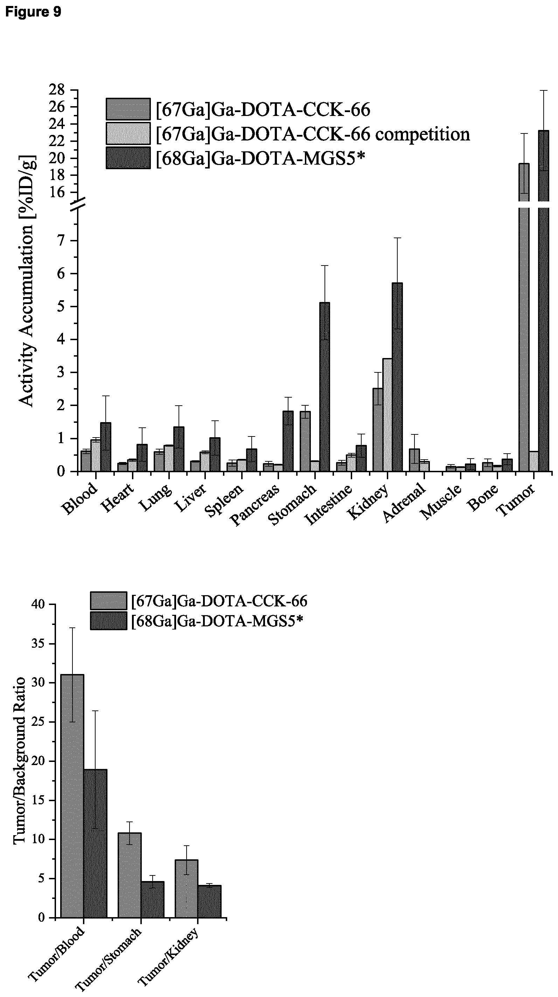

**Fig. 9****:** Biodistribution and competition studies of [⁶⁷Ga]Ga-DOTA-CCK-66 in selected organs (in %ID/g) at 1 h p.i. in AR4-2J tumor-bearing CB17-SCID mice (100 pmol each). For competition studies an excess of [^{nat}Ga]Ga-DOTA-MGS5 (40 nmol) was co-administered. Data is expressed as mean ± SD (n = 4 for biodistribution studies and n = 2 for competition studies). Literature data of [⁶⁸Ga]Ga-DOTA-MGS5 (20 pmol) are depicted for comparison [30]. A comparative analysis of tumor/blood, tumor/stomach and tumor/kidney ratios of the two compounds is depicted on the right.

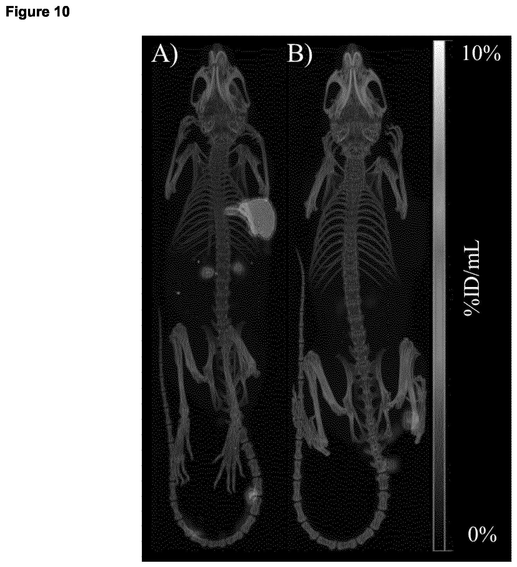

**Fig. 10****:** *A) maximum intensity projection of AR4-2J tumor-bearing CB17-SCID mice injected with [⁶⁷Ga]Ga-DOTA-CCK-66 (100 pmol) at 1 h p.i. B) Representative µSPECT*/*CT image of a competition study of [⁶⁷Ga]Ga-DOTA-CCK-66 (100 pmol) co-administered with [^{nat}Ga]Ga-DOTA-MGS5 (40 nmol) in AR4-2J tumor-bearing CB17-SCID mice at 1 h p.i.*
**Fig. 11****:** *Biodistribution and competition studies of [¹⁷⁷Lu]Lu-DOTA-CCK-66 and [¹⁷⁷Lu]Lu-DOTA-MGS5 in selected organs (in %ID*/*g*) *at 24 h p.i. in AR4-2J tumor-bearing CB17-SCID mice (100 pmol each). For competition studies an excess of [^{nat}Lu]Lu-DOTA-MGS5 (40 nmol) was co-administered. Data is expressed as mean ± SD (n = 4 for biodistribution studies and n = 2 for competition studies). A comparative analysis of tumor*/*blood, tumor*/*stomach and tumor*/*kidney ratios of the two compounds is depicted on the bottom.*

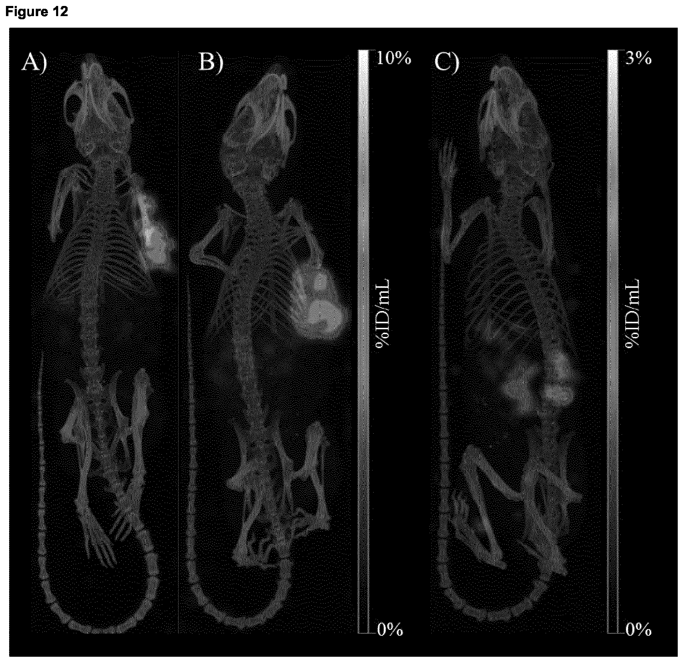

**Fig. 12****:** *Maximum intensity projection of AR4-2J tumor-bearing CB17-SCID mice injected with A) [¹⁷⁷Lu]Lu-DOTA-MGS5 and B) [¹⁷⁷Lu]Lu-DOTA-CCK-66 (100 pmol each). C) Representative µSPECT*/*CT image of a competition study of [¹⁷⁷LuDOTA-CCK-66 (100 pmol) co-administered with [^{nat}Lu]Lu-DOTA-MGS5 (40 nmol) at 24 h p.i.*

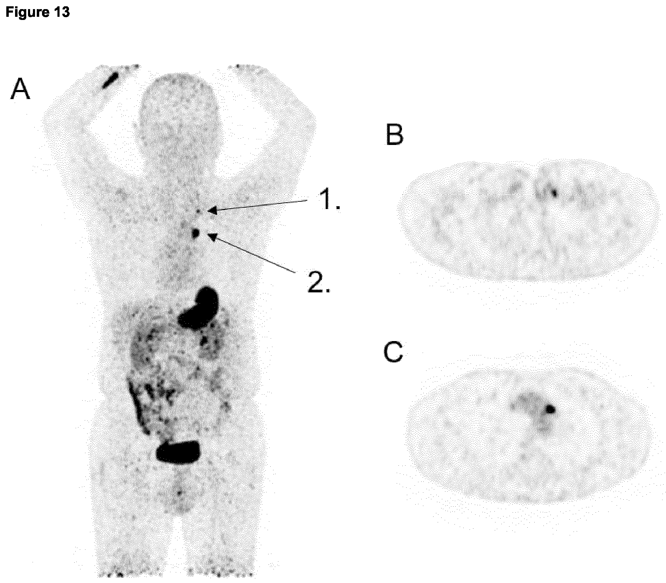

**Fig. 13****:** *(A) PET*/*CT image of patient 1 with MTC using [⁶⁸Ga]Ga-DOTA-CCK-66, which revealed (B) one left retroclavicular lymph node metastasis and (C) a mediastinal metastasis at the aortic arch.*

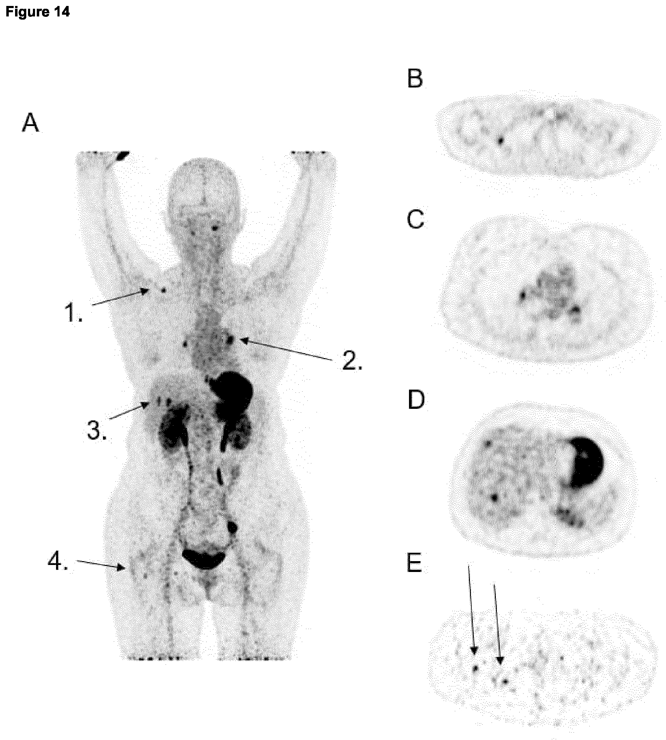

**Fig. 14****:** *(A) PET*/*CT image of patient 2 with MTC using [⁶⁸Ga]Ga-DOTA-CCK-66, which showed (B) a right retroclavicular lymph node metastasis, (C) bilateral hilar lymph node metastases, (D) two liver metastases and (E) bone metastases in the right femur as well as the right os ischii.*

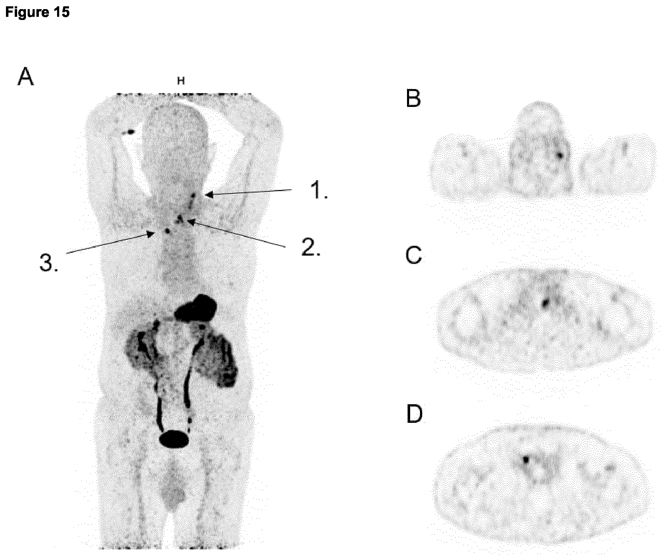

**Fig. 15****:** *(A) PET*/*CT image of patient 3 with MTC using [⁶⁸Ga]Ga-DOTA-CCK-66, which exhibited (B) a lymph node metastasis in the region of the left cervical vascular nerve sheath, (C) a local recurrence in the left thyroid bed and (D) a lymph node metastasis in the upper right mediastinum.*

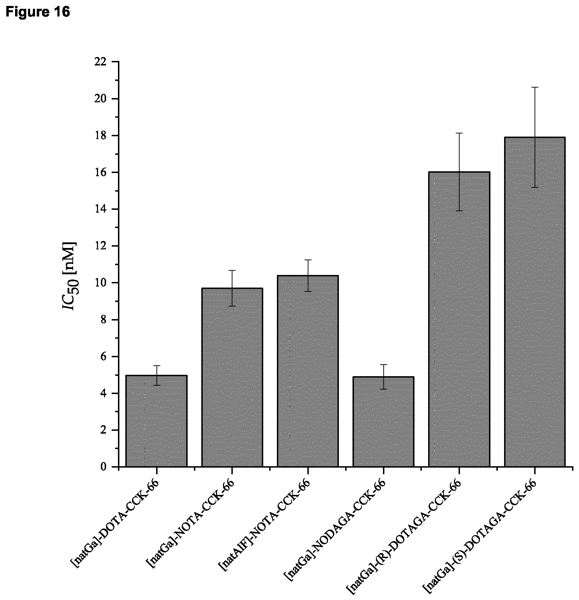

**Fig. 16****:** Schematic representation of CCK-2R affinity (depicted as half-maximal inhibitory concentration, *IC*₅₀) of ^{nat}Lu- and [¹⁹F]AIF-complexated DOTA-CCK-66 analogues containing various chelators (NOTA, NODAGA, (*R*)-DOTAGA and (*S*)-DOTAGA).

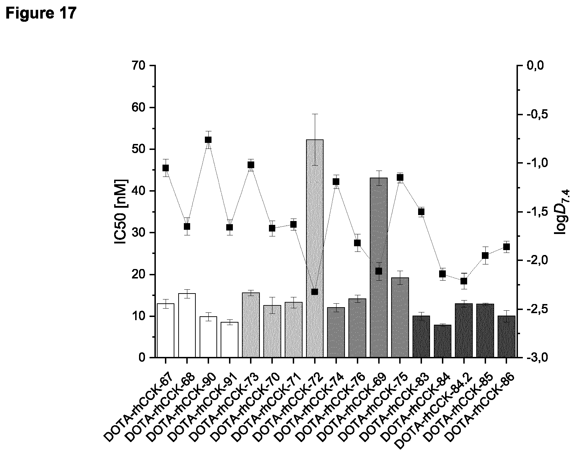

**Fig. 17****:** Schematic representation of the CCK-2R affinity (depicted as half-maximal inhibitory concentration, *IC*₅₀) and lipophilicity (depicted as n-octanol/PBS distribution coefficients, pH = 7.4, log*D*_{7.4}) of [^{nat/177}Lu]Lu-DOTA-rhCCK-67 to -76, [^{nav/177}Lu]Lu-DOTA-rhCCK-83 to -86 and [^{nat/177}Lu]Lu-DOTA-rhCCK-90 to -91 comprising different linker sections (light grey: (PEG)₄, grey: (PEG)₇, dark grey: (PEG)₁₁, white: (cit)₀₋₃-(Hyp)₃₋₈), silicon-based fluoride acceptor moieties (SiFA, SiFA-ipa, SiFAlin, black: log*D*_{7.4}-values) and negative charges (with and without γ-glu in proximity of dap(SiFA)).

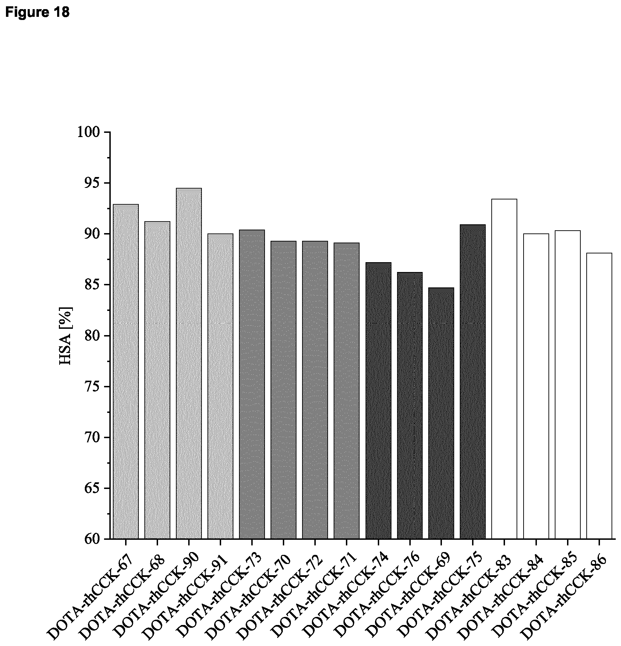

**Fig. 18****:** HSA binding (%) of [^{nat}Lu]Lu-DOTA-rhCCK-67 to -76, [^{nat}Lu]Lu-DOTA-rhCCK-83 to -86 and [^{nat}Lu]Lu-DOTA-rhCCK-90 to -91 comprising different linker sections (light grey: (PEG)₄, grey: (PEG)₇, dark grey: (PEG)₁₁, white: (cit)₀₋₃-(Hyp)₃₋₈), silicon-based fluoride acceptor moieties (SiFA, SiFA-ipa, SiFAlin, black: log*D*_{7.4} values) and negative charges (with and without γ-glu in proximity of dap(SiFA)).

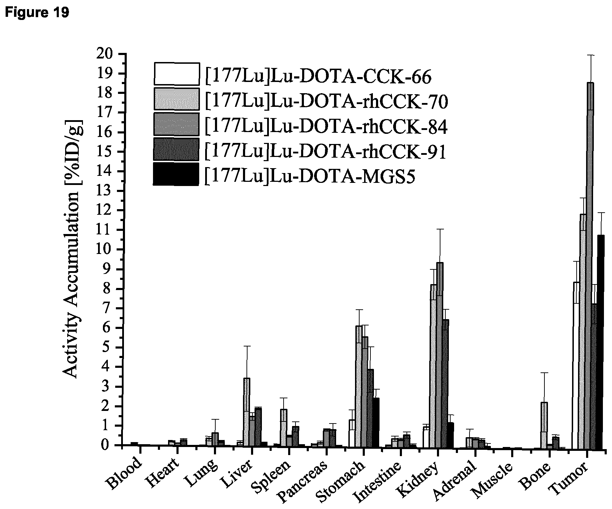

**Fig. 19****:** Biodistribution of [¹⁷⁷Lu]Lu-DOTA-CCK-66 (n = 4), [¹⁷⁷Lu]Lu-DOTA-rhCCK-70 (n = 4), [¹⁷⁷Lu]Lu-DOTA-rhCCK-84 (n = 3), [¹⁷⁷Lu]Lu-DOTA-rhCCK-91 (n = 3) and [¹⁷⁷Lu]Lu-DOTA-MGS5 (n = 4) in selected organs (in %ID/g) at 24 h p.i. in AR4-2J tumor-bearing CB17-SCID mice (100 pmol each). Data is expressed as mean ± SD.

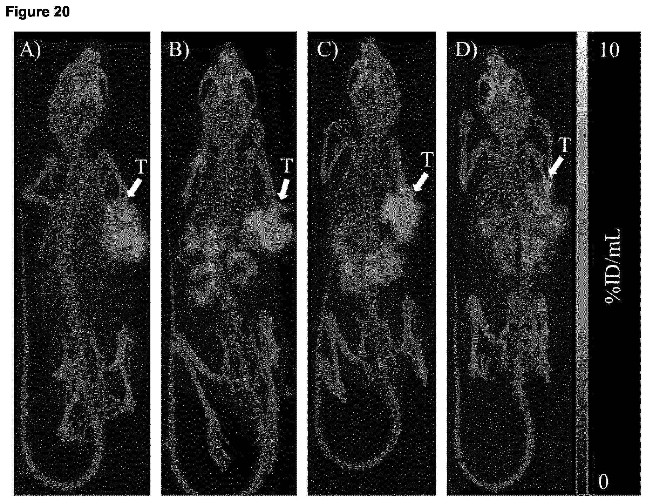

**Fig. 20****:** Maximum intensity projection of AR4-2J tumor-bearing CB17-SCID mice at 24 h p.i. (100 pmol each) injected with A) [¹⁷⁷Lu]Lu-DOTA-CCK-66, B) [¹⁷⁷Lu]Lu-DOTA-rhCCK-70, C) [¹⁷⁷Lu]Lu-DOTA-rhCCK-84 and D) [¹⁷⁷Lu]Lu-DOTA-rhCCK-91.
**Fig. 21****:** Biodistribution of [¹⁸F]F-[^{nat}Lu]Lu-DOTA-rhCCK-84 (1 h p.i.) and [¹⁷⁷Lu]Lu-DOTA-rhCCK-84 (2, 4, 24 h p.i.) in selected organs (in %ID/g) in AR4-2J tumor-bearing CB17-SCID mice (100 pmol each, n = 4). Data is expressed as mean ± SD.
**Fig. 22****:** Biodistribution studies of [¹⁷⁷Lu]Lu-DOTA-rhCCK-84 co-administered with [^{nat}Lu]Lu-DOTA-MGS5 (40 nmol, n = 2, black) or [^{nat}Lu]Lu-DOTA-MG-SiFA-23 (40 nmol, n = 1, grey) in selected organs (in %ID/g) at 24 h p.i. in AR4-2J tumor-bearing CB17-SCID mice (100 pmol each). Data is expressed as mean ± SD.

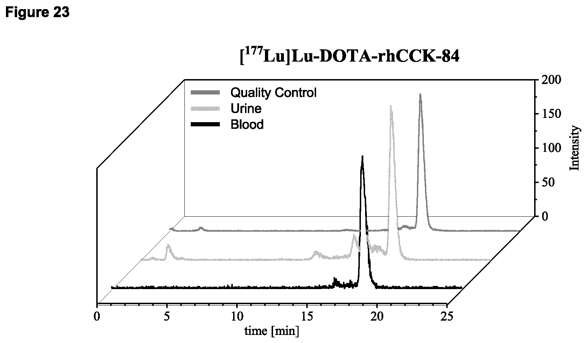

**Fig. 23****:** Representative RP-HPLC chromatograms (10→30% MeCN in H₂O with 0.1% TFA, 5 min and 30→60% MeCN in H₂O with 0.1% TFA, 15 min) of the number of intact compound of [¹⁷⁷Lu]Lu-DOTA-rhCCK-84 (1 nmol, 35 MBq each, n = 3) in murine serum (black) and urine (light grey) at 30 min p.i. Quality control chromatograms of the compounds are depicted in dark grey.

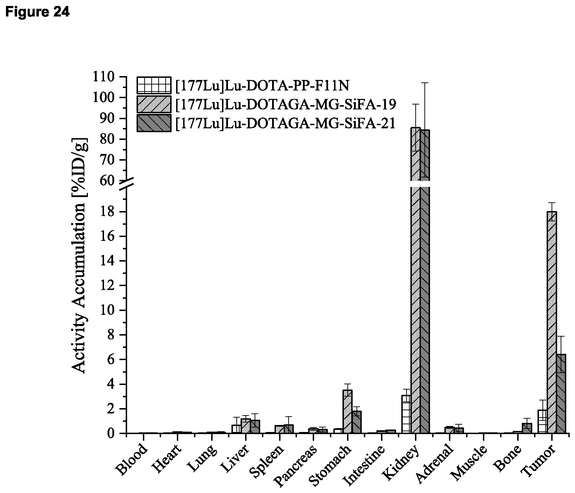

**Fig. 24****:** Biodistribution of [¹⁷⁷Lu]Lu-DOTA-PP-F11N, [¹⁷⁷Lu]Lu-DOTAGA-MG-SiFA-19 and [¹⁷⁷Lu]Lu-DOTAGA-MG-SiFA-21 in selected organs (in %ID/g) at 24 h p.i. in AR4-2J tumor-bearing CB17-SCID mice (100 pmol each). Data is expressed as mean ± SD (n = 4).

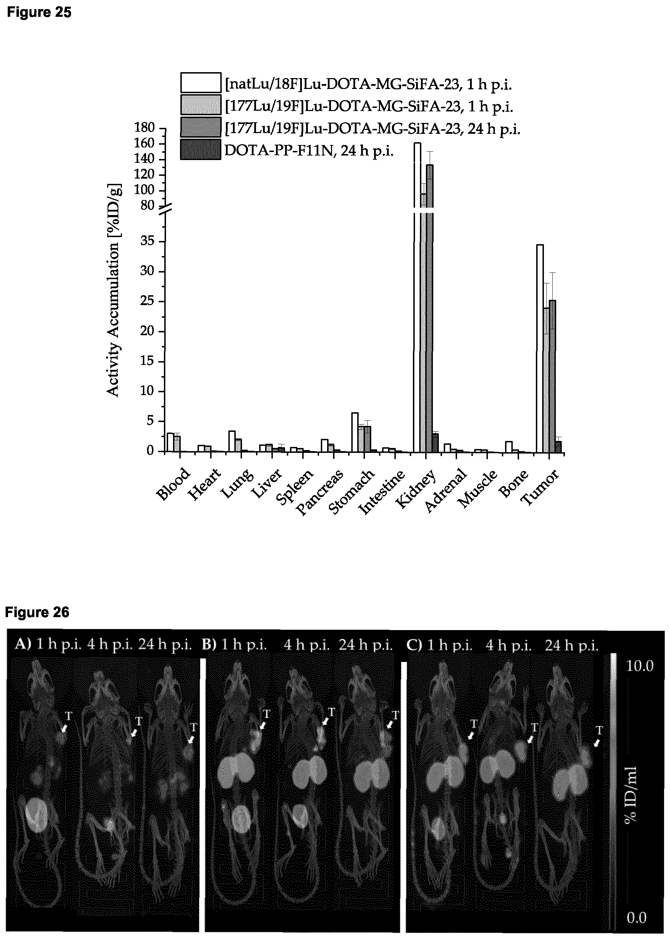

**Fig. 25****:** Biodistribution of [¹⁷⁷Lu]Lu-DOTA-MG-SiFA-23 in selected organs (in %ID/g) at 1 and 24 h p.i. and [^{nat}Lᵤ]Lu-¹⁸F-DOTA-MG-SiFA-23 (n = 1) at 1 h p.i. in AR4-2J tumor-bearing CB17-SCID mice (100 pmol each) in comparison to [¹⁷⁷Lu]Lu-DOTA-PP-F11N at 24 h p.i. Data is expressed as mean ± SD (n = 4).
**Fig. 26****:** Representative *µ*SPECT/CT images of A) [¹⁷⁷Lu]Lu-DOTA-PP-F11N, B) [¹⁷⁷Lu]Lu-DOTAGA-MG-SiFA-21 and C) [¹⁷⁷Lu]Lu-DOTAGA-MG-SiFA-19 at 1, 4 and 24 h p.i. in AR4-2J tumor-bearing CB17- SCID mice (100 pmol each). Tumors (T) are indicated by white arrows.

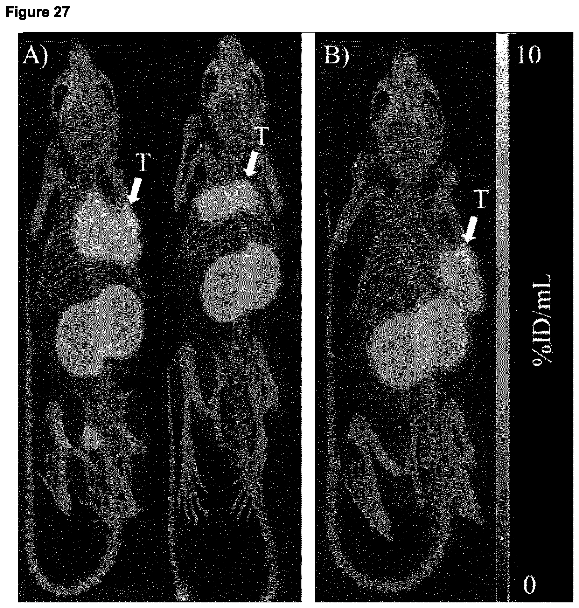

**Fig. 27****:** Representative *µ*SPECT/CT images of A) [¹⁷⁷Lu]Lu-DOTAGA-MG-SiFA-23 at 1 and 24 h p.i. and B) [¹⁸F]F-[^{nat}Lu]Lu-DOTA-MG-SiFA-23 at 1 h p.i. in AR4-2J tumor-bearing CB17-SCID mice (100 pmol each). Tumors (T) are indicated by white arrows.

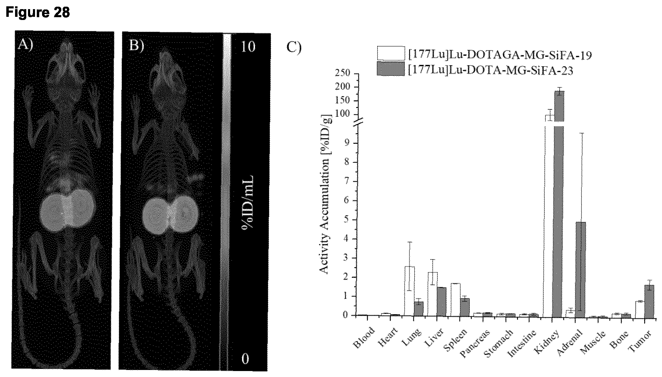

**Fig. 28****:** Representative *µ*SPECT/CT images of A) [¹⁷⁷Lu]Lu-DOTAGA-MG-SiFA-19 and B) [¹⁷⁷Lu]Lu-DOTA-MG-SiFA-23 at 24 h p.i. in AR4-2J tumor-bearing CB17- SCID mice (100 pmol each, n = 1) co-injected with [^{nat}Lu]Lu-DOTA-MGS5 (40 nmol each). C) Biodistribution of [¹⁷⁷Lu]Lu-DOTAGA-MG-SiFA-19 and [¹⁷⁷Lu]Lu-DOTA-MG-SiFA-23 in selected organs (in %ID/g) at 24 h p.i. in AR4-2J tumor-bearing CB17-SCID mice (100 pmol each, n = 2) co-injected with [^{nat}Lu]Lu-DOTA-MGS5 (40 nmol each). Data is expressed as mean ± SD.

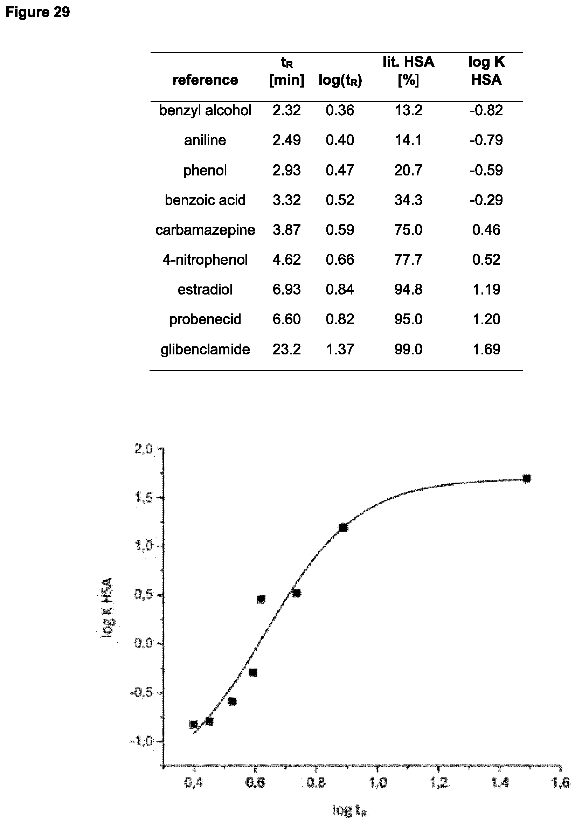

**Fig. 29****:** Example of a sigmoidal plot, showing the correlation between human serum albumin (HSA) binding of selected reference substances and their retention time (*t*_{R}) on a Chiralpak HSA column. HSA binding values of the reference substances were previously published in the literature (lit. HSA [%]) and the respective logarithmic value of the affinity constant (log K HSA) was calculated [31,32].

The examples illustrate the invention.

### Example 1 - N-terminal tetrapeptide of Minigastrin

### 1.1. Overview of the synthesized CCK-2R ligands

### Reference compounds

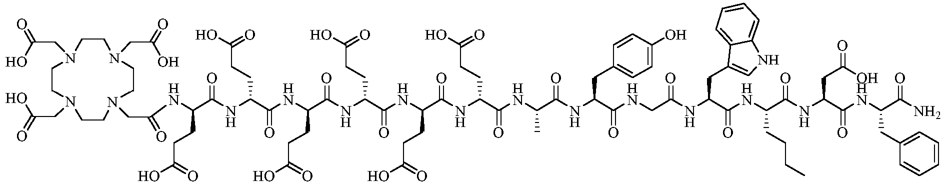

**DOTA-PP-F11N:** RP-HPLC (10→90% MeCN in H₂O with 0.1% TFA, 15 min, λ = 220 nm): *t*_{R} = 7.6 min, K' = 3.5; MS (ESI, positive): m/z calculated for C₉₀H₁₂₃N₁₉O₃₅: 2029.8, found: m/z = 1016.4 [M+2H]²⁺.

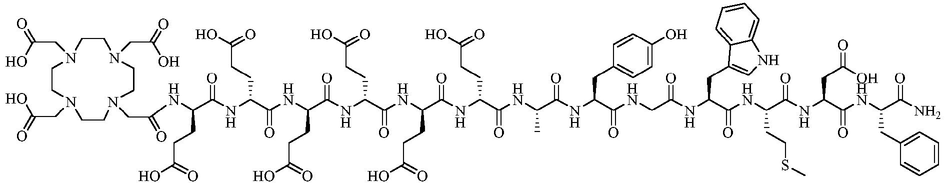

**CP04:** RP-HPLC (10→90% MeCN in H₂O with 0.1% TFA, 15 min, λ = 220 nm): *t*_{R} = 7.2 min, *K'* = 3.3; MS (ESI, positive): m/z calculated for C₈₉H₁₂₁N₁₉O₃₅S: 2047.8, found: m/z = 1024.2 [M+2H]²⁺.

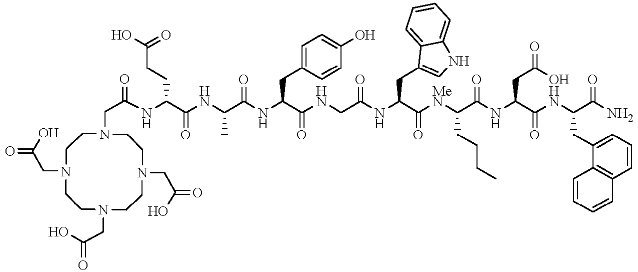

**DOTA-MGS5:** RP-HPLC (10→90% MeCN in H₂O with 0.1% TFA, 15 min, λ = 220 nm): *t*_{R} = 9.2 min; MS (ESI, positive): m/z calculated for C₇₀H₉₂N₁₄O₂₀: 1448.7, found: m/z = 1449.4 [M+H]⁺, 725.3 [M+2H]²⁺.

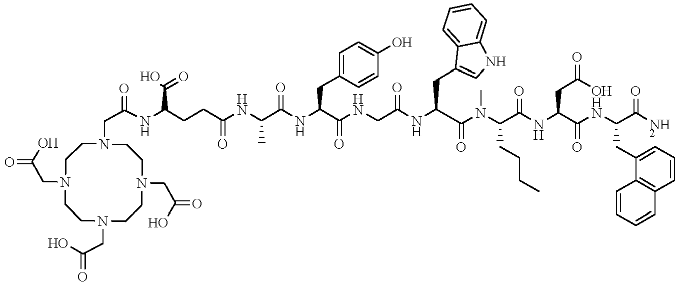

**DOTA-γ-MGS5:** RP-HPLC (10→70% MeCN in H₂O with 0.1% TFA, 15 min, λ = 220 nm): *t*_{R} = 10.9 min; MS (ESI, positive): m/z calculated for C₇₀H₉₂N₁₄O₂₀: 1448.7, found: m/z = 1449.6 [M+H]⁺, 725.5 [M+2H]²⁺.

### Compounds containing a modified tetrapeptidic motif

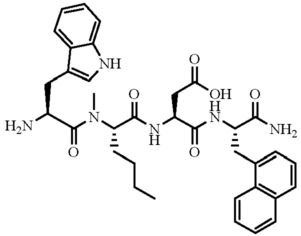

***H*-Trp-(N-Me)Nle-Asp-1-Nal-NH₂:** RP-HPLC (10→70% MeCN in H₂O with 0.1% TFA, 15 min, λ = 220 nm): *t*_{R} = 11.5 min; MS (ESI, positive): m/z calculated for C₃₅H₄₂N₆O₆: 642.8, found: m/z = 643.2 [M+H]⁺.

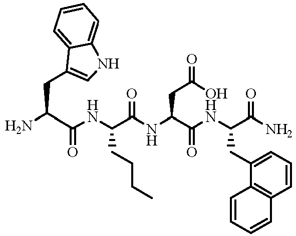

***H*-Trp-Nle-Asp-1-Nal-NH₂:** RP-HPLC (10→70% MeCN in H₂O with 0.1% TFA, 15 min, λ = 220 nm): *t*_{R} = 10.6 min; MS (ESI, positive): m/z calculated for C₃₄H₄₀N₆O₆: 628.7, found: m/z = 628.3 [M+H]⁺, 1255.6 [2M+H]⁺.

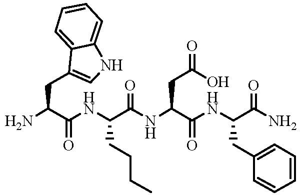

***H*-Trp-Nle-Asp-Phe-NH₂:** RP-HPLC (10→70% MeCN in H₂O with 0.1% TFA, 15 min, λ = 220 nm): *t*_{R} = 9.2 min; MS (ESI, positive): m/z calculated for C₃₀H₃₈N₆O₆: 578.7, found: m/z = 579.2 [M+H]⁺, 1157.3 [2M+H]⁺.

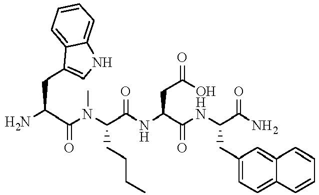

***H*-Trp-(N-Me)Nle-Asp-2-Nal-NH₂:** RP-HPLC (10→70% MeCN in H₂O with 0.1% TFA, 15 min, λ = 220 nm): *t*_{R} = 11.5 min; MS (ESI, positive): m/z calculated for C₃₅H₄₂N₆O₆: 642.8, found: m/z = 643.3 [M+H]⁺, 1285.9 [2M+H]⁺.

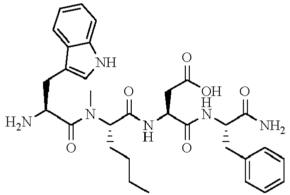

***H*-Trp-(N-Me)Nle-Asp-Phe-NH₂:** RP-HPLC (10→70% MeCN in H₂O with 0.1% TFA, 15 min, λ = 220 nm): *t*_{R} = 10.2 min; MS (ESI, positive): m/z calculated for C₃₁H₄₀N₆O₆: 592.7, found: m/z = 593.3 [M+H]⁺, 1186.3 [2M+H]⁺.

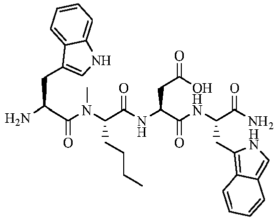

***H*-Trp-(N-Me)Nle-Asp-Trp-NH₂:** RP-HPLC (10→70% MeCN in H₂O with 0.1% TFA, 15 min, λ = 220 nm): *t*_{R} = 10.3 min; MS (ESI, positive): m/z calculated for C₃₃H₄₁N₇O₆: 631.7, found: m/z = 632.3 [M+H]⁺, 1264.8 [2M+H]⁺.

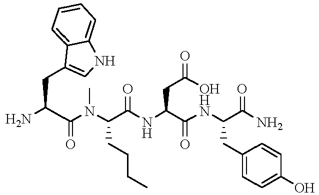

***H*-Trp-(N-Me)Nle-Asp-Tyr-NH₂:** RP-HPLC (10→70% MeCN in H₂O with 0.1% TFA, 15 min, λ = 220 nm): *t*_{R} = 8.79 min; MS (ESI, positive): m/z calculated for C₃₁H₄₀N₆O₇: 608.7, found: m/z = 609.3 [M+H]⁺, 1217.6 [2M+H]⁺.

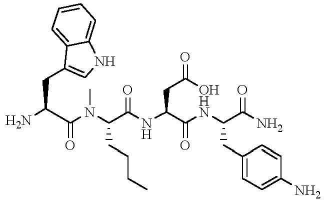

***H*-Trp-(N-Me)Nle-Asp-p-amino-Phe-NH₂:** RP-HPLC (10→70% MeCN in H₂O with 0.1% TFA, 15 min, λ = 220 nm): *t*_{R} = 7.34 min; MS (ESI, positive): m/z calculated for C₃₁H₄₁N₇O₆: 607.3, found: m/z = 608.2 [M+H]⁺, 1215.6 [2M+H]⁺.

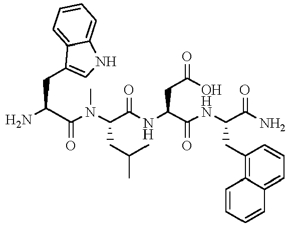

***H*-Trp-(N-Me)Leu-Asp-1-Nal-NH₂:** RP-HPLC (10→70% MeCN in H₂O with 0.1% TFA, 15 min, λ = 220 nm): *t*_{R} = 11.4 min; MS (ESI, positive): m/z calculated for C₃₅H₄₂N₆O₆: 642.3, found: m/z = 643.7 [M+H]⁺, 1285.1 [2M+H]⁺.

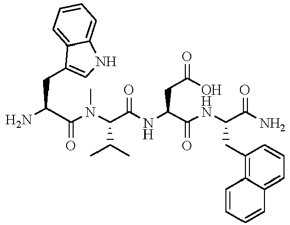

**H-Trp-(N-Me)Val-Asp-1-Nal-NH₂:** RP-HPLC (10→70% MeCN in H₂O with 0.1% TFA, 15 min, λ = 220 nm): *t*_{R} = 10.4 min; MS (ESI, positive): m/z calculated for C₃₄H₄₀N₆O₆: 628.3, found: m/z = 629.3 [M+H]⁺, 1257.0 [2M+H]⁺.

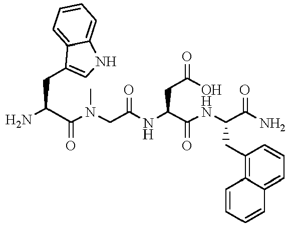

***H*-Trp-(N-Me)Gly-Asp-1-Nal-NH₂:** RP-HPLC (10→70% MeCN in H₂O with 0.1% TFA, 15 min, λ = 220 nm): *t*_{R} = 9.48 min; MS (ESI, positive): m/z calculated for C₃₁H₃₄N₆O₆: 586.3, found: m/z = 587.7 [M+H]⁺.

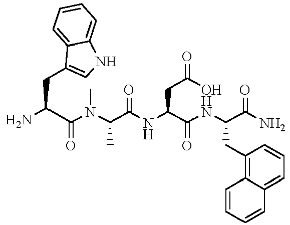

***H*-Trp-(N-Me)Ala-Asp-1-Nal-NH₂:** RP-HPLC (10→70% MeCN in H₂O with 0.1% TFA, 15 min, λ = 220 nm): *t*_{R} = 9.92 min; MS (ESI, positive): m/z calculated for C₃₂H₃₆N₆O₆: 600.3, found: m/z = 601.7 [M+H]⁺, 1202.9 [2M+H]⁺.

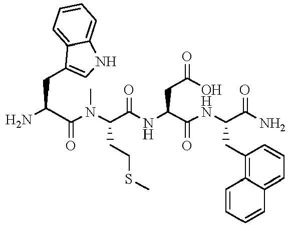

***H*-Trp-(N-Me)Met-Asp-1-Nal-NH₂:** RP-HPLC (10→70% MeCN in H₂O with 0.1% TFA, 15 min, λ = 220 nm): t_{R} = 11.0 min; MS (ESI, positive): m/z calculated for C₃₄H₄₀N₆O₆S: 660.3, found: m/z = 661.8 [M+H]⁺.

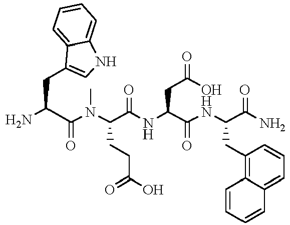

***H*-Trp-(N-Me)Glu-Asp-1-Nal-NH₂:** RP-HPLC (10→70% MeCN in H₂O with 0.1% TFA, 15 min, λ = 220 nm): *t*_{R} = 9.26 min; MS (ESI, positive): m/z calculated for C₃₄H₃₈N₆O₈: 658.3, found: m/z = 659.5 [M+H]⁺.

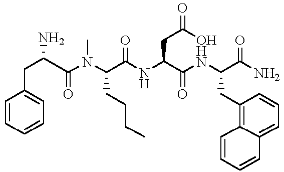

***H*-Phe-(N-Me)Nle-Asp-1-Nal-NH₂:** RP-HPLC (10→70% MeCN in H₂O with 0.1% TFA, 15 min, λ = 220 nm): *t*_{R} = 11.6 min; MS (ESI, positive): m/z calculated for C₃₃H₄₁N₅O₆: 603.3, found: m/z = 604.6 [M+H]⁺, 1208.6 [2M+H]⁺.

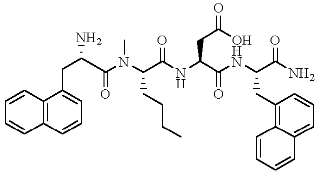

***H*-1-Nal-(N-Me)Nle-Asp-1-Nal-NH₂:** RP-HPLC (10→70% MeCN in H₂O with 0.1% TFA, 15 min, λ = 220 nm): *t*_{R} = 12.9 min; MS (ESI, positive): m/z calculated for C₃₇H₄₃N₅O₆: 653.3, found: m/z = 654.0 [M+H]⁺.

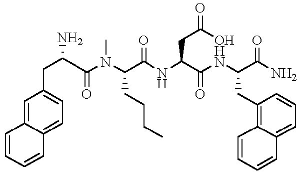

***H*-2-Nal-(N-Me)Nle-Asp-1-Nal-NH₂:** RP-HPLC (10→70% MeCN in H₂O with 0.1% TFA, 15 min, λ = 220 nm): *t*_{R} = 12.8 min; MS (ESI, positive): m/z calculated for C₃₇H₄₃N₅O₆: 653.3, found: m/z = 654.3 [M+H]⁺.

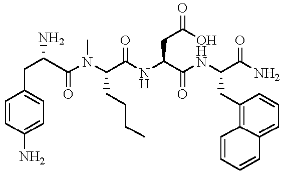

***H*-p-amino-Phe-(N-Me)Nle-Asp-1-Nal-NH₂:** RP-HPLC (10→70% MeCN in H₂O with 0.1% TFA, 15 min, λ = 220 nm): *t*_{R} = 8.65 min; MS (ESI, positive): m/z calculated for C₃₃H₄₂N₆O₆: 618.3, found: m/z = 619.2 [M+H]⁺, 1237.5 [2M+H]⁺.

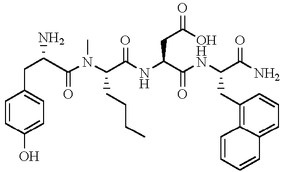

***H*-Tyr-(N-Me)Nle-Asp-1-Nal-NH₂:** RP-HPLC (10→70% MeCN in H₂O with 0.1% TFA, 15 min, λ = 220 nm): *t*_{R} = 10.3 min; MS (ESI, positive): m/z calculated for C₃₃H₄₁N₅O₇: 619.3, found: m/z = 620.3 [M+H]⁺, 1240.2 [2M+H]⁺.

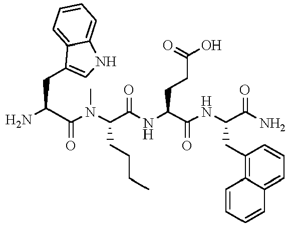

***H*-Trp-(N-Me)Nle-Glu-1-Nal-NH₂:** RP-HPLC (10→70% MeCN in H₂O with 0.1% TFA, 15 min, λ = 220 nm): *t*_{R} = 11.5 min; MS (ESI, positive): m/z calculated for C₃₆H₄₄N₆O₆: 656.3, found: m/z = 656.9 [M+H]⁺, 1314.3 [2M+H]⁺.

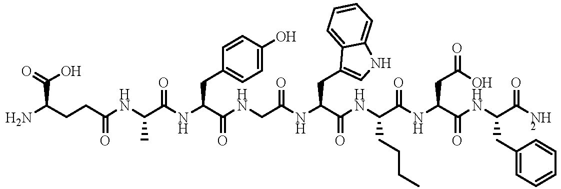

***H*-*γ*-glu-Ala-Tyr-Gly-Trp-Nle-Asp-Phe-NH₂ (CCK-32):** RP-HPLC (10→70% MeCN in H₂O with 0.1% TFA, 15 min, λ = 220 nm): *t*_{R} = 9.7 min; MS (ESI, positive): m/z calculated for C₄₉H₆₂N₁₀O₁₃: 998.5, found: m/z = 998.7 [M+H]⁺.

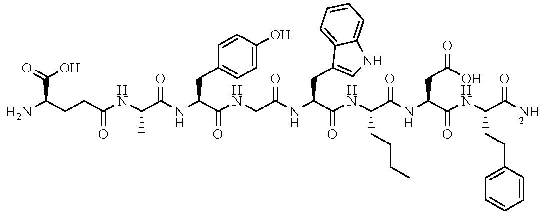

*H*-*γ*-glu-Ala-Tyr-Gly-Trp-Nle-Asp-(homo-Phe)-NH₂ (CCK-33): RP-HPLC (10→70% MeCN in H₂O with 0.1% TFA, 15 min, λ = 220 nm): *t*_{R} = 10.2 min; MS (ESI, positive): m/z calculated for C₅₀H₆₄N₁₀O₁₃: 1012.5, found: m/z = 1013.6 [M+H]⁺, 507.4 [M+2H]²⁺.

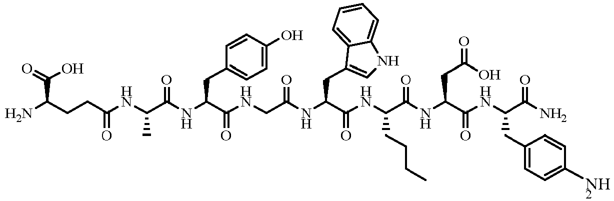

***H*-*γ*-glu-Ala-Tyr-Gly-Trp-Nle-Asp-(p-amino-Phe)-NH₂** (CCK-34): RP-HPLC (10→70% MeCN in H₂O with 0.1% TFA, 15 min, λ = 220 nm): *t*_{R} = 7.5 min; MS (ESI, positive): m/z calculated for C₄₉H₆₃N₁₁O₁₃: 1014.1, found: m/z = 1014.5 [M+H]⁺, 507.9 [M+2H]²⁺.

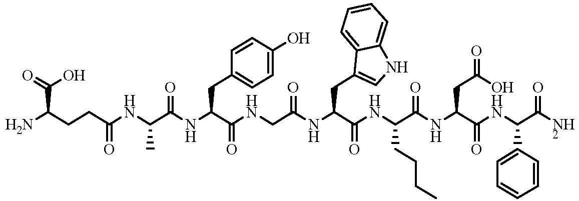

**H*-γ*-glu-Ala-Tyr-Gly-Trp-Nle-Asp-Phg-NH₂** (CCK-35): RP-HPLC (10→70% MeCN in H₂O with 0.1% TFA, 15 min, λ = 220 nm): *t*_{R} = 9.2 min; MS (ESI, positive): m/z calculated for C₄₈H₆₀N₁₀O₁₃: 984.4, found: m/z = 985.6 [M+H]⁺, 1971.4 [2M+H]⁺.

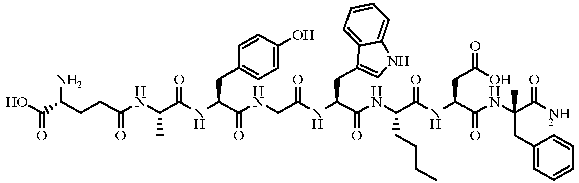

***H*-*γ*-glu-Ala-Tyr-Gly-Trp-Nle-Asp-(*α*-Me)Phe-NH₂ (CCK-36):** RP-HPLC (10→70% MeCN in H₂O with 0.1% TFA, 15 min, λ = 220 nm): *t*_{R} = 9.2 min; MS (ESI, positive): m/z calculated for C₅₀H₆₄N₁₀O₁₃: 1012.5, found: m/z = 1013.3 [M+H]⁺.

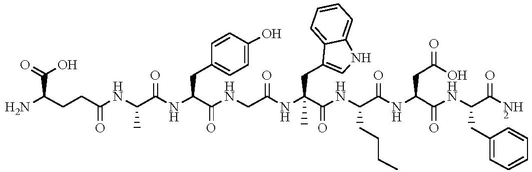

**H-*γ*-glu-Ala-Tyr-Gly-(*α*-Me)Trp-Nle-Asp-Phe-NH₂** (CCK-37): RP-HPLC (10→70% MeCN in H₂O with 0.1% TFA, 15 min, λ = 220 nm): *t*_{R} = 9.9 min; MS (ESI, positive): m/z calculated for C₅₀H₆₄N₁₀O₁₃: 1013.2, found: m/z = 1013.2 [M+H]⁺, 507.3 [M+2H]²⁺.

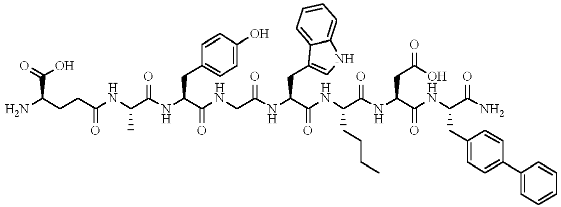

*H*-*γ*-glu-Ala-Tyr-Gly-Trp-Nle-Asp-Bip-NH₂ (CCK-38): RP-HPLC (10→70% MeCN in H₂O with 0.1% TFA, 15 min, λ = 220 nm): *t*_{R} = 11.3 min; MS (ESI, positive): m/z calculated for C₅₅H₆₆N₁₀O₁₃: 1074.5, found: m/z = 1075.1 [M+H]⁺, 565.2 [M+2H]²⁺.

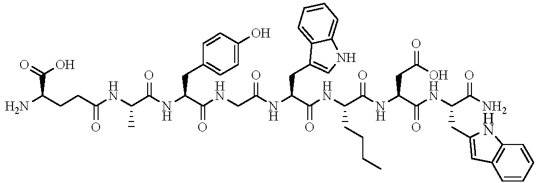

***H*-*γ*-glu-Ala-Tyr-Gly-Trp-Nle-Asp-Trp-NH₂** (CCK-39): RP-HPLC (10→70% MeCN in H₂O with 0.1% TFA, 15 min, λ = 220 nm): *t*_{R} = 9.7 min; MS (ESI, positive): m/z calculated for C₅₁H₆₃N₁₁O₁₃: 1037.5, found: m/z = 1038.2 [M+H]⁺, 519.8 [M+2H]²⁺.

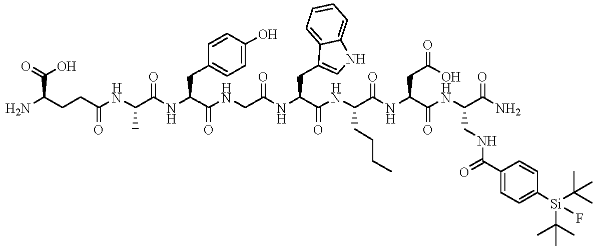

***H*-*γ*-glu-Ala-Tyr-Gly-Trp-Nle-Asp-Dap(SiFA)-NH₂** (CCK-48): RP-HPLC (10→70% MeCN in H₂O with 0.1% TFA, 15 min, λ = 220 nm): *t*_{R} = 14.2 min; MS (ESI, positive): m/z calculated for C₅₈H₈₀FN₁₁O₁₄Si: 1201.6, found: m/z = 1202.8 [M+H]⁺, 602.2 [M+2H]²⁺.

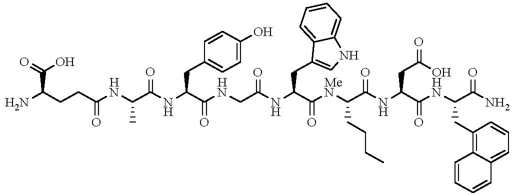

***H*-*γ*-glu-Ala-Tyr-Gly-Trp-(N-Me)Nle-Asp-1-Nal-NH₂ (CCK-49):** RP-HPLC (10→70% MeCN in H₂O with 0.1% TFA, 15 min, λ = 220 nm): *t*_{R} = 11.4 min; MS (ESI, positive): m/z calculated for C₅₄H₆₆N₁₀O₁₃: 1062.5, found: m/z = 1062.8 [M+H]⁺.

### Glycine scan derivatives

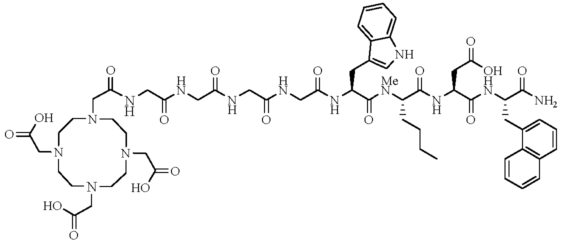

**DOTA-(Gly)₄-Trp-(N-Me)Nle-Asp-1-Nal-NH₂ (DOTA-CCK-55):** RP-HPLC (10→70% MeCN in H₂O with 0.1% TFA, 15 min, λ = 220 nm): *t*_{R} = 10.6 min; MS (ESI, positive): m/z calculated for C₅₉H₈₀N₁₄O₁₇: 1201.6, found: m/z = 1257.0 [M+H]⁺, 629.0 [M+2H]²⁺.

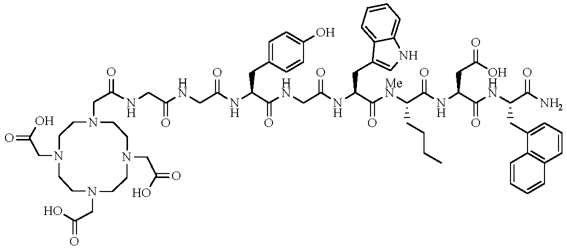

**DOTA-(Gly)₂-Tyr-Gly-Trp-(N-Me)NIe-Asp-1-Nal-NH₂ (DOTA-CCK-56):** RP-HPLC (10→70% MeCN in H₂O with 0.1% TFA, 15 min, λ = 220 nm): *t*_{R} = 11.0 min; MS (ESI, positive): m/z calculated for C₅₆H₈₆N₁₄O₁₈: 1362.6, found: m/z = 1363.1 [M+H]⁺, 682.0 [M+2H]²⁺.

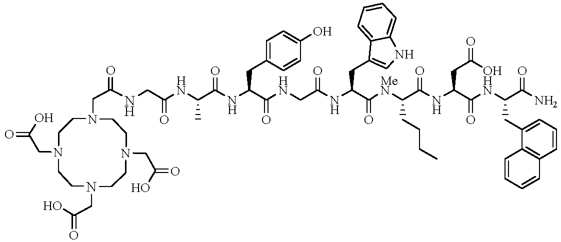

**DOTA-Gly-Ala-Tyr-Gly-Trp-(N-Me)Nle-Asp-1-Nal-NH₂ (DOTA-CCK-57):** RP-HPLC (10→70% MeCN in H₂O with 0.1% TFA, 15 min, λ = 220 nm): *t*_{R} = 11.0 min; MS (ESI, positive): m/z calculated for C₅₇H₈₈N₁₄O₁₈: 1376.6, found: m/z = 1377.1 [M+H]⁺, 689.1 [M+2H]²⁺.

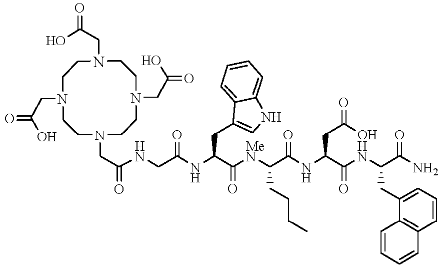

**DOTA-Gly-Trp-(N-Me)Nle-Asp-1-Nal-NH₂ (DOTA-CCK-58):** RP-HPLC (10→70% MeCN in H₂O with 0.1% TFA, 15 min, λ = 220 nm): *t*_{R} = 10.9 min; MS (ESI, positive): m/z calculated for C₅₃H₇₁N₁₁O₁₄: 1085.5, found: m/z = 1086.6 [M+H]⁺, 544.0 [M+2H]²⁺.

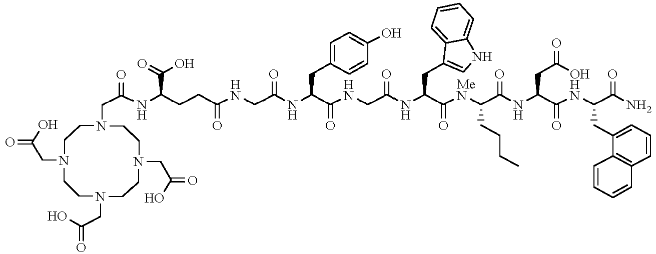

**DOTA-*γ*-glu-Gly-Tyr-Gly-Trp-(N-Me)Nle-Asp-1-Nal-NH₂ (DOTA-CCK-59):** RP-HPLC (10→70% MeCN in H₂O with 0.1% TFA, 15 min, λ = 220 nm): *t*_{R} = 10.8 min; MS (ESI, positive): m/z calculated for C₆₉H₉₀N₁₄O₂₀: 1434.7, found: m/z = 1436.6 [M+H]⁺, 719.0 [M+2H]²⁺.

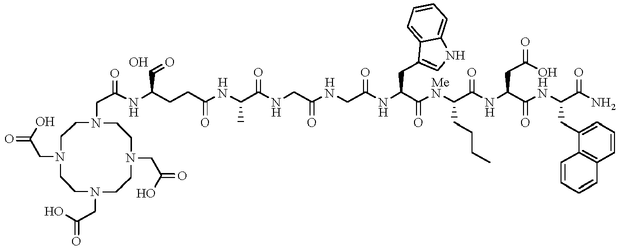

**DOTA-*γ*-glu-Ala-(Gly)₂-Trp-(N-Me)Nle-Asp-1-Nal-NH₂ (DOTA-CCK-60):** RP-HPLC (10→70% MeCN in H₂O with 0.1% TFA, 15 min, λ = 220 nm): *t*_{R} = 10.6 min; MS (ESI, positive): m/z calculated for C₅₃H₈₆N₁₄O₁₉: 1342.6, found: m/z = 1343.9 [M+H]⁺, 672.7 [M+2H]²⁺.

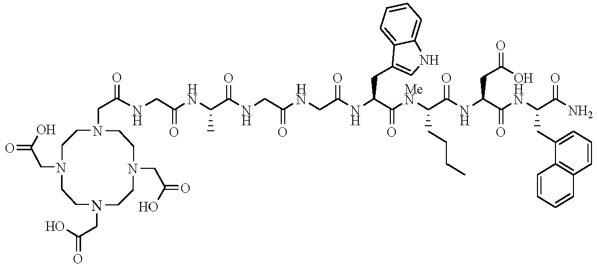

**DOTA-Gly-Ala-(Gly)₂-Trp-(N-Me)Nle-Asp-1-Nal-NH₂ (DOTA-CCK-61):** RP-HPLC (10→70% MeCN in H₂O with 0.1% TFA, 15 min, λ = 220 nm): *t*_{R} = 10.6 min; MS (ESI, positive): m/z calculated for C₆₀H₈₂N₁₄O₁₇: 1270.6, found: m/z = 1272.4 [M+H]⁺, 636.9 [M+2H]²⁺.

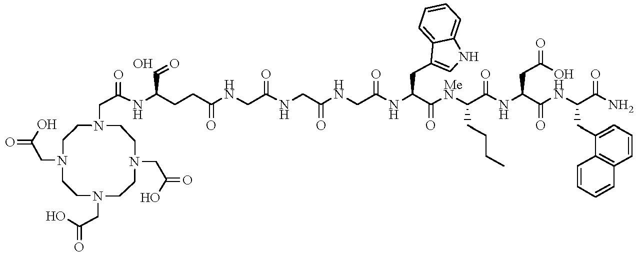

**DOTA-γ-glu-(Gly)₃-Trp-(N-Me)Nle-Asp-1-Nal-NH₂ (DOTA-CCK-62):** RP-HPLC (10→70% MeCN in H₂O with 0.1% TFA, 15 min, λ = 220 nm): *t*_{R} = 10.5 min; MS (ESI, positive): m/z calculated for C₆₂H₈₄N₁₄O₁₉: 1329.4, found: m/z = 1330.5 [M+H]⁺, 665.8 [M+2H]²⁺.

### Compounds containing a PEG linker

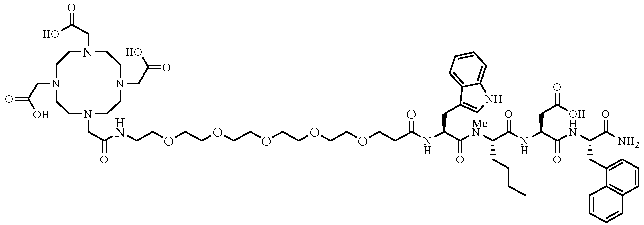

**DOTA-(PEG)₄-Trp-(N-Me)Nle-Asp-1-Nal-NH₂ (DOTA-CCK-50):** RP-HPLC (10→70% MeCN in H₂O with 0.1% TFA, 15 min, λ = 220 nm): *t*_{R} = 11.0 min; MS (ESI, positive): m/z calculated for C₆₄H₉₃N₁₁O₁₉: 1319.7, found: m/z = 1319.7 [M+H]⁺, 659.9 [M+2H]²⁺.

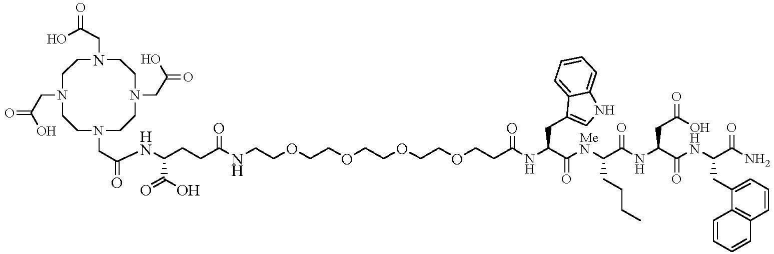

**DOTA-γ-glu-(PEG)₃-Trp-(N-Me)Nle-Asp-1-Nal-NH₂ (DOTA-CCK-66):** RP-HPLC (10→70% MeCN in H₂O with 0.1% TFA, 15 min, λ = 220 nm): *t*_{R} = 10.8 min; MS (ESI, positive): m/z calculated for C₆₇H₉₆N₁₂O₂₁: 1405.6, found: m/z = 1405.3 [M+H]⁺, 702.8 [M+2H]²⁺.

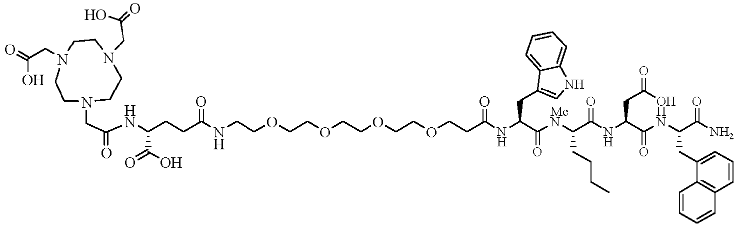

**NOTA-γ-glu-(PEG)₃-Trp-(N-Me)Nle-Asp-1-Nal-NH₂ (NOTA-CCK-66):** RP-HPLC (10→70% MeCN in H₂O with 0.1% TFA, 15 min, λ = 220 nm): *t*_{R} = 11.5 min; MS (ESI, positive): m/z calculated for C₆₃H₈₉N₁₁O₁₉: 1303.6, found: m/z = 1304.0 [M+H]⁺, 652.9 [M+2H]²⁺.

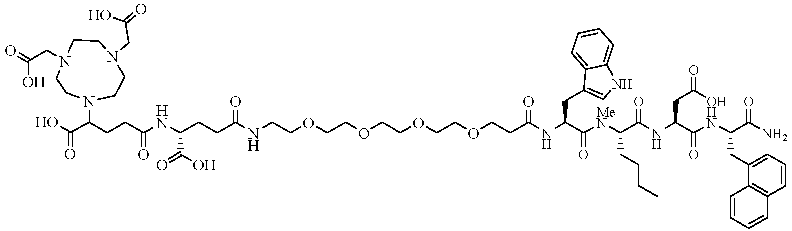

**NODAGA-γ-glu-(PEG)₃-Trp-(N-Me)Nle-Asp-1-Nal-NH₂ (NODAGA-CCK-66):** RP-HPLC (10→70% MeCN in H₂O with 0.1% TFA, 15 min, λ = 220 nm): *t*_{R} = 11.4 min; MS (ESI, positive): m/z calculated for C₆₆H₉₃N₁₁O₂₁: 1376.5, found: m/z = 1375.9 [M+H]⁺, 689.2 [M+2H]²⁺.

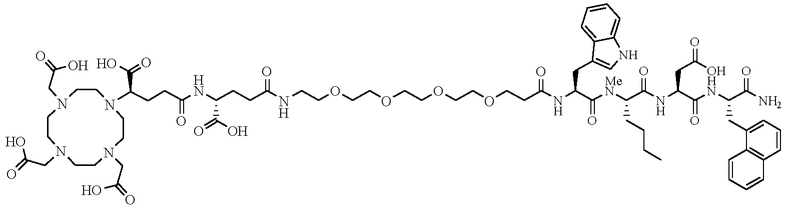

**(*R*)-DOTAGA-*γ*-glu-(PEG)₃-Trp-(N-Me)Nle-Asp-1-Nal-NH₂ ((R)-DOTAGA-CCK-66):** RP-HPLC (10→70% MeCN in H₂O with 0.1% TFA, 15 min, λ = 220 nm): *t*_{R} = 10.9 min; MS (ESI, positive): m/z calculated for C₇₀H₁₀₀N₁₂O₂₃: 1477.6, found: m/z = 1477.4 [M+H]⁺, 739.7 [M+2H]²⁺.

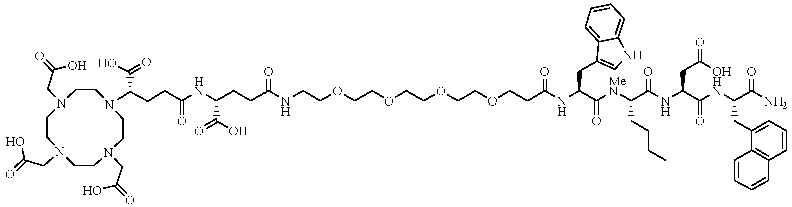

**(S)-DOTAGA-*γ*-glu-(PEG)₃-Trp-(N-Me)Nle-Asp-1-Nal-NH₂** ((S)-DOTAGA-CCK-66): RP-HPLC (10→70% MeCN in H₂O with 0.1% TFA, 15 min, λ = 220 nm): *t*_{R} = 10.9 min; MS (ESI, positive): m/z calculated for C₇₀H₁₀₀N₁₂O₂₃: 1477.6, found: m/z = 1477.4 [M+H]⁺, 739.6 [M+2H]²⁺.

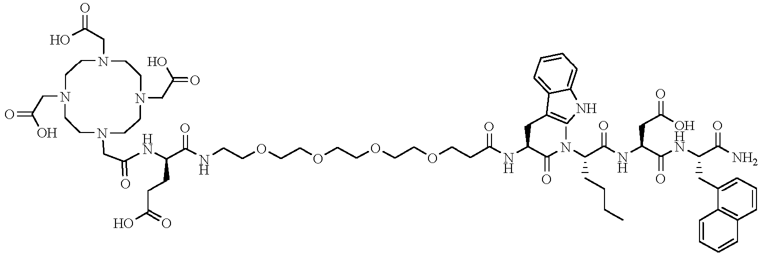

**DOTA-glu-(PEG)₃-Trp-(N-Me)Nle-Asp-1-Nal-NH₂ (DOTA-CCK-66.2):** RP-HPLC (10→70% MeCN in H₂O with 0.1% TFA, 15 min, λ = 220 nm): *t*_{R} = 10.9 min; MS (ESI, positive): m/z calculated for C₆₇H₉₆N₁₂O₂₁: 1405.6, found: m/z = 1405.9 [M+H]⁺, 703.6 [M+2H]²⁺.

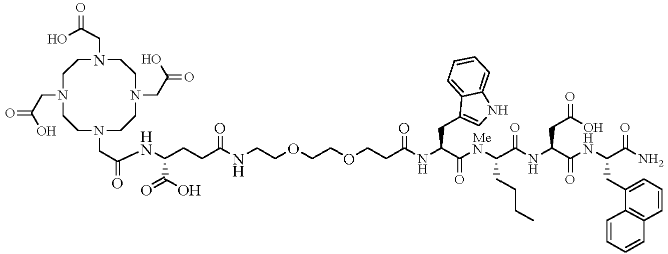

**DOTA-*γ*-glu-PEG-Trp-(N-Me)Nle-Asp-1-Nal-NH₂ (DOTA-CCK-77):** RP-HPLC (10→70% MeCN in H₂O with 0.1% TFA, 15 min, λ = 220 nm): *t*_{R} = 10.8 min; MS (ESI, positive): m/z calculated for C₆₂H₈₆N₁₂O₁₉: 1303.4, found: m/z = 1302.9 [M+H]⁺, 651.6 [M+2H]²⁺.

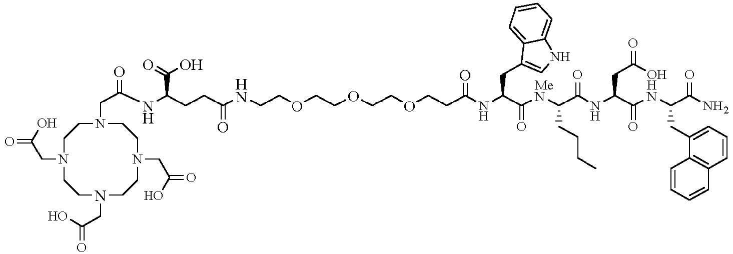

**DOTA-γ-glu-(PEG)₂-Trp-(N-Me)Nle-Asp-1-Nal-NH₂ (DOTA-CCK-63):** RP-HPLC (10→70% MeCN in H₂O with 0.1% TFA, 15 min, λ = 220 nm): *t*_{R} = 10.9 min; MS (ESI, positive): m/z calculated for C₆₅H₉₂N₁₂O₂₀: 1361.5, found: m/z = 1359.5 [M+H]⁺, 680.6 [M+2H]²⁺.

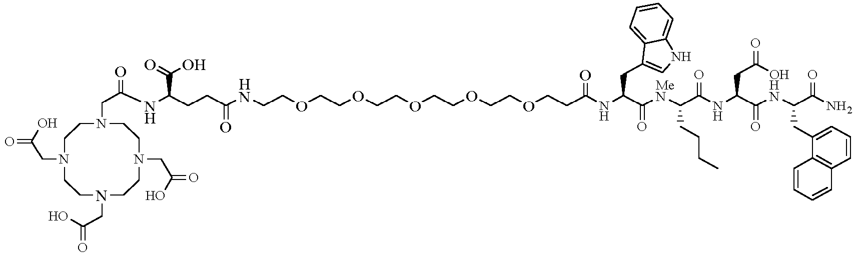

**DOTA-γ-glu-(PEG)₄-Trp-(N-Me)Nle-Asp-1-Nal-NH₂ (DOTA-CCK-78):** RP-HPLC (10→70% MeCN in H₂O with 0.1% TFA, 15 min, λ = 220 nm): *t*_{R} = 11.0 min; MS (ESI, positive): m/z calculated for C₆₉H₁₀₀N₁₂O₂₂: 1449.6, found: m/z = 1447.4 [M+H]⁺, 724.3 [M+2H]²⁺.

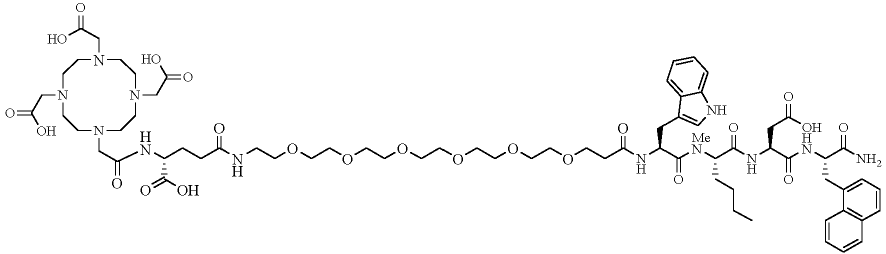

**DOTA-γ-glu-(PEG)₅-Trp-(N-Me)Nle-Asp-1-Nal-NH₂ (DOTA-CCK-79):** RP-HPLC (10→70% MeCN in H₂O with 0.1% TFA, 15 min, λ = 220 nm): *t*_{R} = 11.1 min; MS (ESI, positive): m/z calculated for C₇₁H₁₀₄N₁₂O₂₃: 1493.7, found: m/z = 1491.1 [M+H]⁺, 746.3 [M+2H]²⁺.

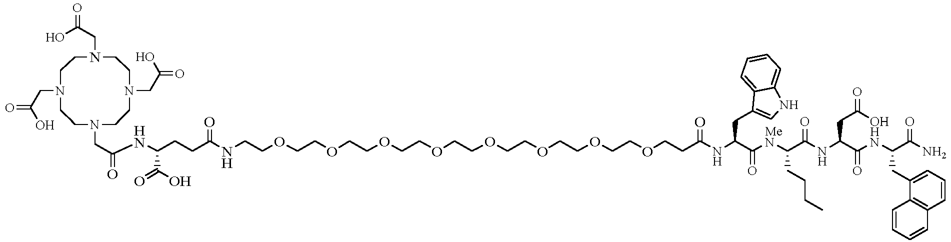

**DOTA-γ-glu-(PEG)₇-Trp-(N-Me)Nle-Asp-1-Nal-NH₂ (DOTA-CCK-80):** RP-HPLC (10→70% MeCN in H₂O with 0.1% TFA, 15 min, λ = 220 nm): *t*_{R} = 11.1 min; MS (ESI, positive): m/z calculated for C₇₅H₁₁₂N₁₂O₂₅: 1581.8, found: m/z = 1582.4 [M+H]⁺, 792.0 [M+2H]²⁺.

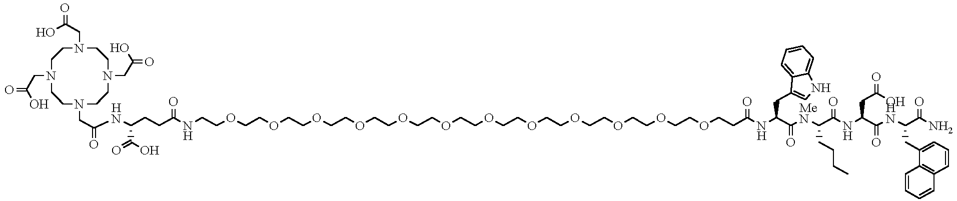

**DOTA-γ-glu-(PEG)₁₁-Trp-(N-Me)Nle-Asp-1-Nal-NH₂ (DOTA-CCK-81):** RP-HPLC (10→70% MeCN in H₂O with 0.1% TFA, 15 min, λ = 220 nm): *t*_{R} = 11.3 min; MS (ESI, positive): m/z calculated for C₈₃H₁₂₈N₁₂O₂₉: 1758.0, found: m/z = 1759.0 [M+H]⁺, 880.0 [M+2H]²⁺.

### PEGylated rhCCK ligands

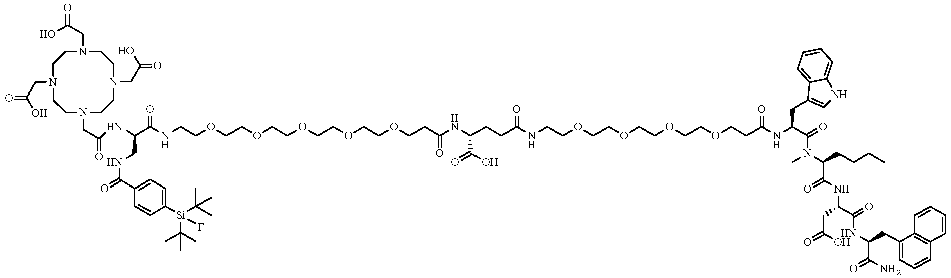

**DOTA-dap(SiFA)-(PEG)₄-γ-glu-(PEG)₃-Trp-(N-Me)Nle-Asp-1-Nal-NH₂ (DOTA-rhCCK-67):** RP-HPLC (10→70% MeCN in H₂O with 0.1% TFA, 15 min, λ = 220 nm): *t*_{R} = 14.1 min; MS (ESI, positive): m/z calculated for C₉₈H₁₄₈FN₁₅O₂₉Si: 2047.4, found: m/z = 1022.9 [M+2H]²⁺.

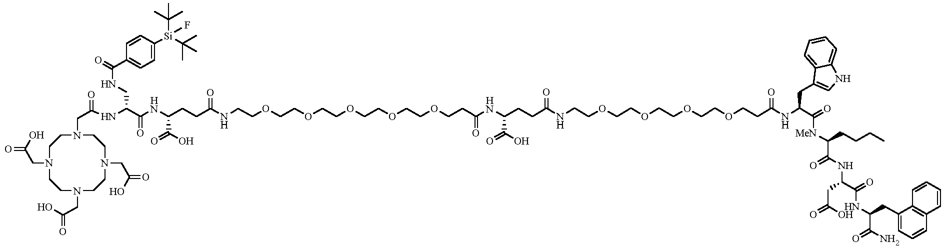

**DOTA-dap(SiFA)-γ-glu-(PEG)₄-γ-glu-(PEG)₃-Trp-(N-Me)Nle-Asp-1-Nal-NH₂ (DOTA-rhCCK-68):** RP-HPLC (10→70% MeCN in H₂O with 0.1% TFA, 15 min, λ = 220 nm): *t*_{R} = 13.3 min; MS (ESI, positive): m/z calculated for C₁₀₃H₁₅₅FN₁₆O₃₂Si: 2175.1, found: m/z = 1087.3 [M+2H]²⁺.

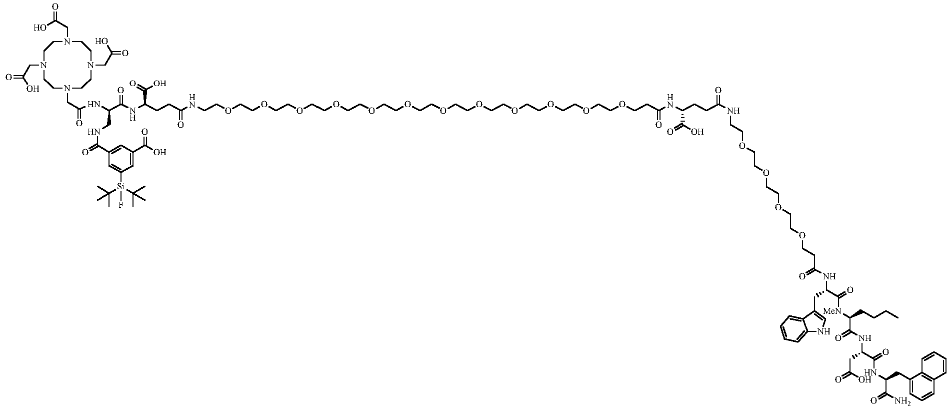

**DOTA-dap(SiFA-ipa)-*γ*-glu-(PEG)₁₁-γ-glu-(PEG)₃-Trp-(N-Me)Nle-Asp-1-Nal-NH₂ (DOTA-rhCCK-69):** RP-HPLC (10→70% MeCN in H₂O with 0.1% TFA, 15 min, λ = 220 nm): *t*_{R} = 12.7 min; MS (ESI, positive): m/z calculated for C₁₁₈H₁₈₃FN₁₆O₄₁Si: 2528.9, found: m/z = 1264.9 [M+2H]²⁺.

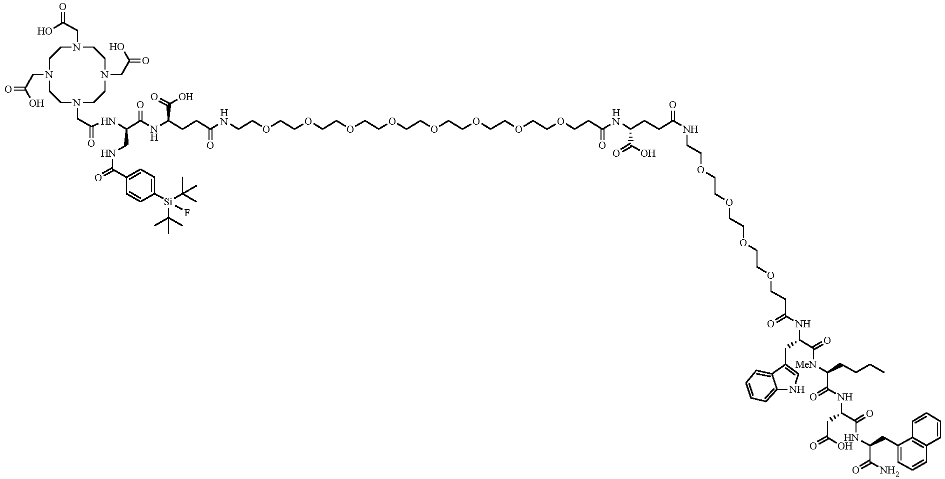

**DOTA-dap(SiFA)-*γ*-glu-(PEG₇-γ-glu-(PEG)₃-Trp-(N-Me)Nle-Asp-1-Nal-NH₂ (DOTA-rhCCK-70):** RP-HPLC (10→70% MeCN in H₂O with 0.1% TFA, 15 min, λ = 220 nm): *t*_{R} = 13.6 min; MS (ESI, positive): m/z calculated for C₁₀₃H₁₅₅FN₁₆O₃₂Si: 2175.1, found: m/z = 1153.8 [M+2H]²⁺.

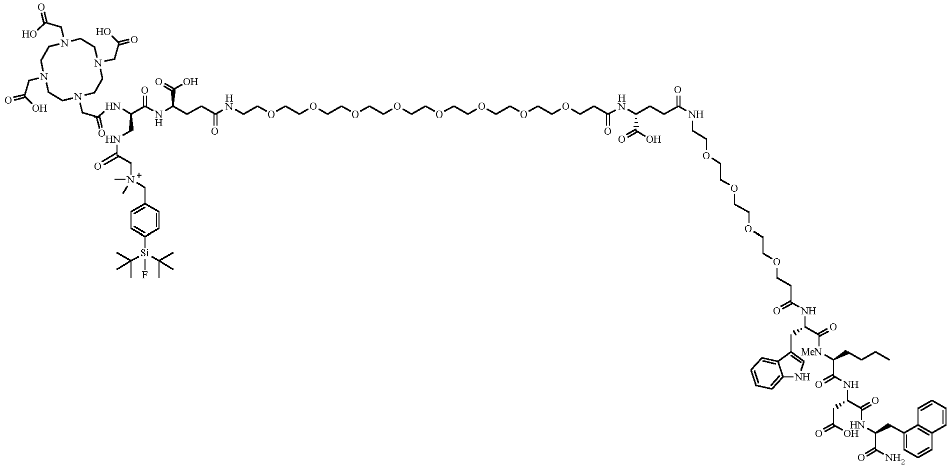

**DOTA-dap(SiFAlin)-γ-glu-(PEG)₇-γ-glu-(PEG)₃-Trp-(N-Me)Nle-Asp-1-Nal-NH₂ (DOTA-rhCCK-71):** RP-HPLC (10→70% MeCN in H₂O with 0.1% TFA, 15 min, λ = 220 nm): *t_{R}* = 13.2 min; MS (ESI, positive): m/z calculated for C₁₁₃H₁₇₇FN₁₇O₃₅Si*: 2380.8, found: m/z = 1191.0 [M+2H]²⁺, 794.1 [M+3H]³⁺.

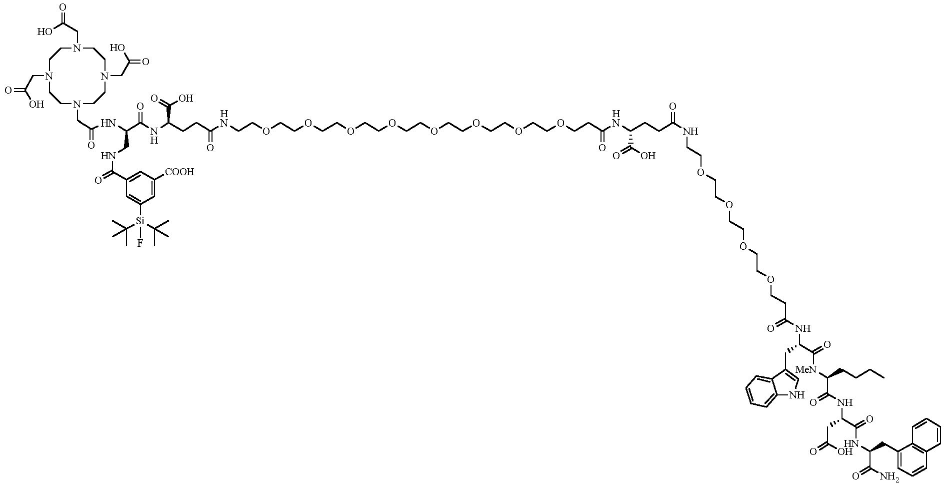

**DOTA-dap(SiFA-ipa)-γ-glu-(PEG)₇-γ-glu-(PEG)₃-Trp-(N-Me)Nle-Asp-1-Nal-NH₂ (DOTA-rhCCK-72):** RP-HPLC (10→70% MeCN in H₂O with 0.1% TFA, 15 min, λ = 220 nm): *t*_{R} = 12.8 min; MS (ESI, positive): m/z calculated for C₁₁₀H₁₆₇FN₁₆O₃₇Si: 2352.7, found: m/z = 1177.8 [M+2H]²⁺, 785.4 [M+3H]³⁺.

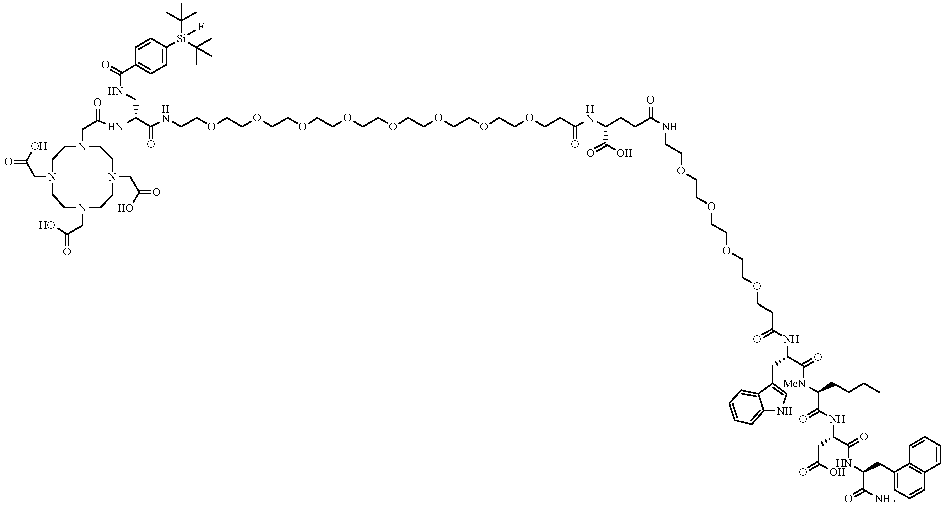

**DOTA-dap(SiFA)-(PEG)₇-γ-glu-(PEG)₃-Trp-(N-Me)Nle-Asp-1-Nal-NH₂ (DOTA-rhCCK-73):** RP-HPLC (10→70% MeCN in H₂O with 0.1% TFA, 15 min, λ = 220 nm): *t*_{R} = 14.1 min; MS (ESI, positive): m/z calculated for C₁₀₄H₁₆₀FN₁₅O₃₂Si: 2179.6, found: m/z = 1090.6 [M+2H]²⁺, 727.3 [M+3H]³⁺.

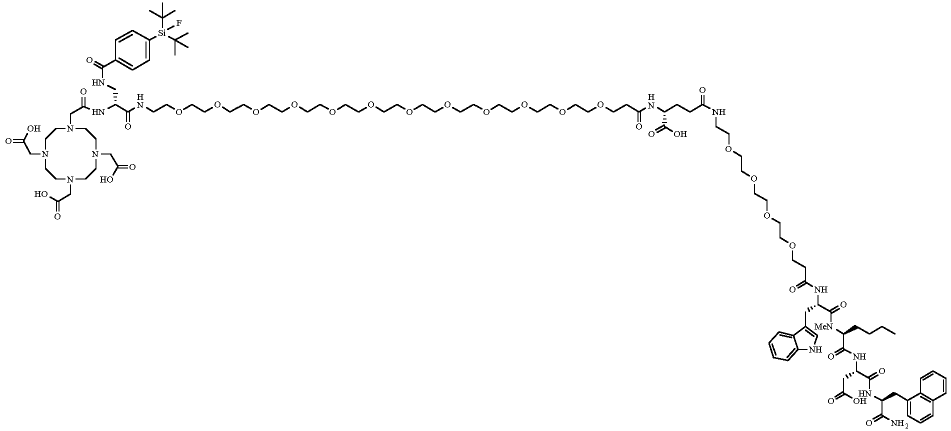

**DOTA-dap(SiFA)-(PEG)₁₁-*γ*-glu-(PEG)₃-Trp-(N-Me)Nle-Asp-1-Nal-NH₂ (DOTA-rhCCK-74):** RP-HPLC (10→70% MeCN in H₂O with 0.1% TFA, 15 min, λ = 220 nm): *t*_{R} = 14.0 min; MS (ESI, positive): m/z calculated for C₁₁₂H₁₇₆FN₁₅O₃₆Si: 2355.8, found: m/z = 1176.8 [M+2H]²⁺, 784.8 [M+3H]³⁺.

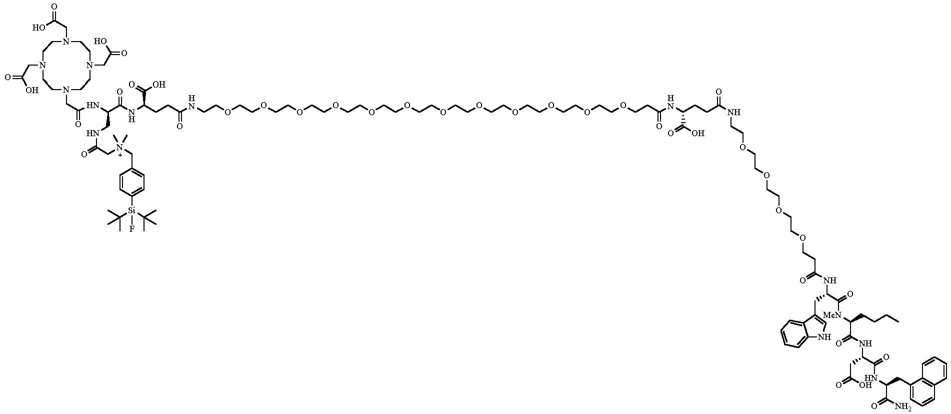

**DOTA-dap(SiFAlin)-γ-glu-(PEG)₁₁-*γ*-glu-(PEG)₃-Trp-(N-Me)Nle-Asp-1-Nal-NH₂ (DOTA-rhCCK-75):** RP-HPLC (10→70% MeCN in H₂O with 0.1% TFA, 15 min, λ = 220 nm): *t*_{R} = 13.2 min; MS (ESI, positive): m/z calculated for C₁₂₁H₁₉₃FN₁₇O₃₉Si⁺: 2557.0, found: m/z = 1279.5 [M+2H]²⁺, 853.2 [M+3H]³⁺.

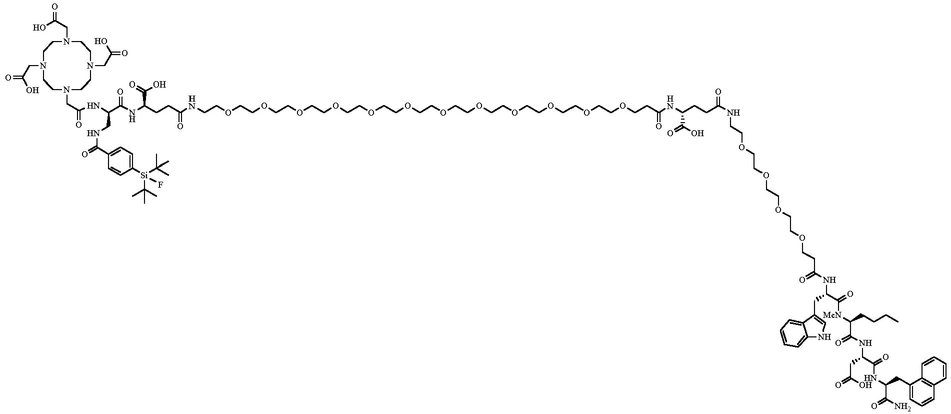

**DOTA-dap(SiFA)-γ-glu-(PEG)₁₁-γ-glu-(PEG)₃-Trp-(N-Me)Nle-Asp-1-Nal-NH₂** (DOTA-rhCCK-76): RP-HPLC (10→70% MeCN in H₂O with 0.1% TFA, 15 min, λ = 220 nm): *t*_{R} = 13.6 min; MS (ESI, positive): m/z calculated for C₁₁₇H₁₈₃FN₁₆O₃₉Si: 2484.9, found: m/z = 1241.4 [M+2H]²⁺, 827.9 [M+3H]³⁺.

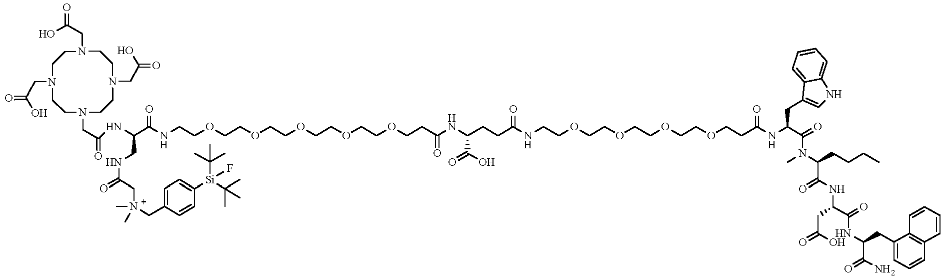

**DOTA-dap(SiFAlin)-(PEG)₄-γ-glu-(PEG)₃-Trp-(N-Me)Nle-Asp-1-Nal-NH₂ (DOTA-rhCCK-90):** RP-HPLC (10→70% MeCN in H₂O with 0.1% TFA, 15 min, λ = 220 nm): *t*_{R} = 13.6 min; MS (ESI, positive): m/z calculated for C₁₀₂H₁₅₈FN₁₆O₂₉Si⁺: 2119.6, found: m/z = 1061.7 [M+2H]²⁺, 708.2 [M+3H]³⁺.

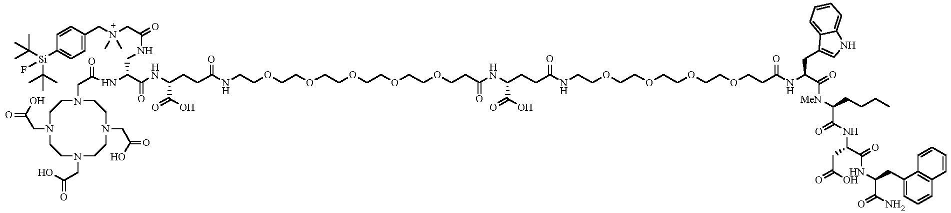

**DOTA-dap(SiFAlin)-γ-glu-(PEG)₄-γ-glu-(PEG)₃-Trp-(N-Me)Nle-Asp-1-Nal-NH₂ (DOTA-rhCCK-91):** RP-HPLC (10→70% MeCN in H₂O with 0.1% TFA, 15 min, λ = 220 nm): *t*_{R} = 13.1 min; MS (ESI, positive): m/z calculated for C₁₀₇H₁₆₅FN₁₇O₃₂Si⁺: 2248.7, found: m/z = 1125.6 [M+2H]²⁺, 750.8 [M+3H]³⁺.

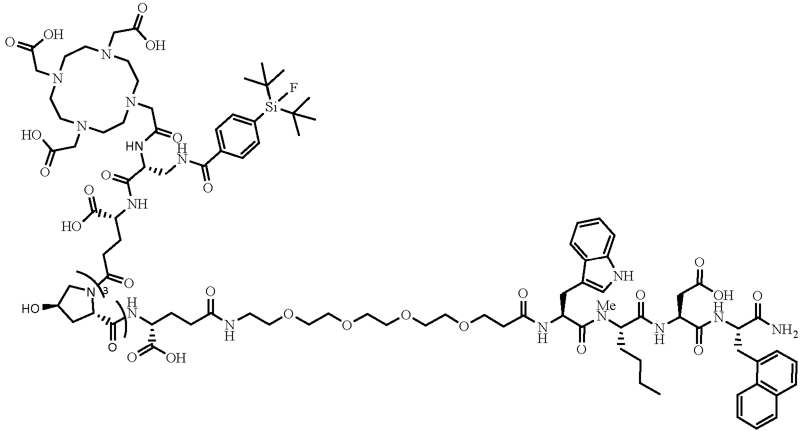

**DOTA-dap(SiFA)-*γ*-glu-(Hyp)₃-*γ*-glu-(PEG)₃-Trp-(N-Me)Nle-Asp-1-Nal-NH₂ (DOTA-rhCCK-83):** RP-HPLC (10→70% MeCN in H₂O with 0.1% TFA, 15 min, λ = 220 nm): *t*_{R} = 13.2 min; MS (ESI, positive): m/z calculated for C₁₀₅H₁₅₁FN₁₈O₃₂Si: 2224.5, found: m/z = 1113.8 [M+2H]²⁺, 743.1 [M+3H]³⁺.

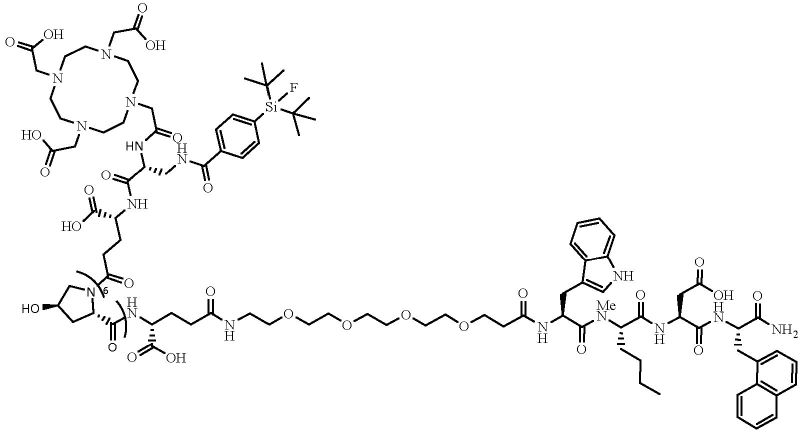

**DOTA-dap(SiFA)-γ-glu-(Hyp)₆-γ-glu-(PEG)₃-Trp-(N-Me)Nle-Asp-1-Nal-NH₂ (DOTA-rhCCK-84):** RP-HPLC (10→70% MeCN in H₂O with 0.1% TFA, 15 min, λ = 220 nm): *t*_{R} = 12.6 min; MS (ESI, positive): m/z calculated for C₁₂₀H₁₇₂FN₂₁O₃₆Si: 2563.9, found: m/z = 1284.3 [M+2H]²⁺, 856.5 [M+3H]³⁺.

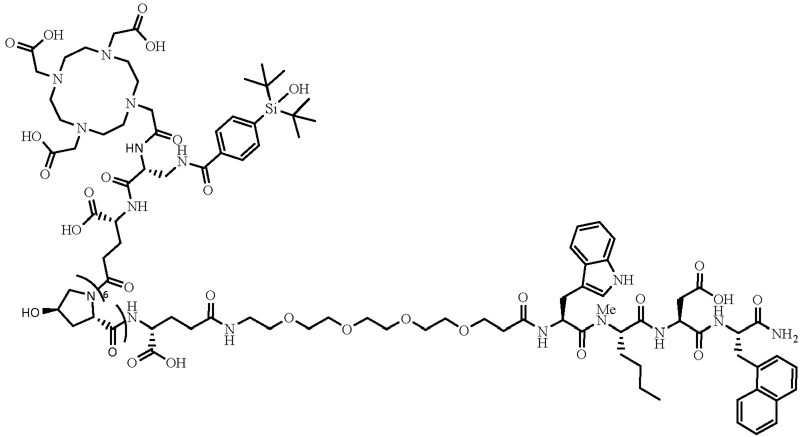

**DOTA-dap(SiOH)-*γ*-glu-(Hyp)₆-γ-glu-(PEG)₃-Trp-(N-Me)Nle-Asp-1-Nal-NH₂ (DOTA-rhCCK-84.2):** RP-HPLC (10→70% MeCN in H₂O with 0.1% TFA, 15 min, λ = 220 nm): *t*_{R} = 11.7 min; MS (ESI, positive): m/z calculated for C₁₂₀H₁₇₃N₂₁O₃₉Si: 2561.9, found: m/z = 1281.6 [M+2H]²⁺, 854.2 [M+3H]³⁺.

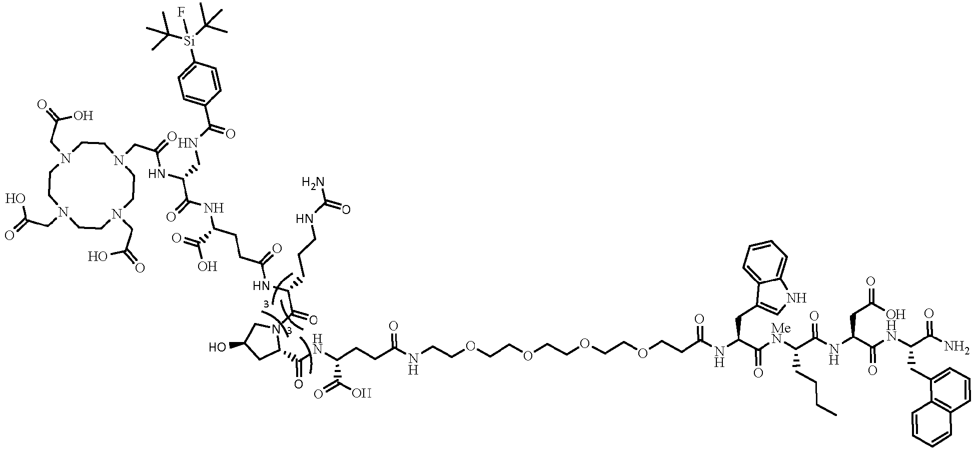

**DOTA-dap(SiFA)-γ-glu-(cit)₃-(Hyp)₃-γ-glu-(PEG)₃-Trp-(N-Me)Nle-Asp-1-Nal-NH₂ (DOTA-rhCCK-85):** RP-HPLC (10→70% MeCN in H₂O with 0.1% TFA, 15 min, λ = 220 nm): *t*_{R} = 12.6 min; MS (ESI, positive): m/z calculated for C₁₂₃H₁₈₄FN₂₇O₃₈Si: 2696.1, found: m/z = 1349.8 [M+2H]²⁺, 900.6 [M+3H]³⁺.

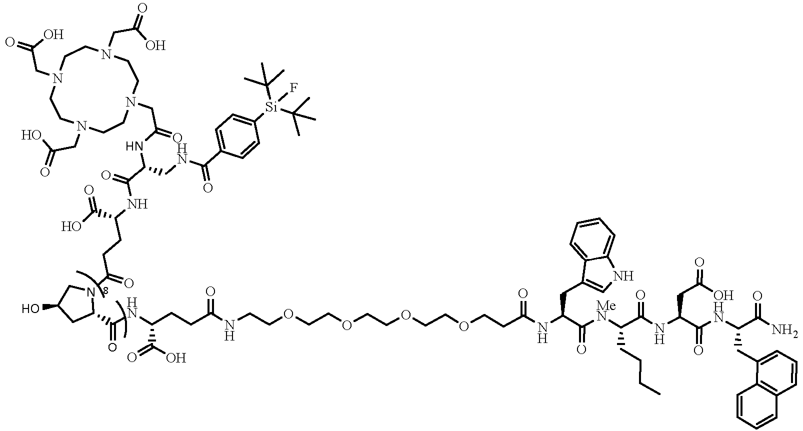

**DOTA-dap(SiFA)-γ-glu-(Hyp)₈-γ-glu-(PEG)₃-Trp-(N-Me)Nle-Asp-1-Nal-NH₂ (DOTA-rhCCK-86):** RP-HPLC (10→70% MeCN in H₂O with 0.1% TFA, 15 min, λ = 220 nm): *t*_{R} = 12.2 min; MS (ESI, positive): m/z calculated for C₁₃₀H₁₈₆FN₂₃O₄₂Si: 2790.1, found: m/z = 1396.1 [M+2H]²⁺, 930.9 [M+3H]³⁺.

### 1.2. In vitro results of the compounds with a modified Trp-(N-Me)Nle-Asp-1-Nal motif

Although most CCK-2R-targeted compounds consist of at least seven amino acids (plus a linker and a chelator), several publications state that the four N-terminal ones are crucial for high CCK-2R affinity [28,30,33]. Indeed, all ^{nat}Lu-labeled reference compounds revealed high CCK-2R affinity (4.9-12.8 nM), while most compounds modified within the N-terminal (four) amino acid sequence exhibited slightly or noticeably decreased CCK-2R affinity **(Tab. 1).**

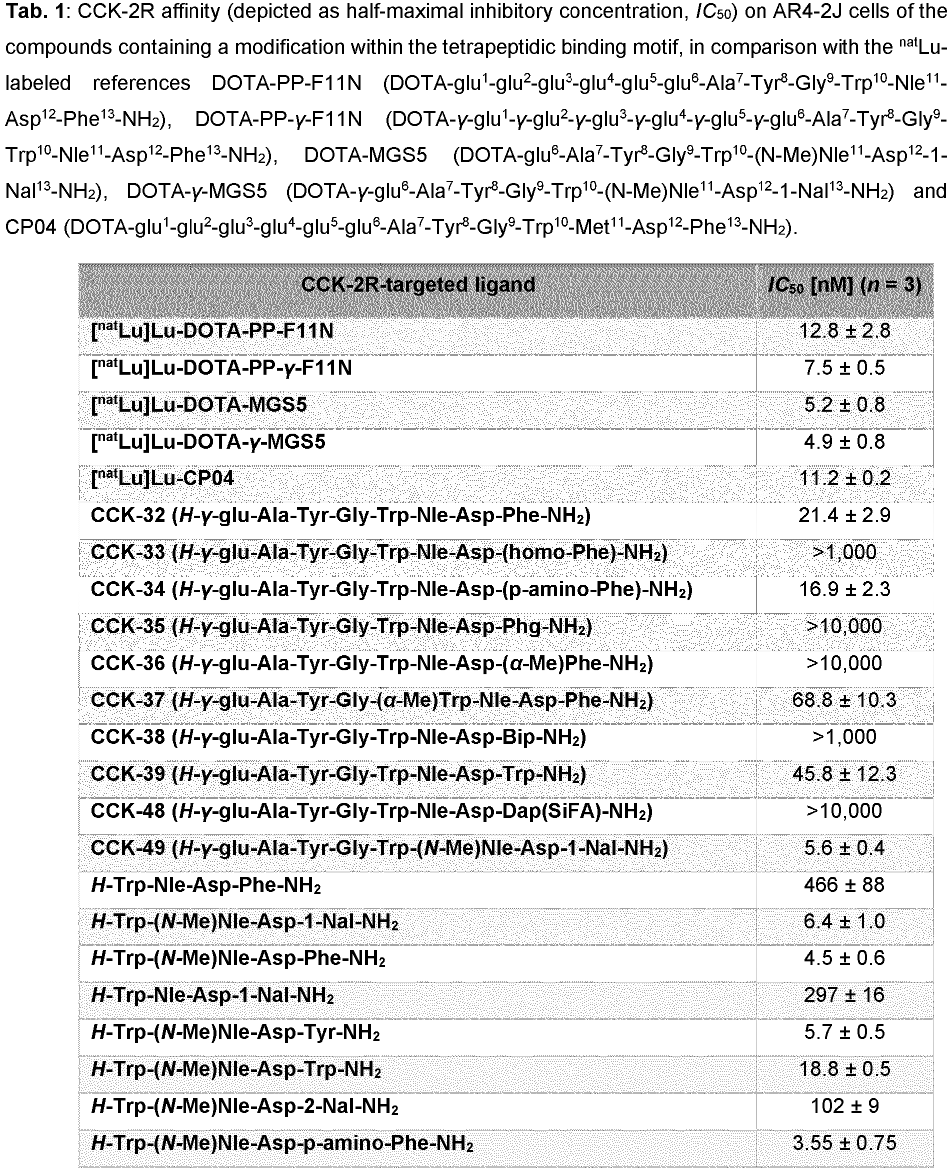

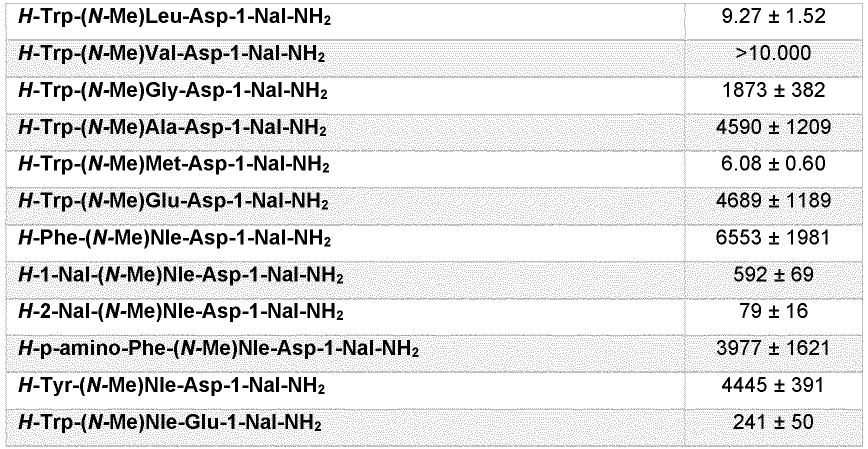

Interestingly, DOTA-PP-F11N without a DOTA chelator and without its six D-glutamates (CCK-32) demonstrated a lower CCK-2R affinity than its parent peptide. Substitution of the C-terminal Phe in CCK-32 by similar aromatic amino acids (CCK-33 to -39) led to a complete loss of CCK-2R affinity for most compounds. Substitution of Trp by (*α*-Me)Trp in CCK-32 led to a threefold higher *IC*₅₀ value, while substitution of Phe by p-amino-Phe led to a slightly decreased *IC*₅₀ value. All these observations point to a conserved site at the C-terminus.

Interestingly, DOTA-MGS5 without a DOTA chelator (CCK-49), which differs from CCK-32 only in two positions ((N-Me)Nle instead of Nle and 1-Nal instead of Phe), showed comparable *IC*₅₀ values than [^{nat}Lu]Lu-DOTA-MGS5 and approximately fourfold lower *IC*₅₀ values than CCK-32. Surprisingly, evaluation of a truncated DOTA-PP-F11N motif, consisting of only the four N-terminal amino acids (H-Trp-Nle-Asp-Phe-NH₂), displayed low CCK-2R affinity, while a truncated DOTA-MGS5 motif (H-Trp-(N-Me)Nle-Asp-1-Nal-NH₂) revealed a comparably high CCK-2R affinity to [^{nat}Lu]Lu-DOTA-MGS5 (Fig. 1).

These results led to the hypothesis that the four N-terminal amino acids of DOTA-MGS5 (H-Trp-(N-Me)Nle-Asp-1-Nal-NH₂) are sufficient for high CCK-2R affinity, while for DOTA-PP-F11N and its analogues, only the complete peptide structure revealed CCK-2R affinities in the low nanomolar range.

Further examination of the tetrapeptidic structure *H*-Trp-(*N*-Me)Nle-Asp-1-Nal-NH₂ (derived from DOTA-MGS5) exhibited that substitution of 1-Nal by Phe led to an even higher CCK-2R affinity, while substitution of Phe by 1-Nal in *H*-Trp-Nle-Asp-Phe-NH₂ (derived from DOTA-PP-F11N) led to a distinctly decreased CCK-2R affinity. It was thus suggested that high CCK-2R affinity for CCK-2-targeted tetrapeptides can only be observed if a methyl group is present at the amide nitrogen between the Trp-Nle bond **(****Fig. 2****).** Keeping this (N-Me)Nle motif within the tetrapeptide, even substitutions at the C-terminus are tolerated (Trp, Tyr and p-amino-Phe instead of 1-Nal in *H*-Trp-(*N*-Me)Nle-Asp-1-Nal-NH₂). However, this position is still conserved, as the substitution of 1-Nal by 2-Nal resulted in a noticeable loss of CCK-2R affinity.

In addition, substitution of Nle by Met and Leu led to tetrapeptides with CCK-2R affinities in a low nanomolar range, whereas substitution by Val, Gly, Ala and Glu was not tolerated with regard to a high CCK-2R affinity. We therefore assume that Nle can only be exchanged by aliphatic amino acids with similar length to maintain high CCK-2R affinity. Neither amino acids with negative charges, nor with smaller aliphatic side chains can be successfully installed in this position. Substitution of Trp by various aromatic amino acids such as Phe, 1-Nal, 2-Nal, p-amino-Phe or Tyr revealed no improvements in terms of CCK-2R affinity, leading to the assumption that a Trp moiety in said position is inevitable for a high binding affinity of the compounds. Furthermore, a noticeable loss of CCK-2R affinity was observed when substituting Ala by Glu.

### 1.3. In vitro results of the glycine scan derivatives

In order to confirm our hypothesis of a sufficient CCK-2R affinity using only the four N-terminal amino acids of DOTA-MGS5, a glycine scan of the remaining four amino acids of the parent peptide was carried out. Therefore, receptor affinity on AR4-2J cells (*IC*₅₀) as well as lipophilicity (log*D*_{7.4}) of the novel conjugates were determined (Tab. 2).

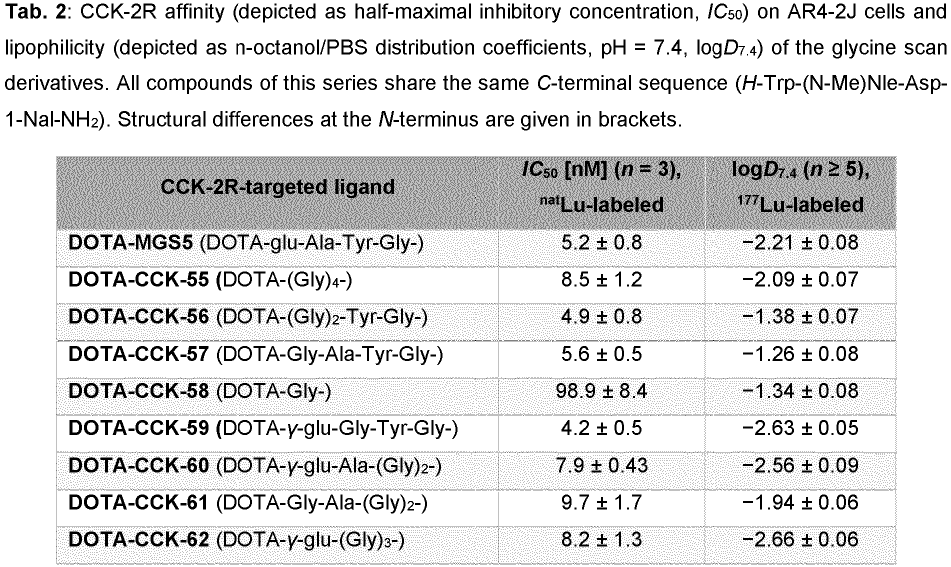

These studies revealed that our assumptions were mostly correct, as the amino acids *γ*-glu/glu, Ala and Tyr could be substituted by Gly residues without a significant loss of CCK-2R affinity in comparison to the parent peptide ([^{nat}Lu]Lu-DOTA-MGS5). These data support the hypothesis that the N-terminal motif, H-Trp-(N-Me)Nle-Asp-1-Nal, is sufficient for high CCK-2R affinity and further N-terminal amino acids can be considered as linker moieties. Nevertheless, the drastic loss of CCK-2R affinity for [^{nat}Lu]Lu-DOTA-CCK-50 demonstrated that a distinct distance of sterically demanding chelators such as DOTA is mandatory for high CCK-2R affinity. Therefore, it was suggested that a simple linker, such as a PEGₓ chain, is necessary but also sufficient between the Trp moiety and the chelator.

Furthermore, these studies showed that the presence of a *γ*-glu moiety within the linker section is beneficial not only for CCK-2R affinity but particularly for overall lipophilicity, as all ligands containing this moiety exhibited log*D*_{7.4} values between -2.66 to -2.56, while those compounds that did not comprise this moiety showed log*D*_{7.4} values between -2.09 to -1.26 **(Tab. 2,** **Fig. 3****).**

As mentioned earlier, no successful strategies to address the metabolically unstable Gly-Trp bond ubiquitously present in CCK-2R agonists have been described yet. Based on the results described in this chapter, it was assumed that amino acids at the N-terminal side of *H*-Trp-(*N*-Me)Nle-Asp-1-Nal-NH₂ (*H*-glu-Ala-Tyr-Gly-) can be replaced by a simple PEGₓ chain, which could also stabilize the overall peptide because a PEG-Trp bond is supposed to be metabolically more stable that a Gly-Trp bond. Therefore, this PEG-containing design was evaluated and is described in the next chapter.

### 1.4. In vitro and in vivo results of the CCK-2R-targeted compounds containing a PEG linker

Due to the results of the previous studies, the four amino acids *γ*-glu-Ala-Tyr-Gly in DOTA-MGS5 were substituted by PEGₓ chains of different length. As there are several reports of a major metabolic instability at the Gly-Trp site, it was assumed that these modifications could further enhance the metabolic stability of these DOTA-MGS5 derivatives. Moreover, some compounds also comprise a *γ-*glu moiety, which was observed to be beneficial for both CCK-2R affinity and lipophilicity. All data were compared to DOTA-MGS5 (Tab. 3).

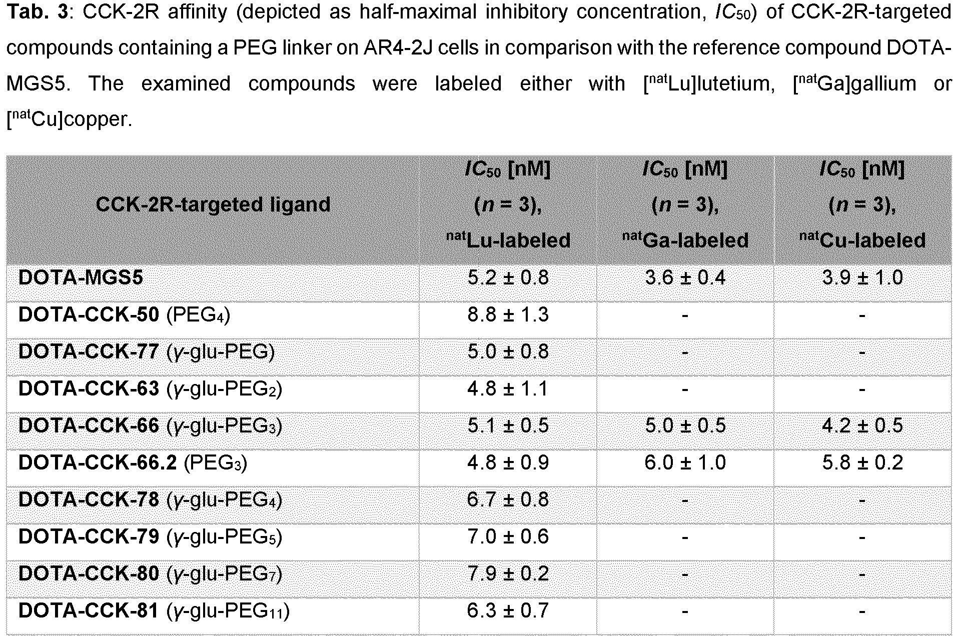

Independent of the length of the PEGₓ chain and chelator-metal chelate used, all compounds revealed an *IC*₅₀ value in the low nanomolar range (3.5-9.0 nM). However, with prolonged PEG chain applied, CCK-2R affinity decreased gradually, which is why highest CCK-2R affinity was observed for DOTA-CCK-77, -63, -66 and -66.2. Furthermore, N-terminal insertion of a *γ*-glu moiety had a positive impact on overall CCK-2R affinity, as DOTA-CCK-78 exhibited lower *IC*₅₀ values than CCK-50 (*IC*₅₀ = 8.84 ± 1.25) despite an equal PEG chain length.

Furthermore, log*D*_{7.4} values of these compounds complexated either with lutetium-177, gallium-67 or copper-64 were evaluated **(Tab. 4).**

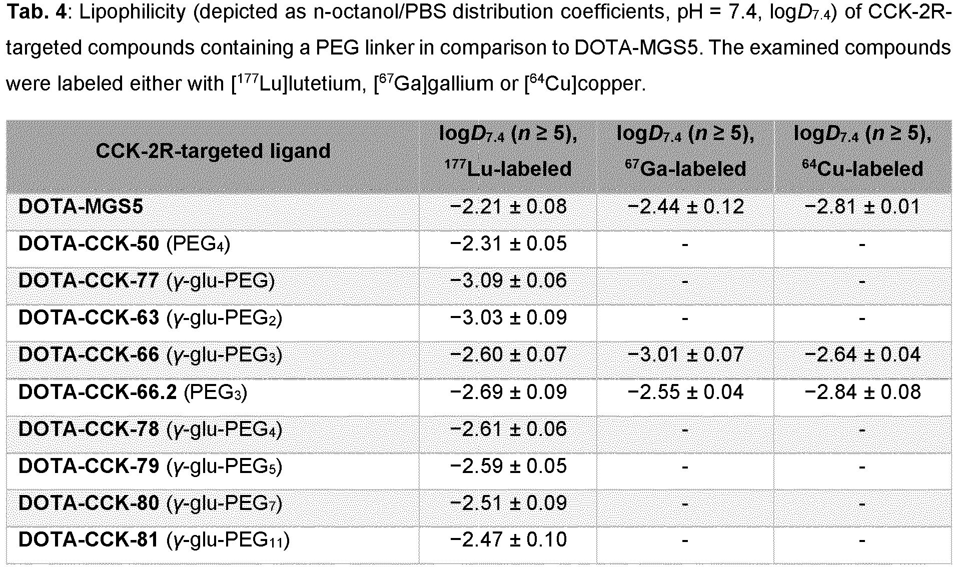

In general, of all ¹⁷⁷Lu-labeled compounds, those containing a shorter PEG chain displayed lower lipophilicity. Moreover, the addition of a *γ*-glu moiety resulted in slightly decreased log*D*_{7.4} values (DOTA-CCK-78 vs. -50). With a log*D*_{7.4} value of <-3, [⁶⁷Ga]Ga-DOTA-CCK-66, [¹⁷⁷Lu]Lu-DOTA-CCK-63 and [¹⁷⁷Lu]Lu-DOTA-CCK-77 were the most hydrophilic compounds of this series. Nevertheless, all compounds revealed log*D*_{7.4} values in a range between -3.1 to -2.3, which is considered ideal for *in vivo* applications.

Among the compounds that demonstrated highest CCK-2R affinity and lowest log*D*_{7.4} values (DOTA-CCK-77, -63, -66 and -66.2), it was decided to further investigate DOTA-CCK-66 and -66.2 alongside DOTA-MGS5 with regard to CCK-2R affinity and lipophilicity when labeled with [^{nat/67}Ga]gallium and [^{nat/64}Cu]copper **(****Figs. 4-5****)** as well as human serum albumin (HSA) binding and metabolic stability.

As *Klingler et al.* reported a favorable biodistribution profile and rapid clearance kinetics for [⁶⁸Ga]Ga-DOTA-MGS5 [30], HSA binding was evaluated for DOTA-CCK-66 and -66.2 and DOTA-MGS5 complexated either with [^{nat}Lu]lutetium, [^{nat}Ga]gallium or [^{nat}Cu]copper **(Tab. 5).**

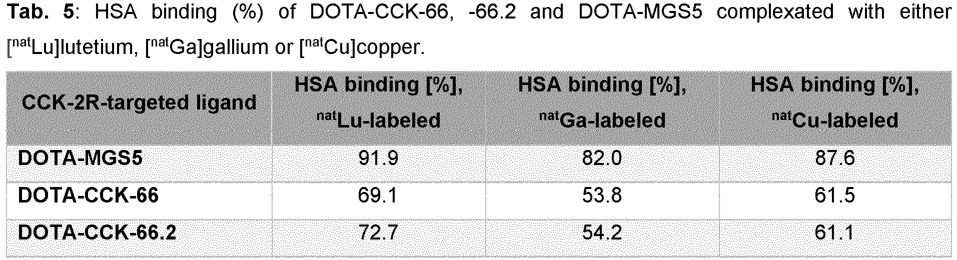

For the different metal chelates of the three PEGylated CCK derivatives evaluated, a trend could be observed **(****Fig. 6****).**

All three ^{nat}Lu-labeled compounds revealed the highest HSA binding, whereas the ^{nat}Cu-complexated peptides showed less interaction with HSA. Furthermore, the least HSA binding was observed for the respective ^{nat}Ga-labeled ligands. In a comparative analysis, DOTA-MGS5 binds to HSA the most, whereas DOTA-CCK-66 as well as DOTA-CCK-66.2 revealed lower HSA affinity. It is thus suggested that both DOTA-CCK-66 and -66.2 will be cleared more rapidly than DOTA-MGS5, irrespective of which radiometal is applied.

Furthermore, stability studies of the ¹⁷⁷Lu-labeled compounds DOTA-MGS5, DOTA-CCK-66 and DOTA-CCK-66.2 were carried out *in vitro* in human serum via incubation at 37 °C for 72 ± 2 h. The more promising compound *in vitro,* [¹⁷⁷Lu]Lu-DOTA-CCK-66, was also evaluated *in vivo* with regard to metabolic stability in murine serum and urine at 30 min p.i. and compared to [¹⁷⁷Lu]Lu-DOTA-MGS5 **(Tab. 6).**

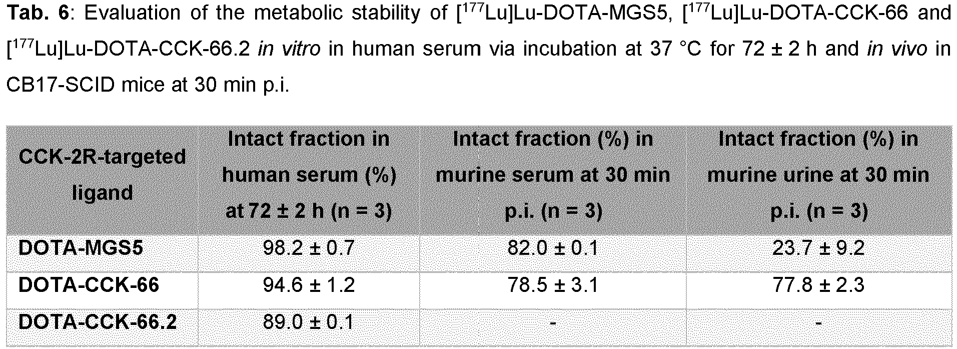

*In vitro,* all three compounds revealed high metabolic stability in human serum after incubation at 37 °C for 72 ± 2 h. However, [¹⁷⁷Lu]Lu-DOTA-MGS5 demonstrated slightly higher intact fractions than the PEGylated compounds **(****Fig. 7****).**

*In vivo,* a slightly higher amount of the parent compound was still intact in murine serum at 30 min p.i. Nevertheless, it could be shown that the intact fraction of [¹⁷⁷Lu]Lu-DOTA-CCK-66 was almost threefold higher in murine urine at 30 min p.i. compared to [¹⁷⁷Lu]Lu-DOTA-MGS5. Hence, it was assumed that [¹⁷⁷Lu]Lu-DOTA-CCK-66 is cleared mostly intact, whereas [¹⁷⁷Lu]Lu-DOTA-MGS5 is cleared mostly metabolized **(****Fig. 8****, Tab. 6),** which could be beneficial for decreased activity accumulation and retention in off-target organs, as metabolites tend to accumulate unspecifically, particularly the kidneys.

In order to examine the pharmacokinetic profile of our novel PEGylated CCK-2R-targeted compound, biodistribution studies were carried out with [⁶⁷Ga]Ga-DOTA-CCK-66 at 1 h p.i. and compared to the respective data of [⁶⁸Ga]Ga-DOTA-MGS5 at the same time point **(****Fig. 9****),** which was published in 2019 [30].

Comparative analysis has to be done carefully, as 100 pmol were injected into each animal and *Klingler et al.* only 20 pmol [30]. However, despite a five times higher peptide amount administered, [⁶⁷Ga]Ga-DOTA-CCK-66 displayed only slightly lower activity levels in the tumor than [⁶⁸Ga]Ga-DOTA-MGS5 at 1 h p.i. As expected, the former revealed lower activity levels in all non-tumor organs, which is likely due to its improved metabolic stability and clearance kinetics. Even though higher activity levels in all organs when lower peptide amounts were administered were assumed, it is likely that tumor uptake would also increase. It is thus suggested that relative tumor-to-background (T/B) ratios are valid, irrespective which peptide amounts were administered. T/B ratios were higher in all organs for [⁶⁷Ga]Ga-DOTA-CCK-66 compared to [⁶⁸Ga]Ga-DOTA-MGS5, particularly relevant for blood, stomach and kidneys. Thus, [⁶⁸Ga]Ga-DOTA-CCK-66, which is chemically identical to [⁶⁷Ga]Ga-DOTA-CCK-66, is a legitimate candidate for clinical translation. This is supported by *µ*SPECT/CT images of [⁶⁷Ga]Ga-DOTA-CCK-66 **(****Fig. 10****).**

Accumulation in the CCK-2R-positive tumor and stomach was specific, which was shown by competition studies via co-administration of excess of [^{nat}Ga]Ga-DOTA-MGS5.

In order to investigate the potential of DOTA-CCK-66 as a theranostic agent, it was additionally evaluated ¹⁷⁷Lu-labeled at 24 h p.i. and compared to [¹⁷⁷Lu]Lu-DOTA-MGS5 **(****Fig. 11****).**

Biodistribution studies at 24 h p.i. revealed slightly lower activity levels in the tumor for [¹⁷⁷Lu]Lu-DOTA-CCK-66 compared to [¹⁷⁷Lu]Lu-DOTA-MGS5 (8.56 ± 1.08 vs. 11.0 ± 1.15 %ID/g). However, tumor/blood ratio was enhanced for the former (2009 ± 469 vs. 1480 ± 329) due to an improved activity clearance from non-tumor organs, which is beneficial for therapeutic applications. Tumor/stomach and tumor/kidney ratios were comparable for both compounds. Competition studies led to significantly decreased activity levels in both stomach and tumor, confirming CCK-2R specificity of [¹⁷⁷Lu]Lu-DOTA-CCK-66. These observations are supported by *µ*SPECT/CT images **(****Fig. 12****).**

For all these reasons, first-in-men studies were carried out in MTC patients who showed elevated serum tumor markers with short calcitonin doubling times. In all three MTC patients evaluated, [⁶⁸Ga]Ga-DOTA-CCK-66 PET/CT demonstrated favorable pharmacokinetics with high accumulation in tumor lesions and the CCK-2R⁺ stomach at 2 h p.i. (Figs. 13A, 14A, and 15A). Apart from the bladder and the ureter as a consequence of physiologic excretion, no significant activity levels were found in other organs. In [⁶⁸Ga]Ga-DOTA-CCK-66 PET/CT multiple MTC-derived lesions could be detected in each patient. Patient 1 had one left retroclavicular lymph node metastasis and a further mediastinal metastasis at the aortic arch. Patient 2 had one right retroclavicular lymph node metastasis, bilateral hilar lymph node metastases, two liver metastases and bone metastases in the right femur as well as the right *os ischii.* Patient 3 had one lymph node metastasis in the left cervical vascular nerve sheath, a local recurrence in the left thyroid bed and a lymph node metastasis in the upper right mediastinum.

All patients benefitted significantly from these findings via [⁶⁸Ga]Ga-DOTA-CCK-66 PET/CT. If not surgically resectable, the identified metastases could be subject to radioligand therapy using [¹⁷⁷Lu]Lu-DOTA-CCK-66, as this compound demonstrated favorable biodistribution profiles with high activity levels in tumor over time and rapid activity clearance from the blood in animals. Further studies in men have to be carried out to confirm these promising results in this small cohort.

In conclusion, the novel PEGylated CCK-2R-targeted compound, [^{67/68}Ga]Ga-/[¹⁷⁷Lu]Lu-DOTA-CCK-66, revealed improved *in vivo* data compared to the clinically applied compound, [⁶⁸Ga]Ga-/[¹⁷⁷Lu]Lu-DOTA-MGS5. T/B ratios are comparable or even enhanced for the former, which can be attributed to the tetrapeptidic design in combination with the PEG linkers. By this, an increased metabolic stability at the Gly-Trp site was demonstrated as a consequence of the substitution of the N-terminal amino acids by a PEGₓ chain. Due to these promising preclinical properties, first-in-men applications in MTC patients using [⁶⁸Ga]Ga-DOTA-CCK-66 were performed, which confirmed the favorable biodistribution profile of this compound already observed in animals. Due to its rapid clearance from the blood, tumor-to-background contrast was high and enabled the detection of multiple tumor manifestations in three MTC patients. Further studies in humans are required to clearly elucidate whether this compound has a beneficial impact on MTC management. However, first results suggest that [⁶⁸Ga]Ga-DOTA-CCK-66 might indeed be a viable option for PET imaging of MTC-derived lesions. Moreover, its ¹⁷⁷Lu-labeled analog could also be a valid therapeutic option, rendering this compound a highly promising theranostic agent.

### 1.5. In vitro and in vivo results of the CCK-2R-targeted compounds containing a PEG linker and different chelators

Due to the results of the previous studies, the DOTA chelator of DOTA-CCK-66 was replaced by various chelators (NOTA, NODAGA, (S)-DOTAGA and (R)-DOTAGA) in order to demonstrate the suitability of combining different chelators with our optimized tetrapeptidic binding motif. **(Tab. 7)**

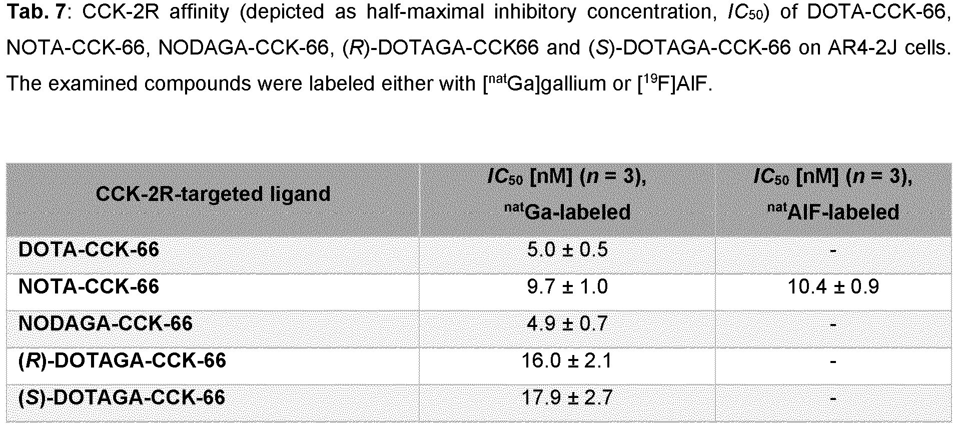

These studies revealed that the DOTA chelator of DOTA-CCK-66 can be substituted by NODAGA without any loss of CCK-2R affinity (Fig. 16). In contrast, substitution of DOTA by NOTA is accompanied by a loss of CCK-2R affinity for both the ^{nat}Ga- and the [¹⁹F]AlF-complexated derivative. Moreover, introduction of the (R)- and (S)-DOTAGA chelator instead of DOTA led to approximately threefold increased *IC*₅₀ values. Despite a lower CCK-2R affinity for most DOTA-CCK-66 analogues, the use of particularly NOTA and NODAGA with [^{nat}Ga]gallium or [¹⁹F]AlF is conceivable for future applications.

### 1.6. In vitro and in vivo results of the first generation of PEGylated rhCCK ligands

Several PEGylated rhCCK compounds were synthesized and evaluated ^{nat/177}Lu/^{nat}Ga-labeled with regard to CCK-2R affinity on AR4-2J cells (*IC*₅₀) as well as lipophilicity (log*D*_{7.4}) **(Tab. 8).**

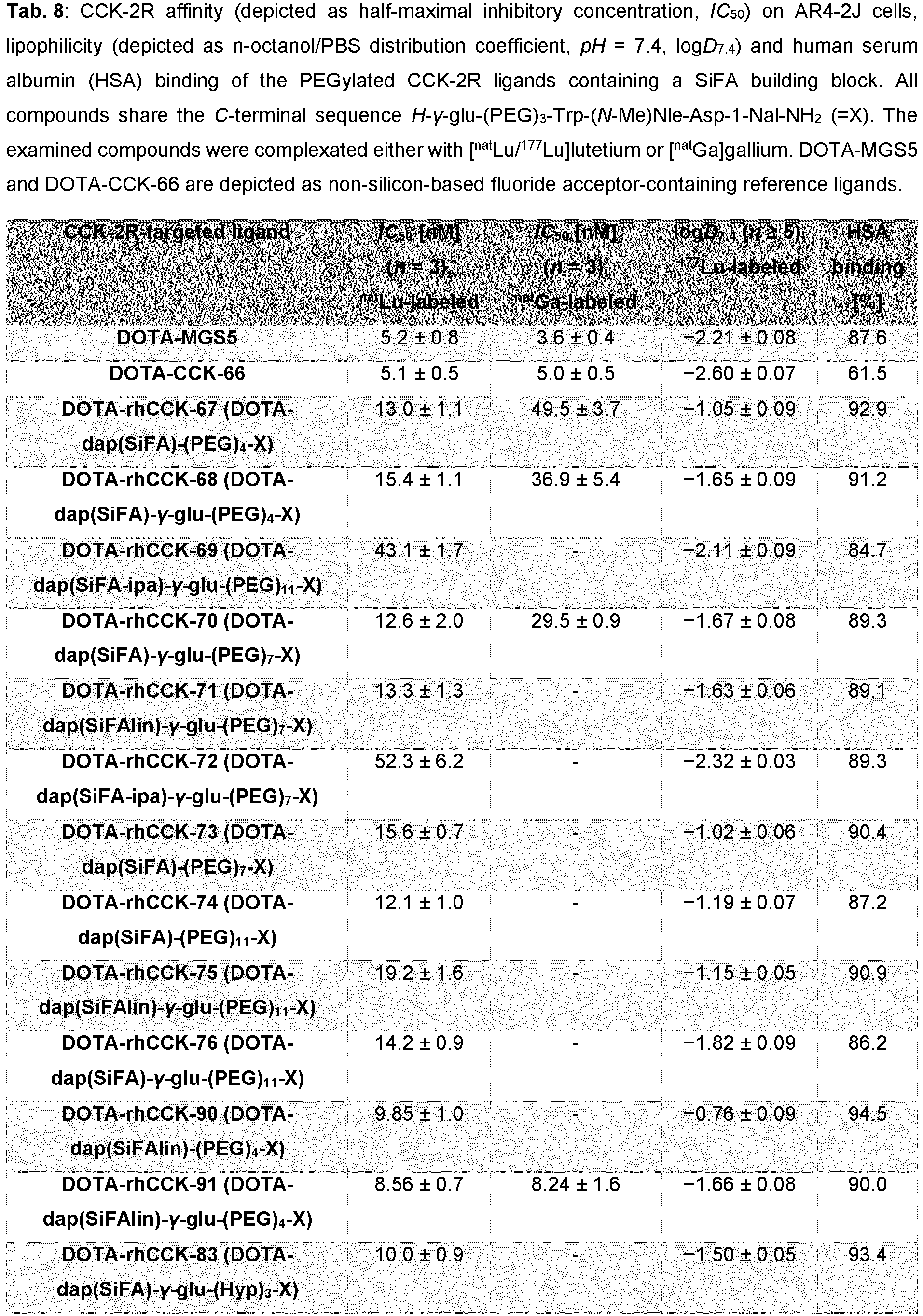

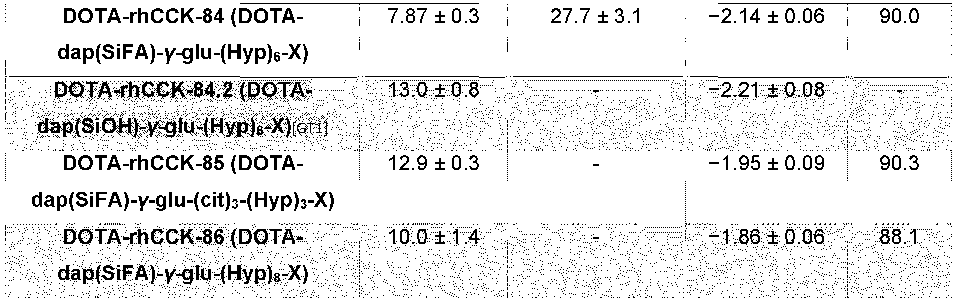

None of the ^{nat}Lu-labeled rhCCK ligands exhibited comparable CCK-2R affinity to [^{nat}Lu]Lu-DOTA-MGS5 or [^{nat}Lu]Lu-DOTA-CCK-66 **(****Fig. 17****).** As expected, [¹⁷⁷Lu]Lu-DOTA-rhCCK-67, [¹⁷⁷Lu]Lu-DOTA-rhCCK-73 and [¹⁷⁷Lu]Lu-DOTA-rhCCK-74, which carry only one negative charge within the linker section, revealed an increased lipophilicity, as the lipophilic SiFA moiety is not compensated completely.

Thus, another *γ*-glu moiety was introduced, which noticeably improved lipophilicity ([^{nat/177}Lu]Lu-DOTA-rhCCK-68, [^{nat/177}Lu]Lu-DOTA-rhCCK-70 and [^{nat/177}Lu]Lu-DOTA-rhCCK-76) while maintaining CCK-2R affinity. In order to further improve lipophilicity, the SiFA was substituted by the more hydrophilic SiFA-ipa ([^{nat/177}Lu]Lu-DOTA-rhCCK-69, [^{nat/177}Lu]Lu-DOTA-rhCCK-72) or SiFAlin ([^{nat/177}Lu]Lu-DOTA-rhCCK-71, [^{nat/177}Lu]Lu-DOTA-rhCCK-75, [^{nat/177}Lu]Lu-DOTA-rhCCK-90, [^{nat/177}Lu]Lu-DOTA-rhCCK-91) building blocks. Compounds containing a SiFA-ipa moiety revealed that log*D*_{7.4} values could be improved by half a magnitude but CCK-2R affinity was distinctly decreased. Substituting the SiFA by a SiFAlin moiety, a comparable CCK-2R affinity was observed for the (PEG)₇ linker containing derivatives. Nevertheless, no improvement with regard to lipophilicity was observed. [^{nat/177}Lu]Lu-DOTA-rhCCK-75, comprising a (PEG)₁₁ linker and a SiFAlin moiety revealed a higher *IC*₅₀ value (19.2 ± 1.6) and lipophilicity (-1.15 ± 0.05) than [^{nat/177}Lu]Lu-DOTA-rhCCK-76 (*IC*₅₀: 14.2 ± 0.9, log*D*_{7.4}: -1.82 ± 0.09), which contains a (PEG)₁₁ linker and a SiFA moiety. Exchanging the SiFA ([^{nat/177}Lu]Lu-DOTA-rhCCK-67, [^{nat/177}Lu]Lu-DOTA-rhCCK-68) by a SiFAlin moiety ([^{nat/177}Lu]Lu-DOTA-rhCCK-90, [^{nat/177}Lu]Lu-DOTA-rhCCK-91) in (PEG)₄-linked rhCCK derivatives had only a minor impact on lipophilicity, but led to enhanced CCK-2R affinity. Surprisingly, DOTA-rhCCK-91 revealed high CCK-2R affinity labeled with both [^{nat}Lu]lutetium (*IC*₅₀ = 8.56 ± 0.7) and [^{nat}Ga]gallium (*IC*₅₀ = 8.24 ± 1.6), which is in contrast to previously evaluated rhCCK derivatives.

In another approach, lipophilicity and CCK-2R affinity of rhCCK derivatives was addressed *via a* polyhydroxyproline linker. Therefore, compounds containing three to eight hydroxyproline moieties in their linker structure (DOTA-rhCCK-83, -84 and -86) were evaluated *in vitro.* Furthermore, a peptide comprising three hydroxyproline and three D-citrulline moieties (DOTA-rhCCK-85) was examined *in vitro.* Out of this series, [^{nat/177}Lu]Lu-DOTA-rhCCK-84 combined the highest hydrophilicity (log*D*_{7.4} = -2.14 ± 0.06) and CCK-2R affinity (*IC*₅₀ = 7.87 ± 0.3).

Not surprisingly, HSA binding (84.7 to 94.5%) was high for all rhCCK derivatives tested **(****Fig. 18****),** which was expected, as increased HSA binding was already observed for other silicon-based fluoride acceptor-comprising compounds, independent of which target molecule was used.

Exhibiting the most attractive *in vitro* data overall, [¹⁷⁷Lu]Lu-DOTA-rhCCK-70, [¹⁷⁷Lu]Lu-DOTA-rhCCK-84 and [¹⁷⁷Lu]Lu-DOTA-rhCCK-91 were evaluated *in vivo* at 24 h p.i. and compared to both [¹⁷⁷Lu]Lu-DOTA-CCK-66 and [¹⁷⁷Lu]Lu-DOTA-MGS5 **(****Fig. 19****).**

[¹⁷⁷Lu]Lu-DOTA-rhCCK-84 revealed the highest activity levels in the tumor at 24 h p.i. (more than and approximately twofold higher compared to [¹⁷⁷Lu]Lu-DOTA-CCK-66 and [¹⁷⁷Lu]Lu-DOTA-MGS5, respectively). Activity levels in the kidneys were slightly enhanced (<10 %ID/g) compared to the non-silicon-based fluoride acceptor-containing references. Overall biodistribution patterns were promising, which is supported by *µ*SPECT/CT images **(****Fig. 20****),** especially in the context of previously developed silicon-based fluoride acceptor-containing minigastrin derivatives that displayed massively increased activity retention in the kidneys (>80 %ID/g at 24 h p.i.) as a consequence of the presence of several negative charges in direct neighborhood to the silicon-based fluoride acceptor building block. Furthermore, it has to be mentioned that activity accumulation and retention in murine kidneys is not directly comparable to the human situation. It has thus to be investigated in future patient studies whether this decelerated activity clearance from the kidneys is also present in men and if it is of concern. Apart from noticeably increased activity levels in the tumor at 24 h p.i., DOTA-rhCCK-84 bears the advantage (over DOTA-CCK-66 and DOTA-MGS5) that it can be easily labeled by fluorine-18, which is beneficial for high-contrast positron-emission tomography.

Besides a favorable tumor uptake (36.3 ± 3.7 %ID/g), biodistribution studies of the structural identical [¹⁸F]F-[^{nat}Lu]Lu-DOTA-rhCCK-84 revealed high activity levels in the blood (11.3 ± 1.3 %ID/g) at 1 h p.i. **(****Fig. 21****).** However, biodistribution studies at 2 and 4 h p.i. confirmed good activity clearance of [¹⁷⁷Lu]Lu-DOTA-rhCCK-84 from the blood over time (3.71 ± 0.46 %ID/g and 1.14 ± 0.18 %ID/g, respectively), while activity levels in the tumor remained high (35.0 ± 2.9 and 34.0 ± 3.6 %ID/g, respectively). Competition studies co-administering excess of [^{nat}Lu]Lu-DOTA-MGS5 or [^{nat}Lu]Lu-DOTA-MG-SiFA-23 led to significantly decreased activity levels in both stomach and tumor, which confirmed CCK-2R specificity of [¹⁷⁷Lu]Lu-DOTA-rhCCK-84 **(****Fig. 22****).**

In addition, *in vivo* stability of [¹⁷⁷Lu]Lu-DOTA-rhCCK-84 was high in both the serum (93.5 ± 2.1%) and the urine (55.4 ± 9.0%) at 30 min p.i. **(****Fig. 23****),** particularly in comparison to the reference compound DOTA-MGS5 **(Tab. 6).** For all the mentioned aspects, this compound might be a promising candidate for clinical translation.

Besides DOTA-rhCCK-84, [¹⁷⁷Lu]Lu-DOTA-rhCCK-70 also exhibited promising *in vivo* data. Activity levels in the tumor at 24 h p.i. were slightly higher than those of [¹⁷⁷Lu]Lu-DOTA-MGS5 but noticeably lower than those of [¹⁷⁷Lu]Lu-DOTA-rhCCK-84 **(****Fig. 19****).** Activity levels in the kidneys were slightly lower than those of [¹⁷⁷Lu]Lu-DOTA-rhCCK-84 but still enhanced compared to the non-silicon-based fluoride acceptor-containing references. Elevated activity levels for [¹⁷⁷Lu]Lu-DOTA-rhCCK-70 in blood, heart, lung, liver, spleen and bone can be partly attributed to incomplete ¹⁷⁷Lu-labeling at the day of experiment (~5% free lutetium-177 injected into the animals) but also to the increased lipophilicity of this compound compared to the non-silicon-based fluoride acceptor-containing ligands. [¹⁷⁷Lu]Lu-DOTA-rhCCK-91 demonstrated the lowest activity levels in the kidneys at 24 h p.i. among all rh-based CCK-2R-targeted compounds but its activity levels in the tumor were also decreased.

Concluded, the biodistribution profiles of [¹⁷⁷Lu]Lu-DOTA-rhCCK-70, -84 and -91 confirmed our assumption that the high activity uptake and retention in the kidneys can be addressed by the reduction of negative charges in the neighborhood of the silicon-based fluoride acceptor moiety (when compared to [¹⁷⁷Lu]Lu-DOTA-MG-SiFA-23, amongst others). Moreover, the novel design of the PEGylated rhCCK ligands resulted in a promising peptide, DOTA-rhCCK-84, which showed distinctly higher activity levels in the tumor compared to [¹⁷⁷Lu]Lu-DOTA-MGS5 or [¹⁷⁷Lu]Lu-DOTA-CCK-66 at 24 h p.i. Moreover, this rh-based compound offers the possibility of facile and rapid ¹⁸F-labeling and revealed a substantially elevated activity accumulation in the tumor at 1, 2, and 4 h p.i., which is why this ligand might be a valid candidate for a theranostic application with fluorine-18 and lutetium-177 in men.

### Example 2 - SiFA-comprising CCK2R-targeted compounds

### 2.1. Overview of the synthesized CCK-2R-targeted ligands

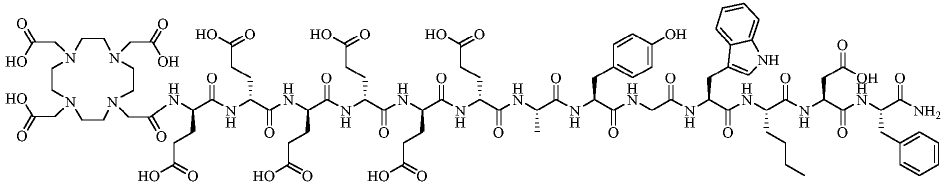

DOTA-PP-F11N (3): RP-HPLC (10→90% MeCN in H₂O with 0.1% TFA, 15 min, λ = 220 nm): *t*_{R} = 7.6 min, K' = 3.5; MS (ESI, positive): m/z calculated for C₉₀H₁₂₃N₁₉O₃₅: 2029.8, found: m/z = 1016.4 [M+2H]²⁺.

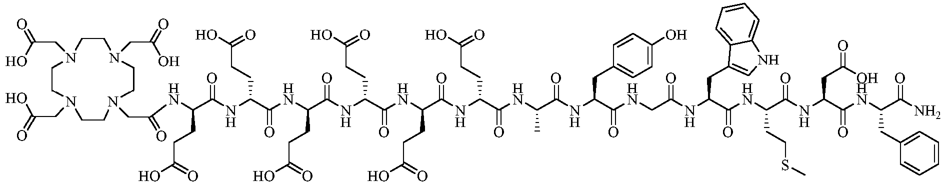

CP04 (4): RP-HPLC (10→90% MeCN in H₂O with 0.1% TFA, 15 min, λ = 220 nm): *t*_{R} = 7.2 min, K' = 3.3; MS (ESI, positive): m/z calculated for C₈₉H₁₂₁N₁₉O₃₅S: 2047.8, found: m/z = 1024.2 [M+2H]²⁺.

In the following, the sequence *H*-glu¹-glu²-glu³-glu⁴-glu⁵-glu⁶-Ala⁷-Tyr⁸-Gly⁹-Trp¹⁰-Nle¹¹-Asp¹²-Phe¹³-NH₂ is defined as F11N.

### MG-SiFA compounds comprising a poly-glutamate linker

In the following, the sequence *H*-glu¹-glu²-glu³-glu⁴-glu⁵-glu⁶-Ala⁷-Tyr⁸-Gly⁹-Trp¹⁰-Nle¹¹-Asp¹²-Phe¹³-NH₂ is defined as F11N.

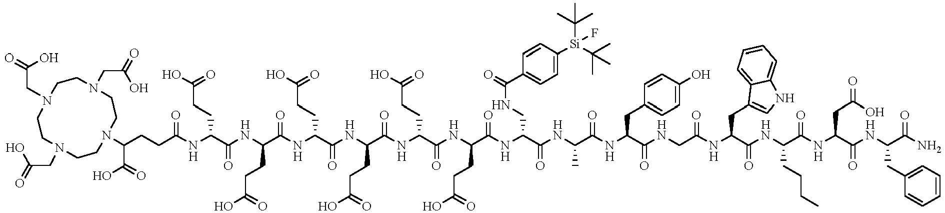

**DOTAGA-glu-[dap(SiFA)⁶]F11N (DOTAGA-MG-SiFA-5):** RP-HPLC (10→90% MeCN in H₂O with 0.1% TFA, 15 min, λ = 220 nm): *t*_{R} = 10.2 min *K*' = 5.0 MS (ESI, positive): m/z calculated for C₁₁₁H₁₅₄FN₂₁O₃₉Si: 2452.0, found: m/z = 1226.8 [M+2H]²⁺.

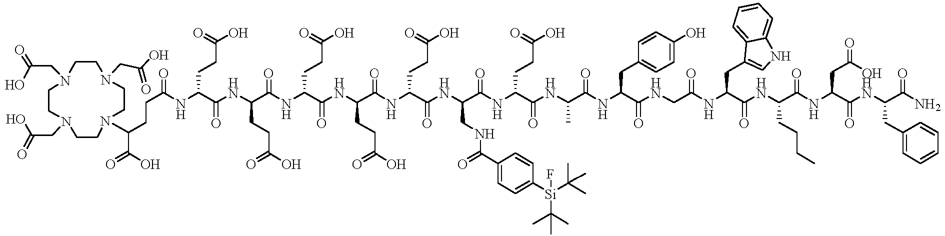

**DOTAGA-glu-[dap(SiFA)⁵]F11N (DOTAGA-MG-SiFA-6):** RP-HPLC (10→90% MeCN in H₂O with 0.1% TFA, 15 min, λ = 220 nm) *t*_{R} = 9.6 min, *K'* = 4.7; MS (ESI, positive): m/z calculated for C₁₁₁H₁₅₄FN₂₁O₃₉Si: 2452.0, found: m/z = 1226.4 [M+2H]²⁺.

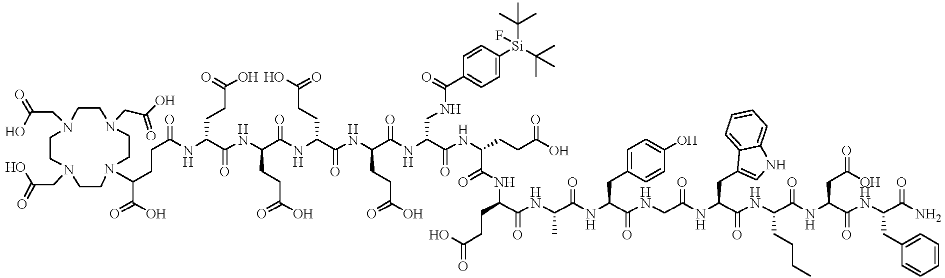

**DOTAGA-glu-[dap(SiFA)⁴]F11N (DOTAGA-MG-SiFA-7):** RP-HPLC (10→90% MeCN in H₂O with 0.1% TFA, 15 min, λ = 220 nm): *t*_{R} = 9.3 min, K' = 5.5; MS (ESI, positive): m/z calculated for C₁₁₁H₁₅₄FN₂₁O₃₉Si: 2452.0, found: m/z = 1226.4 [M+2H]²⁺.

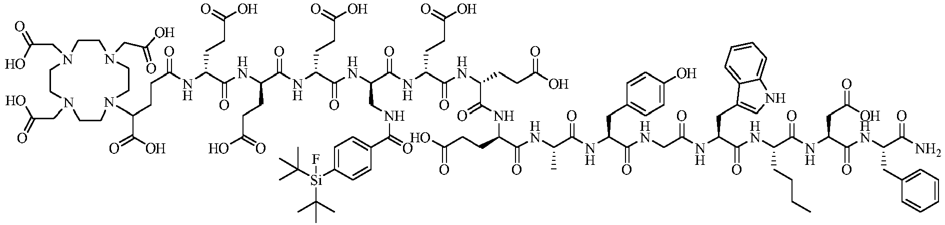

**DOTAGA-glu-[dap(SiFA)³]F11N (DOTAGA-MG-SiFA-8):** RP-HPLC (10→90% MeCN in H₂O with 0.1% TFA, 15 min, λ = 220 nm): *t*_{R} = 9.5 min, K' = 4.6; MS (ESI, positive): m/z calculated for C₁₁₁H₁₅₄FN₂₁O₃₉Si: 2452.0, found: m/z = 1226.7 [M+2H]²⁺.

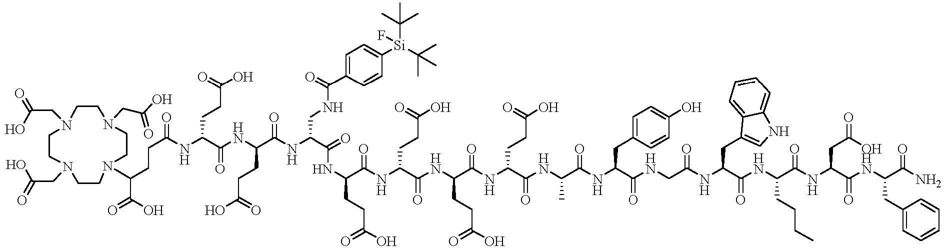

**DOTAGA-glu-[dap(SiFA)²]F11N (DOTAGA-MG-SiFA-9):** Analytical RP-HPLC (10→90% MeCN in H₂O with 0.1% TFA, 15 min, λ = 220 nm): *t*_{R} = 9.1 min, K' = 4.4; MS (ESI, positive): m/z calculated for C₁₁₁H₁₅₄FN₂₁O₃₉Si: 2452.0, found: m/z = 1227.3 [M+2H]²⁺.

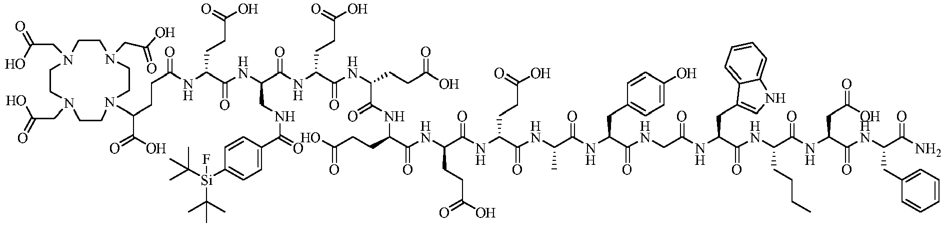

**DOTAGA-glu-[dap(SiFA)¹]F11N (DOTAGA-MG-SiFA-10):** RP-HPLC (10→90% MeCN in H₂O with 0.1% TFA, 15 min, λ = 220 nm): *t*_{R} = 9.2 min, *K'* = 4.5; MS (ESI, positive): m/z calculated for C₁₁₁H₁₅₄FN₂₁O₃₉Si: 2452.0, found: m/z = 1227.4 [M+2H]²⁺.

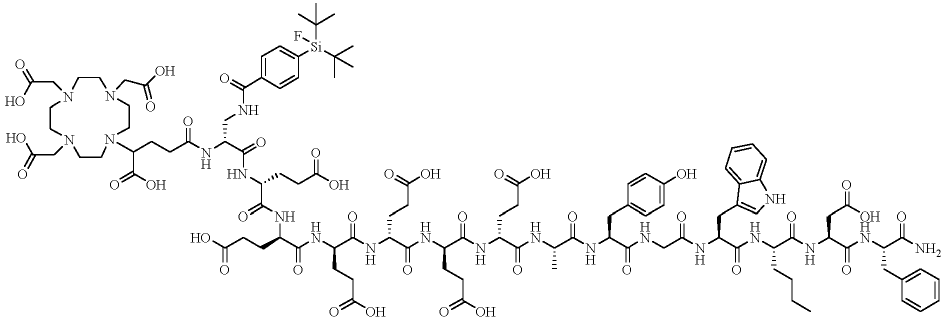

**DOTAGA-dap(SiFA)-F11N (DOTAGA-MG-SiFA-11):** RP-HPLC (10→90% MeCN in H₂O with 0.1% TFA, 15 min, λ = 220 nm): *t*_{R} = 9.6 min, *K'* = 5.6; MS (ESI, positive): m/z calculated for C₁₁₁H₁₅₄FN₂₁O₃₉Si: 2452.0, found: m/z = 1227.0 [M+2H]²⁺.

In the following, the sequence *H*-*γ*-glu¹-γ-glu²-γ-glu³-γ-glu⁴-γ-glu⁵-*γ*-glu⁶-Ala⁷-Tyr⁸-Gly⁹-Trp¹⁰-Nle¹¹-Asp¹²-Phe¹³-NH₂ is defined as *γ*-F11N.

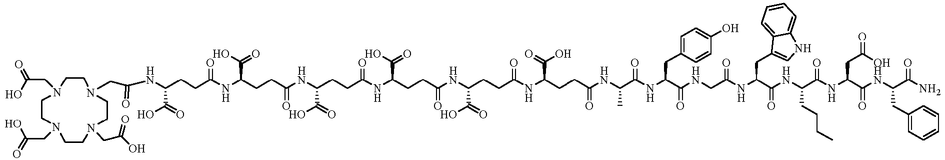

**DOTA-PP-γ-F11N:** RP-HPLC (10→90% MeCN in H₂O with 0.1% TFA, 15 min, λ = 220 nm): *t*_{R} = 7.1 min, K' = 3.2; MS (ESI, positive): m/z calculated for C₉₀H₁₂₃N₁₉O₃₅: 2029.8, found: m/z = 1015.7 [M+2H]²⁺.

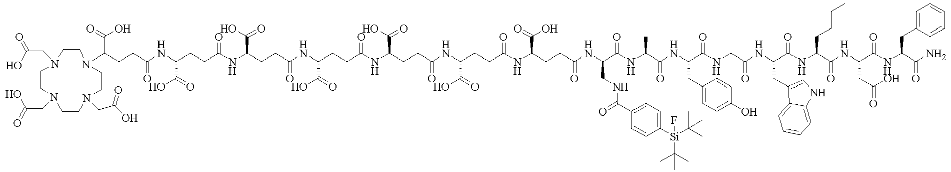

**DOTAGA-γ-glu-[dap(SiFA)⁶]γ-F11N (DOTAGA-MG-SiFA-13):** Analytical RP-HPLC (10→90% MeCN in H₂O with 0.1% TFA, 15 min, λ = 220 nm): *t*_{R} = 10.1 min K' = 6.1 MS (ESI, positive): m/z calculated for C₁₁₁H₁₅₄FN₂₁O₃₉Si: 2452.0, found: m/z = 1227.2 [M+2H]²⁺.

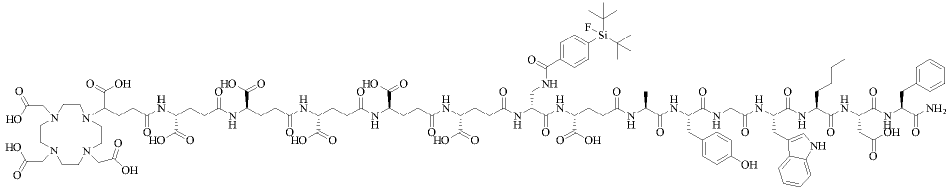

**DOTAGA-γ-glu-[dap(SiFA)⁵]γ-F11N (DOTAGA-MG-SiFA-14):** RP-HPLC (10→90% MeCN in H₂O with 0.1% TFA, 15 min, λ = 220 nm) *t*_{R} = 9.6 min, *K'* = 5.7; MS (ESI, positive): m/z calculated for C₁₁₁H₁₅₄FN₂₁O₃₉Si: 2452.0, found: m/z = 1227.3 [M+2H]²⁺.

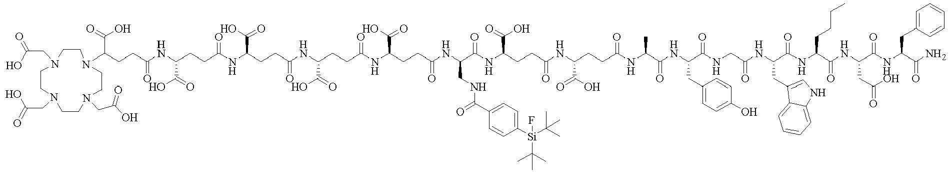

**DOTAGA-γ-glu-[dap(SiFA)⁴]γ-F11N (DOTAGA-MG-SiFA-15):** RP-HPLC (10→90% MeCN in H₂O with 0.1% TFA, 15 min, λ = 220 nm): *t*_{R} = 9.3 min, K' = 5.5; MS (ESI, positive): m/z calculated for C₁₁₁H₁₅₄FN₂₁O₃₉Si: 2452.0, found: m/z = 1226.4 [M+2H]²⁺.

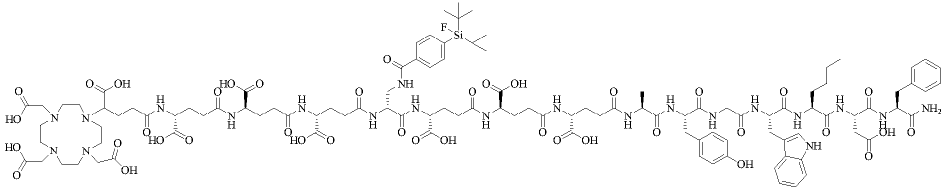

**DOTAGA-γ-glu-[dap(SiFA)³]γ-F11N (DOTAGA-MG-SiFA-16):** RP-HPLC (10→90% MeCN in H₂O with 0.1% TFA, 15 min, λ = 220 nm): *t*_{R} = 9.1 min, *K'* = 5.4; MS (ESI, positive): m/z calculated for C₁₁₁H₁₅₄FN₂₁O₃₉Si: 2452.0, found: m/z = 1226.7 [M+2H]²⁺.

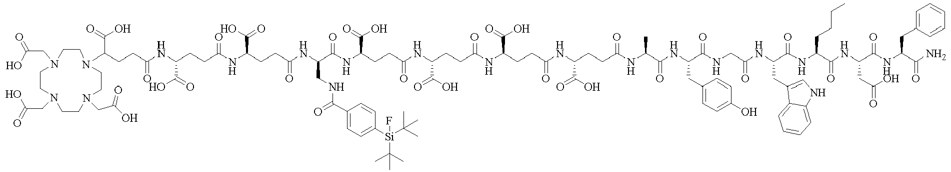

**DOTAGA-γ-glu-[dap(SiFA)²]γ-F11N (DOTAGA-MG-SiFA-17):** RP-HPLC (10→90% MeCN in H₂O with 0.1% TFA, 15 min, λ = 220 nm): *t*_{R} = 9.5 min, K' = 5.6; MS (ESI, positive): m/z calculated for C₁₁₁H₁₅₄FN₂₁O₃₉Si: 2452.0, found: m/z = 1226.6 [M+2H]²⁺.

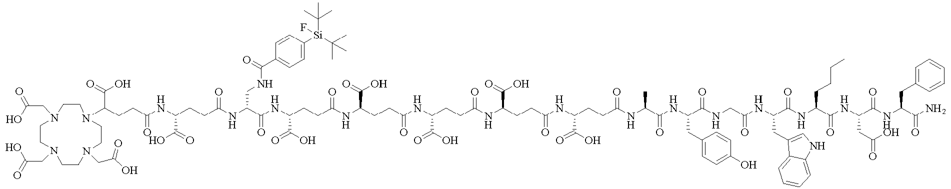

**DOTAGA-γ-glu-[dap(SiFA)¹]γ-F11N (DOTAGA-MG-SiFA-18):** RP-HPLC (10→90% MeCN in H₂O with 0.1% TFA, 15 min, λ = 220 nm): *t*_{R} = 9.6 min, K' = 5.7; MS (ESI, positive): m/z calculated for C₁₁₁H₁₅₄FN₂₁O₃₉Si: 2452.0, found: m/z = 1227.2 [M+2H]²⁺, 817.8 [M+3H]³⁺.

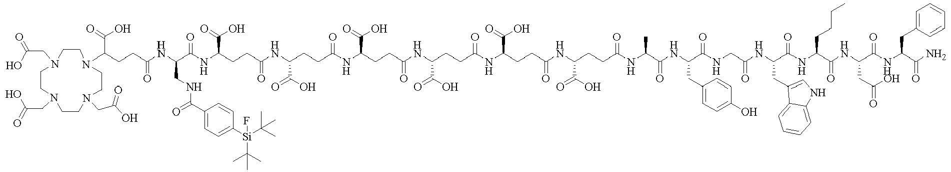

**DOTAGA-dap(SiFA)-γ-F11N (DOTAGA-MG-SiFA-19):** RP-HPLC (10→90% MeCN in H₂O with 0.1% TFA, 15 min, λ = 220 nm): *t*_{R} = 9.5 min, *K'* = 5.6; MS (ESI, positive): m/z calculated for C₁₁₁H₁₅₄FN₂₁O₃₉Si: 2452.0, found: m/z = 1227.3 [M+2H]²⁺, 818.7 [M+3H]³⁺.

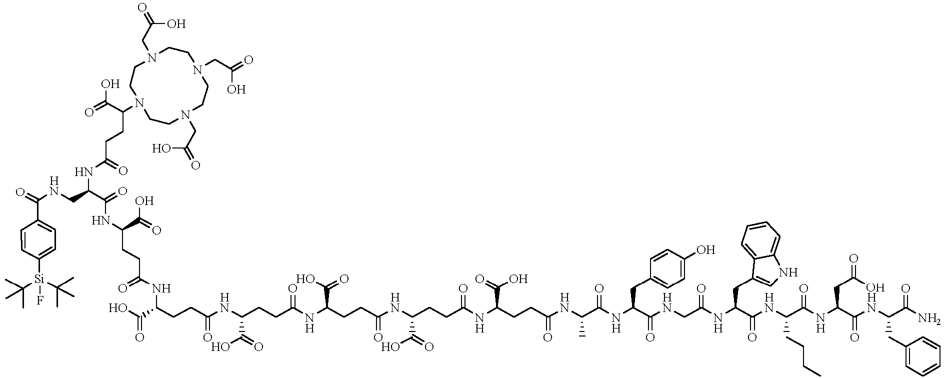

**DOTA-MG-SiFA-19: RP-HPLC (10→90% MeCN in H₂O with 0.1% TFA, 15 min, λ = 220 nm):** *t*_{R} = 10.0 min; MS (ESI, positive): m/z calculated for C₁₀₈H₁₅₀FN₂₁O₅₇Si: 2380.0, found: m/z = 1190.9 [M+2H]²⁺, 794.4 [M+3H]³⁺.

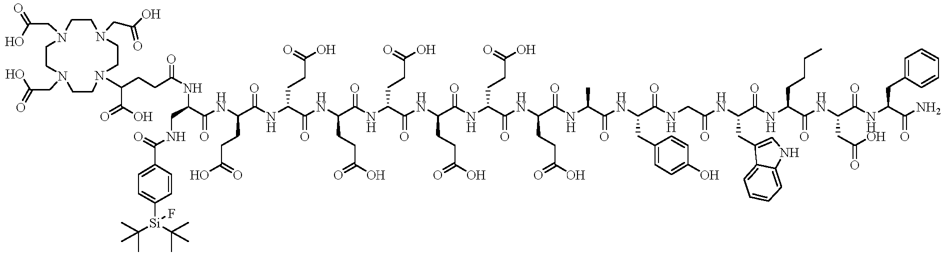

**DOTAGA-dap(SiFA)-glu-F11N (DOTAGA-MG-SiFA-20):** RP-HPLC (10→90% MeCN in H₂O with 0.1% TFA, 15 min, λ = 220 nm): *t*_{R} = 9.6 min, K' = 4.7; MS (ESI, positive): m/z calculated for C₁₁₆H₁₆₁FN₂₂O₄₂Si: 2581.1, found: m/z = 1292.0 [M+2H]²⁺.

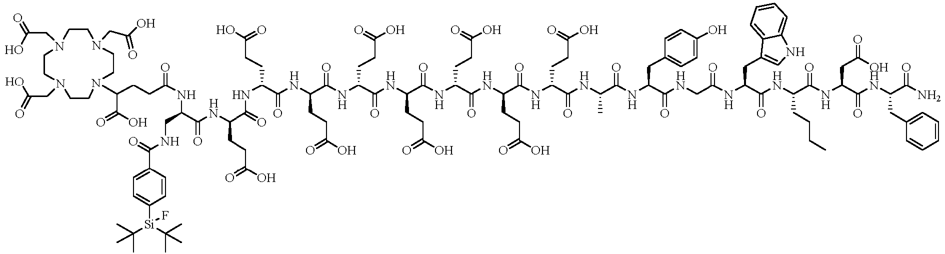

**DOTAGA-dap(SiFA)-(glu)₂-F11N (DOTAGA-MG-SiFA-21):** Analytical RP-HPLC (10→90% MeCN in H₂O with 0.1% TFA, 15 min, λ = 220 nm): *t*_{R} = 9.4 min, K' = 4.6; MS (ESI, positive): m/z calculated for C₁₂₂H₁₆₉FN₂₂O₄₅Si: 2710.1, found: m/z = 1355.9 [M+2H]²⁺.

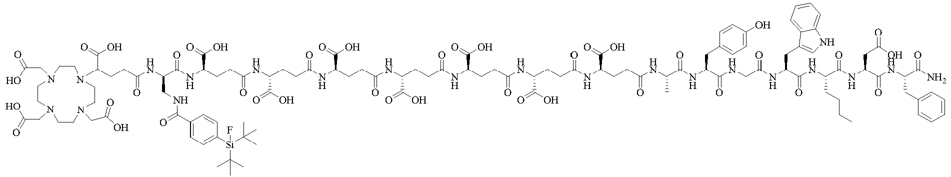

**DOTAGA-dap(SiFA)-γ-glu-γ-F11N (DOTAGA-MG-SiFA-22):** Analytical RP-HPLC (10→90% MeCN in H₂O with 0.1% TFA, 15 min, λ = 220 nm): *t*_{R} = 9.4 min, K' = 4.6; MS (ESI, positive): m/z calculated for C₁₁₆H₁₆₁FN₂₂O₄₂Si: 2581.1, found: m/z = 1291.8 [M+2H]²⁺, 861.6 [M+3H]³⁺.

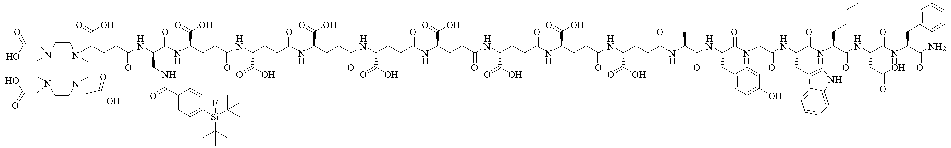

**DOTAGA-dap(SiFA)-(γ-glu)₂-γ-F11N (DOTAGA-MG-SiFA-23):** Analytical RP-HPLC (10→90% MeCN in H₂O with 0.1% TFA, 15 min, λ = 220 nm): *t*_{R} = 9.3 min, K' = 4.5; MS (ESI, positive): m/z calculated for C₁₂₁H₁₆₈FN₂₃O₄₅Si: 2710.1, found: m/z = 1356.1 [M+2H]²⁺, 904.2 [M+3H]³⁺.

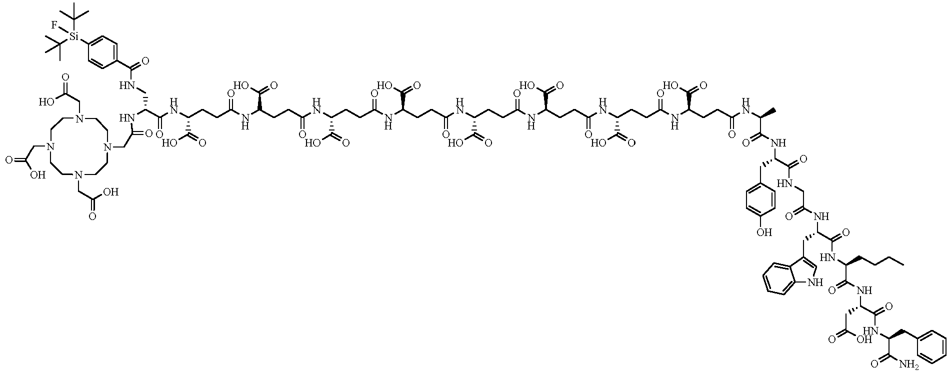

**DOTA-MG-SiFA-23:** RP-HPLC (10→90% MeCN in H₂O with 0.1% TFA, 15 min, λ = 220 nm): *t*_{R} = 9.8 min; MS (ESI, positive): m/z calculated for C₁₁₈H₁₆₄FN₂₃O₄₃Si: 2638.1, found: m/z = 1320.3 [M+2H]²⁺, 880.5 [M+3H]³⁺.

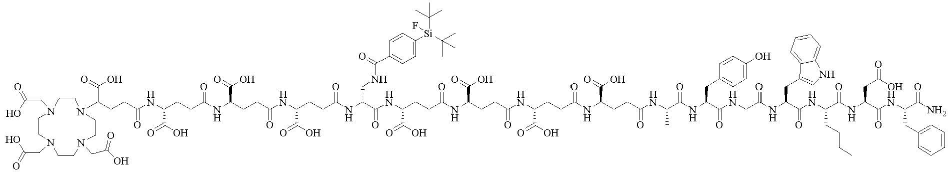

**DOTAGA-(γ-glu)₂-[dap(SiFA)²]γ-F11N (DOTAGA-MG-SiFA-24):** Analytical RP-HPLC (10→90% MeCN in H₂O with 0.1% TFA, 15 min, λ = 220 nm): *t*_{R} = 9.4 min, K' = 4.6; MS (ESI, positive): m/z calculated for C₁₁₆H₁₆₁FN₂₂O₄₂Si: 2581.1, found: m/z = 1292.5 [M+2H]²⁺, 862.0 [M+3H]³⁺.

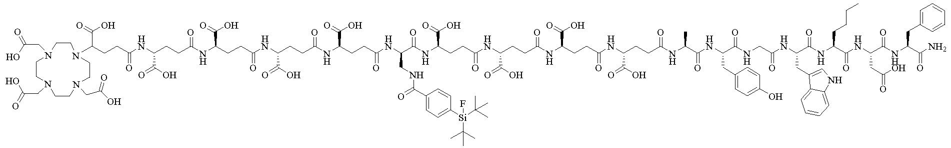

**DOTAGA-(γ-glu)₃-[dap(SiFA)²]γ-F11N (DOTAGA-MG-SiFA-25):** Analytical RP-HPLC (10→90% MeCN in H₂O with 0.1% TFA, 15 min, λ = 220 nm): *t*_{R} = 9.3 min, K' = 4.5; MS (ESI, positive): m/z calculated for C₁₂₂H₁₆₉FN₂₂O₄₅Si: 2710.1, found: m/z = 1357.1 [M+2H]²⁺, 905.1 [M+3H]³⁺.

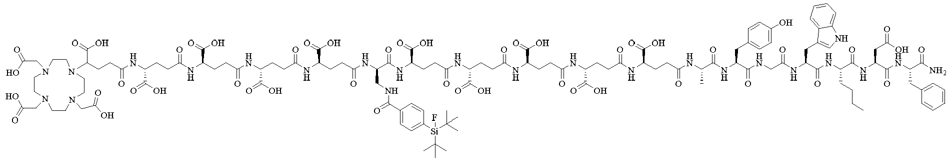

**DOTAGA-(γ-glu)₄-[dap(SiFA)¹]γ-F11N (DOTAGA-MG-SiFA-26):** RP-HPLC (10→90% MeCN in H₂O with 0.1% TFA, 15 min, λ = 220 nm): *t*_{R} = 9.2 min, K' = 4.5; MS (ESI, positive): m/z calculated for C₁₂₆H₁₇₅FN₂₄O₄₈Si: 2839.2, found: m/z = 1422.4 [M+2H]²⁺, 948.3 [M+3H]³⁺. 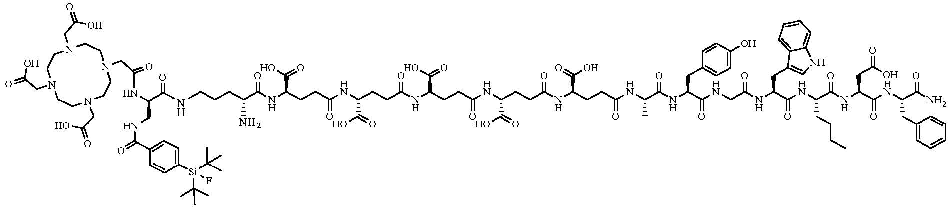 **DOTA-dap(SiFA)-γ-orn-(γ-glu)₅-F11N (DOTA-MG-SiFA-27):** RP-HPLC (10→90% MeCN in H₂O with 0.1% TFA, 15 min, λ = 220 nm): *t*_{R} = 9.5 min; MS (ESI, positive): m/z calculated for C₁₀₈H₁₅₃FN₂₃O₃₅Si: 2366.1, found: m/z = 1183.8 [M+2H]²⁺, 789.6 [M+3H]³⁺.

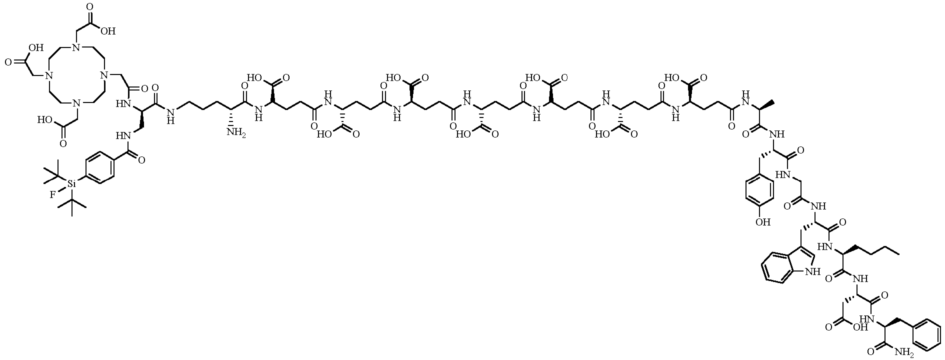

**DOTA-dap(SiFA)-γ-orn-(γ-glu)₇-F11N (DOTA-MG-SiFA-28):** RP-HPLC (10→70% MeCN in H₂O with 0.1% TFA, 15 min, λ = 220 nm): *t*_{R} = 11.5 min; MS (ESI, positive): m/z calculated for C₁₁₈H₁₆₇FN₂₄O₄₁Si: 2623.2, found: m/z = 1312.6 [M+2H]²⁺.

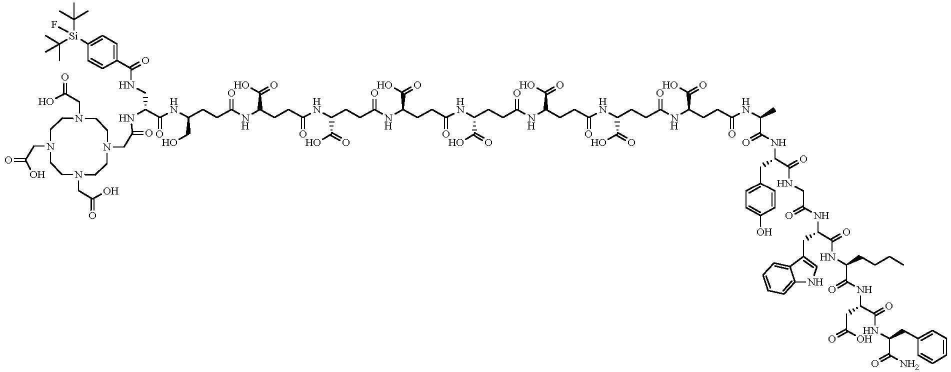

**DOTA-dap(SiFA)-Glutamol-(γ-glu)₇-F11N (DOTA-MG-SiFA-29):** RP-HPLC (10→70% MeCN in H₂O with 0.1% TFA, 15min, A=220nm): *t*_{R}=11.5 min; MS (ESI, positive): m/z calculated for C₁₁₈H₁₆₆FN₂₃O₄₂Si: 2625.8, found: m/z = 1313.6 [M+2H]²⁺, 876.1 [M+3H]³⁺.

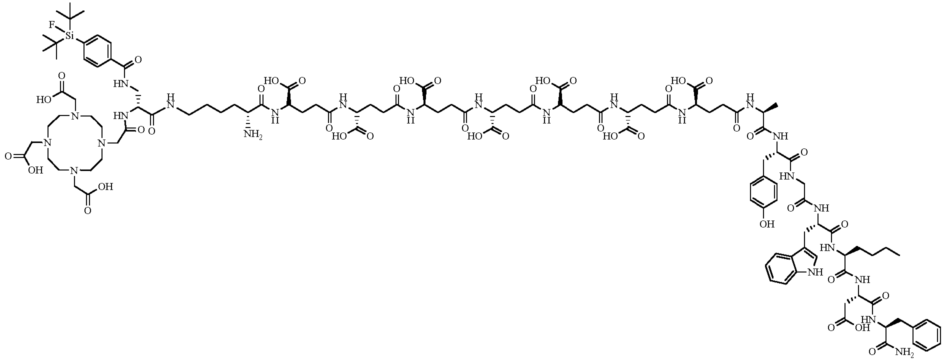

**DOTA-dap(SiFA)-γ-lys-(γ-glu)₇-F11N (DOTA-MG-SiFA-30):** RP-HPLC (10→70% MeCN in H₂O with 0.1% TFA, 15 min, λ = 220 nm): *t*_{R} = 11.4 min; MS (ESI, positive): m/z calculated for C₁₁₉H₁₆₉FN₂₄O₄₁Si: 2637.2, found: m/z = 1319.5 [M+2H]²⁺, 880.1 [M+3H]³⁺.

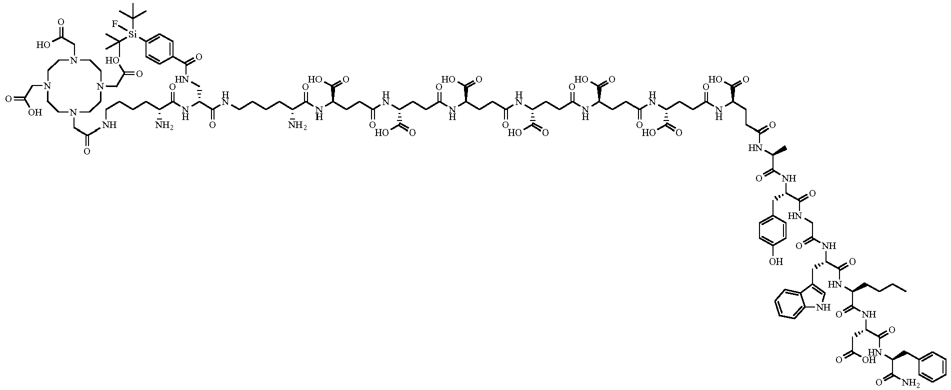

**DOTA-γ-lys-dap(SiFA)-γ-lys-(γ-glu)₇-F11N (DOTA-MG-SiFA-31):** RP-HPLC (10→70% MeCN in H₂O with 0.1% TFA, 15 min, λ = 220 nm): *t*_{R} =11.0 min; MS (ESI, positive): m/z calculated for C₁₂₅H₁₈₁FN₂₆O₄₂Si: 2767.1, found: m/z = 1383.7 [M+2H]²⁺, 922.9 [M+3H]³⁺.

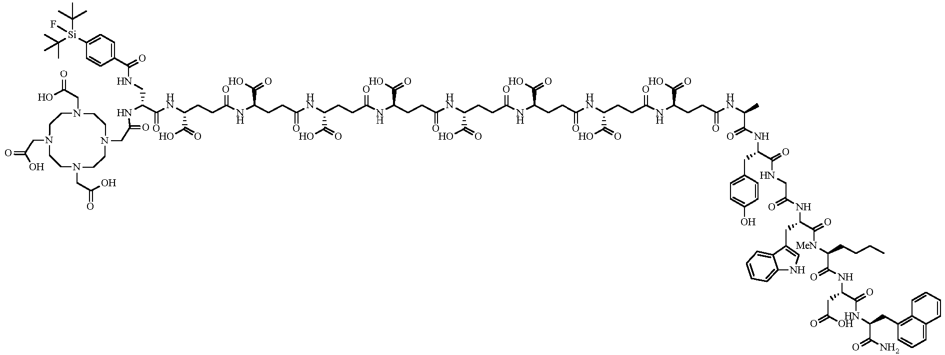

**DOTA-dap(SiFA)-(γ-glu)₈-MGS5 (DOTA-MG-SiFA-40):** RP-HPLC (10→70% MeCN in H₂O with 0.1% TFA, 15 min, λ = 220 nm): *t*_{R} = 12.5 min; MS (ESI, positive): m/z calculated for C₁₂₃H₁₆₈FN₂₃O₄₃Si: 2702.1, found: m/z = 1352.8 [M+2H]²⁺.

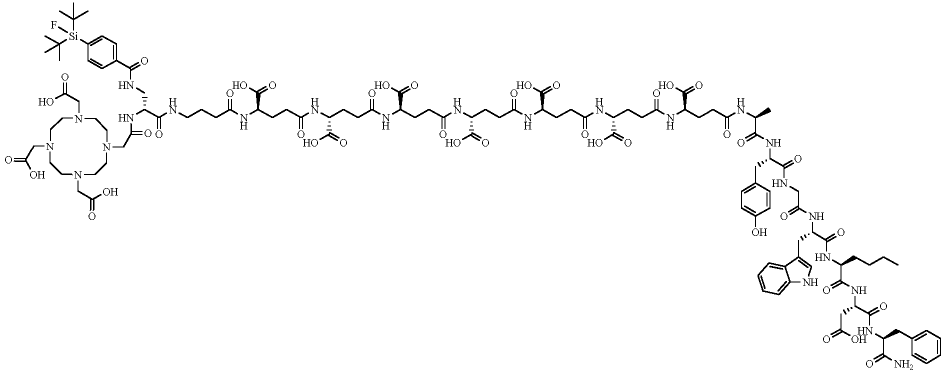

**DOTA(-dap(SiFA)-GABA-(γ-glu)₇-F11N (DOTA-MG-SiFA-44):** RP-HPLC (10→70% MeCN in H₂O with 0.1% TFA, 15 min, λ = 220 nm): *t*_{R} = 11.8 min; MS (ESI, positive): m/z calculated for C₁₁₇H₁₆₄FN₂₃O₄₁Si: 2594.1, found: m/z = 1298.9 [M+2H]²⁺, 866.3 [M+3H]³⁺.

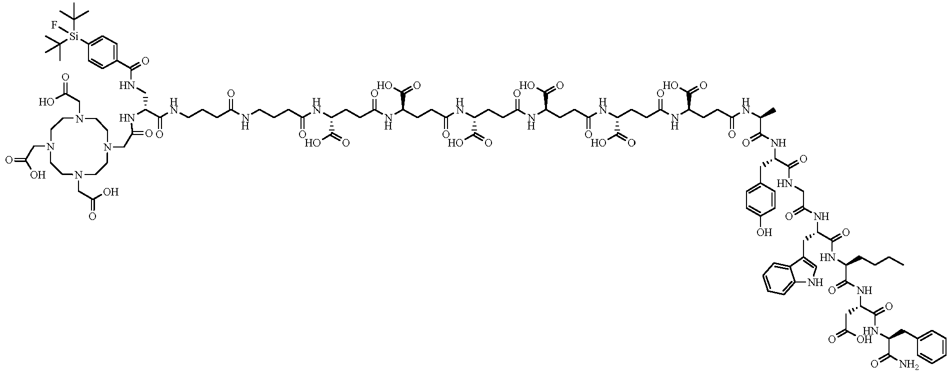

**DOTA-dap(SiFA)-(GABA)₂-(γ-glu)₆-F11N (DOTA-MG-SiFA-45):** RP-HPLC (10→70% MeCN in H₂O with 0.1% TFA, 15 min, λ = 220 nm): *t*_{R} = 11.8 min; MS (ESI, positive): m/z calculated for C₁₁₆H₁₆₄FN₂₃O₃₉Si: 2550.1, found: m/z = 1277.0 [M+2H]²⁺, 851.6 [M+3H]³⁺.

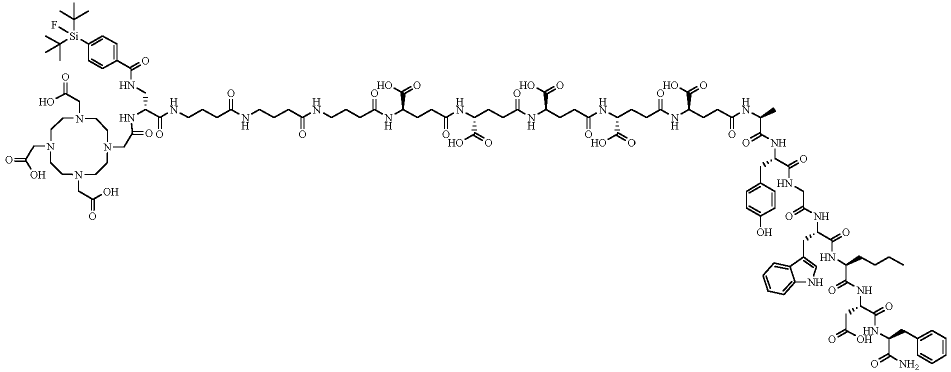

**DOTA-dap(SiFA)-(GABA)₃-(*γ*-glu)₅-F11N (DOTA-MG-SiFA-46):** RP-HPLC (10→70% MeCN in H₂O with 0.1% TFA, 15 min, λ = 220 nm): *t*_{R} = 11.9 min; MS (ESI, positive): m/z calculated for C₁₁₅H₁₆₄FN₂₃O₃₇Si: 2507.8, found: m/z = 1254.5 [M+2H]²⁺, 836.7 [M+3H]³⁺.

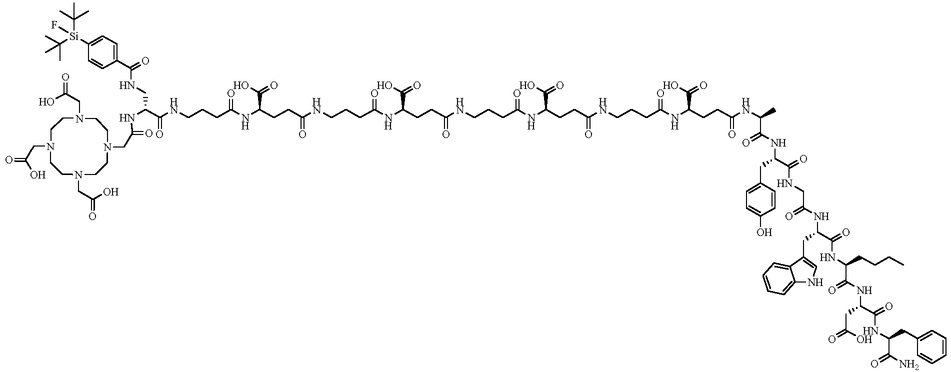

**DOTA-dap(SiFA)-(GABA-γ-glu)₄-F11N (DOTA-MG-SiFA-47):** RP-HPLC (10→70% MeCN in H₂O with 0.1% TFA, 15 min, λ = 220 nm): *t*_{R} = 11.8 min; MS (ESI, positive): m/z calculated for C₁₁₄H₁₆₄FN₂₃O₃₅Si: 2462.2, found: m/z = 1232.5 [M+2H]²⁺, 822.0 [M+3H]³⁺.

### 2.2. In vitro and in vivo results of MG-SiFA compounds comprising a poly-glutamate linker

Several MG-SiFA compounds were synthesized and evaluated *in vitro* ^{nat/177}Lu-labeled. CCK-2R affinity on AR4-2J cells (*IC*₅₀) as well as lipophilicity (log*D*_{7.4}) was determined. Moreover, the reference ligands ^{nat/177}Lu-DOTA-PP-F11N and ^{nat/177}Lu-CP04 (^{nat/177}Lu-DOTA-(glu)₆-Ala-Tyr-Gly-Trp-Met-Asp-Phe-NH₂) were synthesized and evaluated as well **(Tab. 2).**

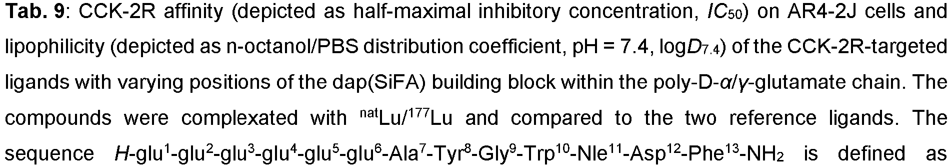

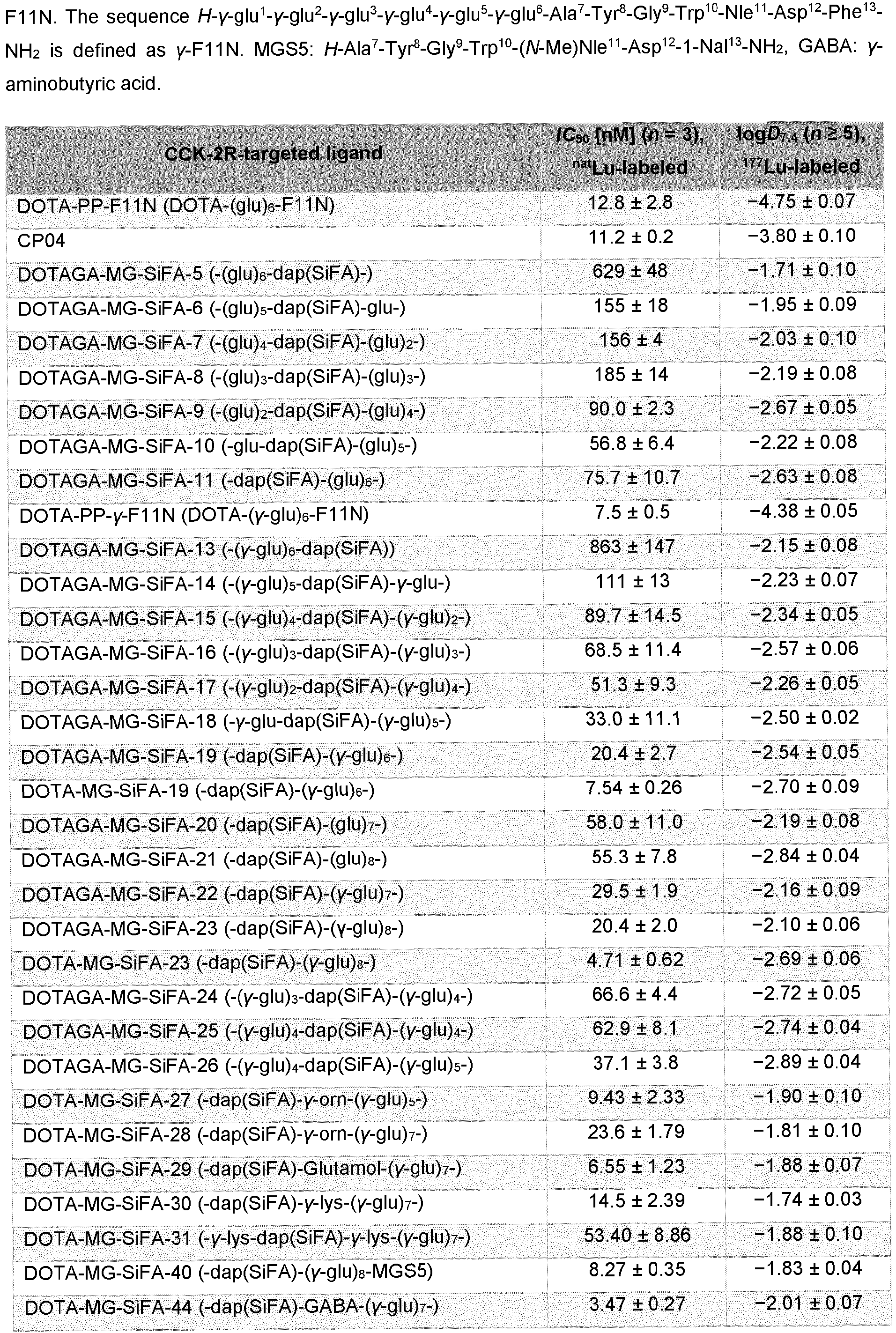

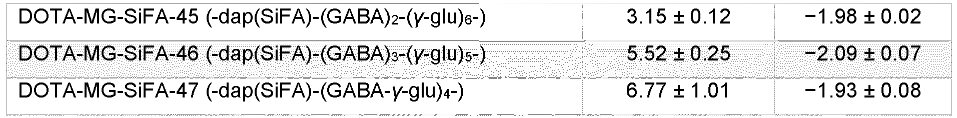

In general, minigastrin analogues comprising a poly-*γ*-glu linker ([^{nat}Lu]Lu-DOTAGA-MG-SiFA13-19) showed improved CCK-2R affinity compared to their counterparts containing the exact amount of *α*-glu residues within their linker ([^{nat}Lu]Lu-DOTAGA-MG-SiFA5-11). This was also observed for [^{nat}Lu]Lu-DOTA-PP-*γ*-F11N, which displayed a 1.7-fold lower *IC*₅₀ value than [^{nat}Lu]Lu-DOTA-PP-F11N. It is suggested that this is due to enhanced separation of the dap(SiFA) but also the chelator moiety with regard to the binding sequence (Ala⁷-Tyr⁸-Gly⁹-Trp¹⁰-Nle¹¹-Asp¹²-Phe¹³-NH₂), as the respective peptide backbone is distinctly prolonged using *γ*-glu instead of *α*-glu. In case of the compounds containing a dap(SiFA) moiety, highest CCK-2R affinity was observed when this group was separated by at least five or six *α*-/γ-glu moieties. Not surprisingly, among the SiFA-containing MG derivatives that additionally comprise a DOTAGA chelator, [^{nat}Lu]Lu-DOTAGA-MG-SiFA-19 revealed highest CCK-2R affinity. Interestingly, further addition of *γ*-glu moieties ([^{nat}Lu]Lu-DOTAGA-MG-SiFA-22/-23) did not result in improved CCK-2R affinity. However, substitution of DOTAGA by DOTA in [^{nat}Lu]Lu-DOTAGA-MG-SiFA-23 led to a massively increased CCK-2R affinity ([^{nat}Lu]Lu-DOTA-MG-SiFA-23: 4.71 ± 0.62 nM), which surpassed even the reference ligands. It is assumed that the receptor prefers a neutral overall charge of the chelate at the respective site. Similar observation was made for substitution of DOTAGA by DOTA in [^{nat}Lu]Lu-DOTAGA-MG-SiFA-19, which led to threefold lower *IC*₅₀ value.

Not surprisingly, all ¹⁷⁷Lu-labeled MG-SiFA ligands also revealed an elevated lipophilicity compared to the non-SiFA-containing reference ligands. However, as mentioned earlier it is suggested that this highly hydrophilic character impedes an enhanced tumor accumulation as a consequence of rapid clearance of the administered activity into the bladder. For this reason, it is assumed that the decreased hydrophilicity of the ¹⁷⁷Lu-labeled MG-SiFA conjugates results in a prolonged circulation time *in vivo* and thus enhanced tumor uptake. Most of our ¹⁷⁷Lu-labeled MG-SiFA compounds displayed log*D*_{7.4} values in a range between -3 and -2. *Klingler et al.* reported pharmacophore-modified CCK-2R-targeted ligands, which exhibited logD values in a similar range to our MG-SiFA conjugates. Noteworthy, tumor accumulation was excessively high for their ligands [⁶⁸Ga]Ga-/[¹¹¹In]In-/[¹⁷⁷Lu]Lu-DOTA-MGS5 and [^{99m}Tc]Tc-HYNIC-MGS11 [30,34]. Therefore, it is supposed that this lipophilicity range could be perfectly suited for CCK-2R-addressing derivatives.

For a better comparison, the most promising DOTAGA-containing conjugates comprising a poly-*α-*glutamate and a poly-*γ*-glutamate linker, respectively, were chosen for further studies *in vivo.* Thus, biodistribution studies with both [¹⁷⁷Lu]Lu-DOTAGA-MG-SiFA-21 and [¹⁷⁷Lu]Lu-DOTAGA-MG-SiFA-19 were carried out at 24 h p.i. and compared to the reference [¹⁷⁷Lu]Lu-DOTA-PP-F11N (Fig. 24).

Furthermore, [¹⁷⁷Lu]Lu-DOTA-MG-SiFA-23, which displayed higher CCK-2R affinity than the reference compounds, was evaluated *in vivo* at 1 and 24 h p.i. (Fig. 25).

As discussed earlier, [^{nat}Lu]Lu-DOTAGA-MG-SiFA-21 displayed an approximately fivefold and [^{nat}Lu]Lu-DOTAGA-MG-SiFA-19 an approximately twofold increased *IC*₅₀ value than [^{nat}Lu]Lu-DOTA-PP-F11N, while [^{nat}Lu]Lu-DOTA-MG-SiFA-23 displayed an almost threefold lower *IC*₅₀ value than [^{nat}Lu]Lu-DOTA-PP-F11N. All three MG-SiFA derivatives also revealed a distinctly higher lipophilicity than the reference ligand. Nevertheless, [¹⁷⁷Lu]Lu-DOTA-MG-SiFA-23 revealed the highest activity levels in the tumor at 24 h p.i. (> 25 %ID/g), which were more than 8-fold higher than those of [¹⁷⁷Lu]Lu-DOTA-PP-F11N. Interestingly, both DOTAGA-MG-SiFA compounds showed three- to sixfold increased activity levels in the tumor at 24 h p.i. despite their lower CCK2R affinity, which supports our hypothesis of a benefit of a decelerated activity clearance [35]. A second reference ligand, [¹⁷⁷Lu]Lu-CP04, which is structurally highly related to [¹⁷⁷Lu]Lu-DOTA-PP-F11N is reported to exhibit 0.63 ± 0.08 %ID/g in tumor tissue at 24 h p.i. in A431-CCK-2R(+) tumor-bearing mice, which further strengthens our data [36]. The improved tumor uptake and decelerated clearance over time was also highlighted by *µ*SPECT/CT imaging at 1, 4 and 24 h p.i. **(****Fig. 26****)** for the DOTAGA-conjugated MG-SIFA compounds as well as at 1 and 24 h p.i. for [¹⁷⁷Lu]Lu-DOTA-MG-SiFA-23 **(****Fig. 27****).** Accumulation of both [¹⁷⁷Lu]Lu-DOTAGA-MG-SiFA-19 and [¹⁷⁷Lu]Lu-DOTA-MG-SiFA-23 in the CCK-2R-positive tumor and stomach was specific, which was shown by competition studies via co-administration of excess of [^{nat}Lu]Lu-DOTA-MGS5 **(****Fig. 28****).**

Moreover, it was evident that tumor accumulation was also distinctly higher at 1 h p.i. for all MG-SiFA ligands compared to the reference, which is important for a potential use of these compounds for imaging when labeled with fluorine-18. Due to the presence of a chelator and a SiFA moiety, very similar or even the same biodistribution profiles for a ^{nat}Lu/¹⁸F-labeled and a ¹⁷⁷Lu/¹⁹F-labeled MG-SiFA ligand, respectively are expected. Activity levels in the stomach were also higher for all MG-SiFA ligands compared to the reference, which was expected, as the stomach is a CCK-2R-positive organ. Interestingly, activity levels in the liver were comparably low to those of [¹⁷⁷Lu]Lu-DOTA-PP-F11N despite their increased lipophilicity. It is thus suggested that the introduction of a SiFA moiety into minigastrin analogues is well suited. However, it has to be mentioned that all three MG-SiFA compounds suffered from massively elevated activity levels in the kidneys at 24 h p.i. (> 80 %ID/g), which was not visible for the reference compound. While there is not yet an explanation for this observation it is assumed that there seems to be an unknown synergistic effect of the SiFA moiety and the negative charges within the linker that causes this increased kidney uptake and retention. However, this should be of minor concern when ¹⁸F-labeled, as the physical half-life of this isotope is short.

Nonetheless, it was tried to address this issue by reduction or separation of negative charges in close proximity to the dap(SiFA) building block. The N-terminal *γ*-glu moiety was substituted either by *γ*-orn (DOTA-MG-SiFA-27/-28), Glutamol (DOTA-MG-SiFA-29) or *γ*-lys (DOTA-MG-SiFA-30/-31). Interestingly, both CCK-2R affinity and lipophilicity was noticeably impaired by those substitutions. Each of these compounds ([¹⁷⁷Lu]Lu-DOTA-MG-SiFA-27-31) revealed a log*D*_{7.4} value of almost one magnitude higher than that of [¹⁷⁷Lu]Lu-DOTA-MG-SiFA-23 **(Tab. 9).** Moreover, *IC*₅₀ values were slightly enhanced for [¹⁷⁷Lu]Lu-DOTA-MG-SiFA-27 and -29 but noticeably increased for [¹⁷⁷Lu]Lu-DOTA-MG-SiFA-28, -30 and particularly -31. It is thus assumed that the charge distribution at the N-terminal region is very delicate, as small modifications seem to have a major impact.

Based on these results, it was decided to reduce negative charges by substitution of one or more *γ*-glu by *γ*-aminobutyric acid (GABA) moieties to retain the backbone length but with a less amount of negative charges, which resulted in the four compounds DOTA-MG-SiFA-44-47. Each of these ¹⁷⁷Lu-labeled compounds revealed a similar log*D*_{7.4} value, which was more than half a magnitude higher than that of [¹⁷⁷Lu]Lu-DOTA-MG-SiFA-23 **(Tab. 9).** However, CCK-2R affinity was comparable or even higher than that of [¹⁷⁷Lu]Lu-DOTA-MG-SiFA-23, which was surprising, as it was supposed that the negative charges are important for high CCK-2R affinity. Nevertheless, due to elevated lipophilicity, the GABA-comprising MG-SiFA compounds have not been evaluated *in vivo.*

Concluded, the functionality of the addition of a silicon-based fluoride acceptor building block into the linker section of minigastrin derivatives was proven. The resulting MG-SiFA compounds enable facile and rapid ¹⁸F-labeling for imaging of MTC via PET. Moreover, the addition of a lipophilic SiFA moiety also vastly improved overall pharmacokinetics due to a decelerated activity clearance, which resulted in distinctly increased activity levels in the tumor at 1 but also at 24 h p.i. Although evaluated at 24 h p.i., it has to be mentioned that a use of these MG-SiFA ligands is not recommended for targeted radiotherapy with lutetium-177, as these compounds suffer from elevated kidney retention. However, this elevated kidney uptake is of limited concern when ¹⁸F-labeled, as PSMA inhibitors generally reveal higher uptake values at 1 h p.i. The diagnostic value of DOTA-MG-SiFA-23 was confirmed by biodistribution and imaging studies at 1 h p.i. **(****Figs. 22** and **24****)** both ¹⁷⁷Lu- and ¹⁸F-labeled, which is why this compound holds great promise for a future clinical use for MTC imaging..

### Example 3 - Material and Methods

### 3.1. General

The Fmoc-(9-fluorenylmethoxycarbonyl-) and all other protected amino acid analogues were purchased from *Bachem Inc.* (Bubendorf, Switzerland), *Sigma-Aldrich GmbH* (Munich, Germany) or *Iris Biotech GmbH* (Marktredwitz, Germany). The H-Rink amide ChemMatrix^{®} resin (35-100 mesh particle size, 0.4-0.6 mmol/g loading) was purchased from *Sigma-Aldrich GmbH* (Munich, Germany). *CheMatech* (Dijon, France) delivered the chelators DOTA(*^{t}*Bu)₃ as well as DOTAGA(*^{t}*Bu)₄. Peptide syringes were obtained from *VWR International GmbH* (Bruchsal, Germany).

All necessary solvents and other organic reagents were purchased from either, *Alfa Aesar* (Karlsruhe, Germany), *Sigma-Aldrich GmbH* (Munich, Germany) or *VWR International GmbH* (Bruchsal, Germany). Solid-phase synthesis of the peptides was carried out by manual operation using a Scilogex MX-RL-E Analog Rotisserie Tube Rotator *(Scilogex,* Rocky Hill, CT, USA). H₂O was used after purification by a Barnstead MicroPure system *(Thermo Fisher Scientific Inc.,* Waltham, MA, USA).

Analytical and preparative reversed-phase high performance liquid chromatography (RP-HPLC) was performed using Shimadzu gradient systems *(Shimadzu Deutschland GmbH,* Neufahrn, Germany), each equipped with a SPD-20A UV/Vis detector (220 nm, 254 nm). Different gradients of MeCN (0.1% TFA) in H₂O (0.1% TFA) were used as eluents for all RP-HPLC operations.

For analytical measurements, a MultoKrom 100-5 C18 (5 µm, 125 × 4.6 mm) column (CS *Chromatographie GmbH,* Langerwehe, Germany) was used at a flow rate of 1 mL/min. Both specific gradients and the corresponding retention times *t*_{R} as well as the capacity factor K' are cited in the text. Preparative RP-HPLC purification was done with a MultoKrom 100-5 C18 (5 µm, 250 × 10 mm) column *(CS Chromatographie GmbH,* Langerwehe, Germany) at a constant flow rate of 10 mL/min.

Analytical and preparative radio RP-HPLC was performed using a MultoKrom 100-5 C18 (5 µm, 125 × 4.6 mm) column *(CS Chromatographie GmbH,* Langerwehe, Germany). Electrospray ionization-mass spectra for characterization of the substances were acquired on an expression^{L} CMS mass spectrometer *(Advion Ltd.,* Harlow, UK).

For radiolabeling, [¹⁷⁷Lu]LuCl₃ (Molar Activity (*A*_{M}) > 3,000 GBq/mg, 740 MBq/mL, 0.04 M HCl, *ITG GmbH,* Garching, Germany) was used. Radioactivity was detected through connection of the outlet of the UV-photometer to an AceMate 925-Scint Nal(TI) well-type scintillation counter from *EG&G Ortec* (Oak Ridge, TN, USA). Radioactive probes were measured by a WIZARD^{2®} 2480 Automatic *γ*-Counter *(Perkin Elmer,* Waltham, MA, USA) and determination of *IC*₅₀ values was carried out using GraphPad Prism 6 (*GraphPad Software Inc.,* San Diego, CA, USA). For radio TLC, a Scan-RAM^{™} Scanner with Laura^{™} software *(LabLogic Systems Ltd.,* Broomhill, Sheffield, United Kingdom) was used.

NMR spectra were recorded on a Bruker *(Billerica,* United States) AVHD-300 or AVHD-400 spectrometers at 300 K. Lyophilization was accomplished using an Alpha 1-2 LDplus lyophilizer *(Martin Christ Gefriertrocknungsanlagen GmbH,* Osterode am Harz, Deutschland) combined with a RZ-2 vacuum pump *(Vacuubrand GmbH & Co KG,* Olching, Germany).

For *in vitro* and *in vivo* studies, RPMI 1640 medium (supplemented with 5 mM L-Gln, 5 mL non-essential amino acids (100×) and 10% FCS) was used as nutrient medium. For culture passaging, a phosphate buffered saline (PBS Dulbecco, without Ca²⁺/Mg²⁺) solution with 0.1% EDTA *(v*/*v)* was used to detach the cells. All mentioned solutions as well as fetal bovine serum (FBS Superior) were obtained from *Biochrom GmbH* (Berlin, Germany). Solving of purified products was applied using Tracepur^{®} H₂O *(Merck KGaA,* Darmstadt, Germany). Bovine serum albumin (BSA) was purchased from *Biowest* (Nuaillé, France).

Cells were cultured in CELLSTAR^{®} cell culture flasks and seeded in 24-well plates *(Greiner Bio-One GmbH,* Kremsmünster, Austria) after being counted with a Neubauer hemocytometer *(Paul Marienfeld,* Lauda-Königshofen, Germany) using Trypan Blue (0.4% in 0.81% NaCl and 0.06% potassium phosphate) solution *(Sigma-Aldrich GmbH,* Munich, Germany). Cells were handled inside a MSC Advantage laminar flow cabinet and maintained in a Heracell 150i incubator *(Thermo Fisher Scientific Inc.,* Waltham, MA, USA) at 37 °C in a humidified 5% CO₂ atmosphere. The CCK-2R expressing rat pancreatic tumor cells AR4-2J were obtained from ECACC *(European Collection of Cell Cultures,* Salisbury, UK).

### 3.2. Synthesis protocols

### 3.2.1 Solid-phase peptide synthesis following the Fmoc-strategy

### On-resin peptide formation using DIPEA

The respective side-chain protected Fmoc-AA-OH (1.5 eq.) was dissolved in NMP and pre-activated by adding TBTU (1.5 eq.), HOAt (1.5 eq.) and DIPEA (4.5 eq.). After activation for 10 min, the solution was added to resin-bound free amine peptide and shaken for 1.5 h at room temperature (rt). Subsequently, the resin was washed with NMP (6 × 20 mL/g resin) and after Fmoc deprotection, the next amino acid was coupled analogously.

### On-resin peptide formation using 2,4,6-collidine

For the coupling reactions of amino acids to the free N-terminus of a peptide containing a dap(Dde) moiety or coupling of the dap(Dde) amino acid, 2,4,6-collidine was used as base. Therefore, the respective side-chain protected Fmoc-AA-OH (1.5 eq.) was dissolved in NMP and pre-activated by adding TBTU (1.5 eq.), HOAt (1.5 eq.) and 2,4,6-collidine (6.0 eq.). After activation for 10 min, the solution was added to resin-bound free amine peptide and shaken for 1.5 h at rt. Subsequently, the resin was washed with NMP (6 × 20 mL/g resin) and after Fmoc deprotection, the next amino acid was coupled analogously.

### On-resin Fmoc deprotection

The resin-bound Fmoc-peptide was treated with 20% piperidine in NMP *(v*/*v)* for 5 min and subsequently for 15 min. Afterwards, the resin was washed with NMP (6 × 20 mL/g resin).

### On-resin Fmoc deprotection of Fmoc-D-Cit-OH

The resin-bound Fmoc-peptide was treated with 20% piperidine in NMP *(v*/*v)* for 15 min and the solvent was filtered off. This procedure was repeated two times. Afterwards, the resin was washed with NMP (6 × 20 mL/g resin).

### On-resin Dde deprotection

Dde deprotection was performed adding a solution of imidazole (75 eq.), hydroxylamine hydrochloride (100 eq.) in NMP (7 mL) and DCM (3 mL) to the resin and shaking for 2 h at rt. After completed deprotection, the resin was washed with NMP (6 × 20 mL/g resin).

### Conjugation of a chelator or a SiFA moiety

The protected chelator DOTA(*^{t}*Bu)₃ or DOTAGA(*^{t}*Bu)₄ or a SiFA moiety (1.5 eq.) was dissolved in NMP and pre-activated adding TBTU (1.5 eq.), HOAt (1.5 eq.) and 2,4,6-collidine (6.0 eq.). After activation for 10 min, the solution was added to resin-bound N-terminal deprotected peptide (1.0 eq.) and shaken for 3 h at rt. Subsequently, the resin was washed with NMP (3 × 20 mL/g resin) and DCM (3 × 20 mL/g resin).

### Conjugation of SiFA-Br

In order to generate a SiFAlin moiety, the resin was swollen in DCM. A solution of DIPEA (6.0 eq.) and SiFA-Br (3.0 eq.) in DCM (2 mL) was added to the resin and shaken overnight. The resin was washed with DCM (5 × 5 mL) and dried in a desiccator.

### Conjuagation of SiFA-ipa

After pre-activation for 10 min at rt, the resin-bound peptide was treated with a solution of SiFA-ipa (3.0 eq.) and HOAt (3.0 eq.) in NMP (4 mL/g resin), as well as a solution of TBTU (3 eq.) and 2,4,6-collidine (11.0 eq.) in NMP (4 mL/g resin). The reaction mixture was shaken for 3 h at rt and filtered of afterwards. Then, the resin was washed with NMP (6 × 10 mL/g resin).

**Peptide cleavage from the resin with concomitant deprotection of acid labile protecting groups** The fully protected resin-bound peptide was washed with DCM, afterwards dissolved in a mixture of TFA/TIPS/H₂O (*v*/*v*/*v*; 95/2.5/2.5) and shaken for 60 min. The solution was filtered off and the resin was treated in the same way for another 60 min. Both filtrates were combined and stirred for additional 6 h at rt. After removing TFA under a stream of nitrogen, the crude product was obtained.

### p-Nosyl protection

The resin bound peptide was dissolved in a solution of *p*-Nosylchloride (5.0 eq.) and 2,4,6-Collidine (10.0 eq.) in NMP (10 mL/g resin) and shaken for 30 min at RT. After deprotection, the resin was washed with NMP (6 × 10 mL/g resin).

### p-Nosyl deprotection

A solution of β-Mercaptoethanol (10 eq.) and DBU (5.0 eq.) in NMP (10 mL/g resin) was added to the p-Nosyl protected resin-bound peptide and shaken for 2x 15 min at RT. Afterwards, the resin was washed with NMP (6 × 10 mL/g resin).

### N-Methylation

The resin was dissolved in a solution of PPh₃ (0.3 M in anhydrous THF, 5.0 eq.) and MeOH (10 eq.) and the resin was shaken for 1 min at RT. Thereafter, DIAD (5.0 eq.) was added and the reaction mixture was shaken over 30 min at RT. After repetition of the previous procedure, the resin was washed with NMP (6 × 10 mL/g resin).

### 3.2.2. Synthesis and characterization of the silicon-based fluoride acceptor building blocks

Synthesis of the respective silicon-based fluoride acceptor building blocks (iv, v and vii) was performed according to a previously published procedure, which was slightly modified by our group. All water- and oxygen-sensitive reactions were executed in dried reaction vessels under an argon atmosphere using a vacuum gas manifold.

### ((4-Bromobenzyl)oxy)(tert-butyl)dimethylsilane (i)

To a stirred solution of 4-bromobenzylalcohol (4.68 g, 25.0 mmol, 1.0 eq.) in anhydrous DMF (70 mL) imidazole (2.04 g, 30.0 mmol, 1.2 eq.) and TBDMSCI (4.52 g, 30.0 mmol, 1.2 eq.) were added and the resulting mixture was stirred at rt for 16 h. The mixture was then poured into ice-cold H₂O (250 mL) and extracted with Et₂O (5 × 50 mL). The combined organic fractions were washed with sat. aq. NaHCO₃ (2 × 100 mL) and brine (100 mL), dried, filtered and concentrated *in vacuo* to give the crude product which was purified by flash column chromatography (silica, 5% EtOAc/petrol) to give i as a colorless oil (7.18 g, 95%).

RP-HPLC (50→100% MeCN in H₂O with 0.1% TFA, 15 min, λ = 220 nm): *t*_{R} = 15 min. K' = 7.43.

### Di-tert-butyl[4-((tert-butyldimethylsilyloxy)methyl)phenyl]fluorosilane (ii)

At -78 °C under magnetic stirring, a solution of tBuLi in pentane (7.29 mL, 1.7 mol/L, 12.4 mmol 2.4 eq.) was added to a solution of ((4-bromobenzyl)oxy)(tert-butyl)dimethylsilane (i) (1.56 g, 5.18 mmol, 1.0 eq.) in dry THF (15 mL). After the reaction mixture had been stirred for 30 min at -78 °C, the suspension obtained was added dropwise over a period of 30 min to a cooled (-78 °C) solution of di-*tert-*butyldifluorosilane (1.12 g, 6.23 mmol, 1.2 eq.) in dry THF (10 mL). The reaction mixture was allowed to warm to rt over a period of 12 h and then hydrolyzed with saturated aqueous NaCl solution (100 mL). The organic layer was separated and the aqueous layer was extracted with diethyl ether (3 × 50 mL). The combined organic layers were dried over magnesium sulfate and filtered. The filtrate was concentrated *in vacuo* to afford ii as a yellowish oil (1.88 g, 95%). It was used for subsequent reactions without further purification.

RP-HPLC (50→100% MeCN in H₂O with 0.1% TFA, 15 min, λ = 220 nm): *t*_{R} = 19 min. K' = 9.67.

### 4-(Di-tert-butylfluorosilanyl)benzyl alcohol (iii)

A catalytic amount of concentrated aqueous HCl (0.5 mL) was added to a suspension of ii (1.88 g, 4.92 mmol, 1.0 eq.) in methanol (50 mL). The reaction mixture was stirred for 18 h at rt and then the solvent and the volatiles were removed under reduced pressure. The residue was redissolved in diethyl ether (40 mL) and the solution was washed with saturated aqueous NaHCO₃ solution. The aqueous layer was extracted with diethyl ether (3 × 50 mL). The combined organic layers were dried over magnesium sulfate and filtered. The filtrate was concentrated *in vacuo* to afford iii as a yellowish oil (1.29 g, 98%) that solidified. The product was used without further purification.

RP-HPLC (50→100% MeCN in H₂O with 0.1% TFA, 15 min, λ = 220 nm): *t*_{R} = 8.2 min. K' = 3.61.

### 4-(Di-tert-butylfluorosilyl)benzoic acid ((4-SiFA)Bz-OH, iv)

At rt, 5.8 g KMnO₄ (36.7 mmol, 1.5 eq.) were dissolved in H₂O and added to a solution of iii (6.61 g, 24.6 mmol, 1.0 eq.), tert-butanol (65 mL), dichloromethane (9 mL), and 1.25 M NaH₂PO₄·H₂O buffer (36 mL) at pH 4.0-4.5. After the mixture had been stirred for 25 min, it was cooled on ice for 10 min, whereupon excess KMnO₄ (7.8 g, 49.2 mmol, 2.0 eq.) was added. The reaction mixture was stirred for 2 h on ice and subsequently allowed to warm to rt for 30 min. The reaction was then quenched by the addition of saturated aqueous Na₂SO₃ solution (50 mL). Upon addition of 2 M aqueous HCl, all of the MnO₂ dissolved. The resulting solution was extracted with diethyl ether (3 × 100 mL). The combined organic layers were washed with saturated aqueous NaHCO₃ solution, dried over MgSO₄, filtered, and concentrated under reduced pressure to provide a white solid, which was purified by recrystallization from Et₂O/*n*-hexane (1:3, for 12 h) to give iv (2.57 g, 37%).

¹H NMR (300 MHz, CDCl₃): d [ppm] = 8.10 (d, 2H ³*J*(¹H,¹H) = 8.1 Hz; H*ₘ*), 7.74 (d, 2H, ³*J*(¹H,¹H) = 8.1 Hz; Hₒ), 1.07 (s, 18H; CCH₃).

¹³C{¹H} NMR (101 MHz, DMSO-D₆): d [ppm] = 167.2 (s; COOH), 138.3 (d, ²*J*(¹³C,¹⁹F) = 14 Hz; C*ₚ*), 133.8 (d, ³*J*(¹³C,¹⁹F) = 4 Hz; C*ₘ*), 132.1 (s; C*ᵢ*), 128.3 (s; C*ₒ*), 26.9 (s; C*C*H₃), 19.7 (d, ²*J*(¹³C,¹⁹F) = 12 Hz; CCH₃).

¹⁹F{²⁹Si} NMR (376 MHz, DMSO-D₆): d [ppm] = -187.2.

²⁹Si{¹H}INEPT NMR (79 MHz, DMSO-D₆): d [ppm] = 14.1 (d, ¹*J*(¹⁹F,²⁹Si) = 299 Hz).

RP-HPLC (50→100% MeCN in H₂O with 0.1% TFA, 15 min, λ = 220 nm): *t_{R}* = 8.5 min. K' = 3.78. ESI-MS (positive): calculated monoisotopic mass (C₁₅H₂₃FO₂Si): 282.15; found: m/z = 283.2 [M+H]⁺, 265.2 [M-H₂O+H]⁺.

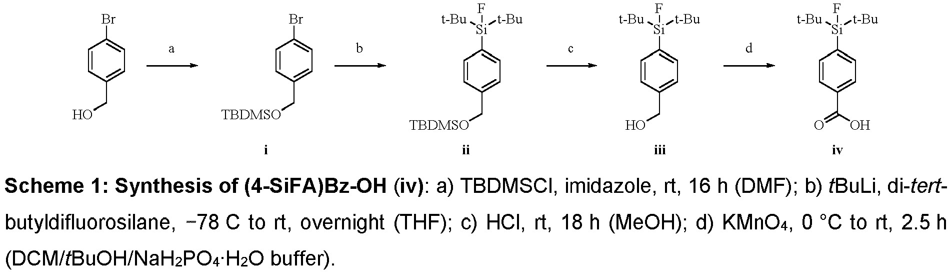

### 4-(Di-tert-butylfluorosilanyl)benzyl bromide (4-SiFA)Bz-Br (v)

4-(Di-tert-butylfluorosilanyl)benzyl bromide was synthesized analogously to (4-SiFA)Bz-OH (iv). Only for the last step 4-(di-tert-butylfluorosilanyl)benzyl alcohol (iii) (2.41 g, 8.96 mmol, 1.0 eq.) was dissolved in anhydrous DCM (40 mL) and CBr₄ (2.59 g, 9.86 mmol, 1.1 eq.) was added under a argon counterflow. Afterwards, the reaction mixture was cooled to 0 °C and PPh₃ (3.27 g, 9.86 mmol, 1.1 eq., dissolved in 30 mL anhydrous DCM) was added dropwise over a period of 30 min. Then, the reaction mixture was stirred for 2 h at rt and the solvents were removed *in vacuo.* The crude product was purified by flash column chromatography (silica, 100% pentane).

RP-HPLC (50→100% MeCN in H₂O with 0.1% TFA, 15 min, λ = 220 nm): *t_{R}* = 17.3 min.

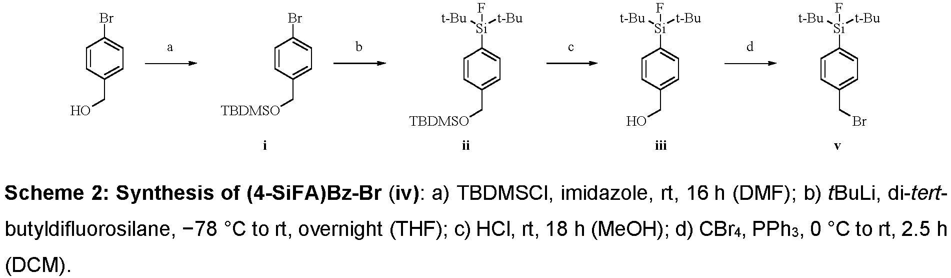

### Di-tert-butyl(3,5-dimethylphenyl)fluorosilane (vi)

A solution of 4.54 g 1-bromo-3,5-dimethylbenzene (25.1 mmol, 1.0 eq.) in 73.1 mL dry THF was cooled to -78 °C and 34.7 mL of tBuLi (55.5 mmol, 1.6 M in pentane, 2.2 eq.) were added dropwise and stirred for 30 min at -78 °C. The reaction mixture was then added to a solution of 5.0 g of di-*tert-*butyldifluorosilane (27.7 mmol, 1.1 eq.) in 49.1 mL THF at -78 °C and stirred overnight while allowing to warm to rt under pressure control. The reaction was stopped by addition of 100 mL brine. The aqueous layer was extracted with Et₂O (3 × 100 mL), combined organic phases were dried over MgSO₄ and the solvent was removed under reduced pressure to obtain 6.6 g di-tert-butyl(3,5-dimethylphenyl)fluorosilane (vi, 24.8 mmol, 99 %) as a colorless solid.

¹H NMR (500 MHz, CDCl₃): d [ppm] = 7.19 (s, 2H; H*ₒ*), 7.04 (s, 1H; H*ₚ*), 2.33 (s, 6 H; CH₃), 1.06 (s, 18 H; CCH₃). RP-HPLC (50 to 100% B in 15 min, 100% B for 10 min): *t_{R}=* 16.4 min. K' = 8.21.

### 5-(Di-tert-butylfluorosilyl)isophthalic acid ((5-SiFA)Ip-OH, vii)

To a solution of 1.1 g di-tert-butyl(3,5-dimethylphenyl)fluorosilane (i) (4.0 mmol, 1.0 eq.) in 16.8 mL tBuOH/DCM (v/v= 3.5/1) were added 16.0 mL of a NaH₂PO₄·H₂O solution (40.0 mmol, 2.5 M in H₂O, 10.0 eq.). To the solution 7.6 g KMnO₄ (48.0 mmol, 12.0 eq.) were added at rt, the reaction was carefully heated stepwise to 75 °C and stirred for 24 h. The reaction was quenched by the addition of a saturated aqueous NaSO₃ solution (50 mL). Concentrated aqueous HCl (10 mL) was added to completely dissolve MnO₂. The solution was extracted with Et₂O (3 × 100 mL), the combined organic phases were dried over MgSO₄ and the solvent removed under reduced pressure to obtain ii (1.3 g, 4.0 mmol, 100%) as a colorless solid.

¹H NMR (400 MHz, DMSO-D₆): *d* ppm = 8.53 (t, 1 H, ⁴*J*(¹H,¹H) = 1.7 Hz; H_{Ar-2}), 8.32 (d, 2 H, ⁴*J*(¹H,¹H) = 1.6 Hz; H_{Ar-4},₋₆), 1.03 (s, 18 H; CH₃).

¹³C{¹H} NMR (101 MHz, DMSO-D₆): d [ppm] = 166.5 (s; COOH), 137.9 (d, ³*J*(¹³C,¹⁹F) = 4 Hz; C_{Ar-4},₋₆), 134.0 (d, ²*J*(¹³C, ¹⁹F) = 14 Hz; C_{Ar-5}), 131.4 (s; C_{Ar-2}), 130.8 (s; C_{Ar-1},₋₃), 26.8 (s; CCH₃), 19.7 (d, ²*J*(¹³C,¹⁹F) = 12 Hz; *C*CH₃).

¹⁹F{²⁹Si) NMR (376 MHz, DMSO-D₆): d [ppm] = -187.1.

²⁹Si{¹H}INEPT NMR (79 MHz, DMSO-D₆): d [ppm] = 13.8 (d, ¹J(¹⁹F,²⁹Si) = 299 Hz). RP-HPLC (50 to 100% B in 15 min): *t_{R}* = 5.7 min. K' = 2.20. ESI-MS (positive): calculated monoisotopic mass (C₁₆H₂₃FO₄Si): 326.13; found: m/z = 327.2 [M+H]⁺, 309.2 [M-H₂O+H]⁺.

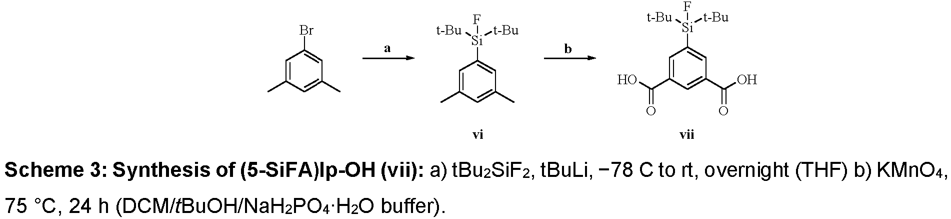

### 3.3. Labeling experiments

*^{nat}Lu-Labeling:* The purified chelator-containing ligand (10⁻³ M in Tracepur^{®} H₂O, 1.0 eq.) and [^{nat}Lu]LuCl₃ (20 mM in Tracepur^{®} H₂O, 2.5 eq.) were diluted with Tracepur^{®} H₂O to a final concentration of 10⁻⁴ M and heated to 95 °C for 30 min. After cooling to rt, the crude product was obtained.

*^{nat}Ga-Labeling:* ^{nat}Ga-complexation was accomplished stirring a solution of Ga(NO₃)₃ (20 µL, 10 mM, 2.0 eq.), peptide precursor (80 µL, 1 mM) and Tracepur^{®} H₂O (710 µL) at 70 °C for 30 min.

*^{nat}Cu-Labeling:* The CCK-2R ligands were complexated with [^{nat}Cu]copper using a solution of Cu(OAc)₂ (24 µL, 10 mM, 3.0 eq.), peptide precursor (80 µL, 1 mM) and Tracepur^{®} H₂O (694 µL) at 80 °C for 30 min.

*[¹⁹F]AlF-Labeling:* AlCl₃ (3.70 mg, 2.80 µmol, 4 eq.) and NaF (1.20 mg, 2.80 µmol, 4 eq.) were preactivated for 10 min at rt in NaOAc-buffer (pH = 4.2, 80/20 HoAc/NaOAc, *v*/*v,* 0.5 M). Afterwards, the solution was added to the labeling precursor (700 µL, 700 nmol, 10⁻³ M in DMSO, 1 eq.) and DMSO was added to the reaction solution (1/1, *v*/*v,* DMSO/NaOAc buffer). The reaction mixture was heated to 110 °C for 30 min and [¹⁹F]AlF-NOTA-labeled peptide was purified via RP-HPLC.

*¹⁷⁷Lu-Labeling:* ¹⁷⁷Lu-labeling was done using a procedure developed within the group. Therefore, a solution of the purified chelator-containing ligand (10⁻³ M in Tracepur^{®} H₂O, 1 µL), NaOAc buffer (1 M, pH = 5.50, 10 µL) and approximately 10-30 MBq [¹⁷⁷Lu]LuCl₃ (0.04 M in HCl, *Isotope Technologies Munich SE,* Garching, Germany) were diluted with HCl (0.04 M) to a total volume of 90 µL and heated to 95 °C for 10 min. Immediately after labeling, sodium ascorbate (0.1 M, 10 µL) was added to prevent radiolysis. Incorporation of ¹⁷⁷Lu was determined by radio TLC (ITLC-SG chromatography paper, mobile phase: 0.1 M trisodium citrate). Radiochemical purity of the labeled compound was determined by radio RP-HPLC.

*⁶⁷Ga-Labeling:* To a solution of labeling precursor (1 µL, 1 nmol, 1 mM in DMSO) and 4-(2-hydroxyethyl)-1-piperazineethanesulfonic acid-buffer (7 µL, 2.5 M, HEPES), [⁶⁷Ga]GaCl₃ (10-30 MBq, *Curium Germany GmbH,* Berlin, Germany) was added and the reaction mixture was heated to 90 °C for 15 min. Radiochemical purity was determined using radio RP-HPLC and radio TLC.

*⁶⁴Cu-Labeling:* A solution of labeling precursor (1 µL, 1 nmol, 1 mM in DMSO), NaOAc buffer (10 µL, 1 M, pH = 5.5) and [⁶⁴Cu]CuCl₂ (5-20 MBq, 925 MBq/mL, *DSD Pharma,* Purkersdorf, Austria) were heated to 80 °C for 10 min. After the reaction solution cooled down, sodium ascorbate (10 µL,1 M) was added. Subsequently, the radiochemical purity was determined using radio RP-HPLC and radio TLC.

*¹⁸F-Labeling:* On the day of the experiment, 100 to 3000 MBq of [¹⁸F]fluoride in H₂O (2.5 mL) were loaded on a SEP-Pak^{®} Light (46 mg) Accell^{™} Plus QMA cartridge preconditioned with H₂O (10 mL). Afterwards, [¹⁸F]fluoride was dried with anhydrous DMSO (8 mL) and inversely eluted with ammonium formate (100-300 µL, 0.3 µM). Thereafter, a solution of eluate (30 to 100 µL) and peptide precursor (5 to 15 nmol) were heated (40 to 60 °C) for 5 min, the reaction mixture was diluted in PBS (10 mL, pH = 3) and the ¹⁸F-labeled peptide was loaded onto an Oasis^{®} HLB (30 mg) Light Cartridge. After washing the cartridge with PBS (10 mL, pH = 7.4), the peptide was inversely eluted with 300 µL of H₂O/EtOH (1:7). Radiochemical purity was determined using radio RP-HPLC and radio TLC.

### 3.4. In vitro experiments

### n-Octanol-PBS distribution coefficient (logD_{7.4})

Approximately 1 MBq of the labeled tracer was dissolved in 1 mL of a 1/1 mixture (*v*/*v*) of phosphate buffered saline (PBS, pH = 7.4) and *n*-octanol in an Eppendorf tube (1.5 mL). After vigorous mixing of the suspension for 3 min at rt, the vial was centrifuged at 9,000 rpm for 5 min (Biofuge 15, *Heraeus Sepatech,* Osterode, Germany) and 200 µL aliquots of both layers were measured in a *γ*-counter. The experiment was repeated at least five times.

### Determination of IC₅₀

AR4-2J cells were harvested 24 ± 2 h before the experiment and seeded in 24-well plates (2.0 × 10⁵ cells in 1 mL/well). After removal of the nutrient medium (RPMI 1640, 5 mM L-Gln, 5 mL non-essential amino acids (100×), 10% FCS), the cells were washed once with 500 µL PBS. Thereafter, 200 µL nutrient medium (5% BSA, *v*/*v*) were added. Next, 25 µL per well of solutions containing either nutrient medium (5% BSA, *v*/*v*) as control or the respective compound in increasing concentration (10⁻¹⁰ - 10⁻⁴ M), were added with subsequent addition of 25 µL [¹⁷⁷Lu]Lu-DOTA-PP-F11N (0.3 pmol/well).

All experiments were performed in triplicate for each concentration. After 3 h incubation at 37 °C, the experiment was terminated by removal of the medium and consecutive rinsing with 300 µL PBS. The media of both steps were combined in one fraction and represent the amount of free radiolabeled reference. Afterwards, the cells were lysed with 300 µL of 1 M NaOH for at least 15 min and united with the 300 µL NaOH of the following washing step. Quantification of bound and free radiolabeled reference was accomplished in a *γ*-counter. *IC*₅₀ determination for each conjugate was repeated twice.

### Determination of Internalization kinetics

For the determination of the internalization kinetics of the various peptides, AR4-2J cells (3.0×10⁵) were seeded into poly-lysine coated 24-well plates adding 1 mL of nutrient medium (RPMI 1640, 5 mM L-Gln, 5 mL non-essential amino acids (100×), 10% FCS). Afterwards, the cells were incubated for 24 ± 2 h at 37 °C in a humidified atmosphere (5% CO₂).

On the day of the experiment, the medium was removed and each well was washed with incubation medium (RPMI 1640, 5 mM L-Gln, 5 mL non-essential amino acids (100×)) (300 µL). Afterwards, 200 µL of nutrient medium (RPMI 1640, 5 mM L-Gln, 5 mL non-essential amino acids (100×), 10% FCS) was added to each well, followed by addition of either 25 µL of nutrient medium (RPMI 1640, 5 mM L-Gln, 5 mL non-essential amino acids (100×) (*n* = 3) or 25 µL DOTA-PP-F11N (10 µmol) for blockade (*n* = 3). Afterwards, 25 µL of the respective ¹⁷⁷Lu-labeled peptide (0.3 pmol/well) are added to each well (*n* = 6). Thereafter, the assay was incubated for various time points (1, 2, 4 and 6 h) at 37 °C in a humidified atmosphere (5% CO₂). After incubation, the cells were put on ice and the supernatant was collected. Then, the cells were washed with an ice-cold incubation medium (RPMI 1640, 5 mM L-Gln, 5 mL non-essential amino acids (100×)) (300 µL) and both fractions were unified. In order to displace the peptides from the cell membrane, 300 µL of an ice-cold glycine buffer (1 M, pH = 2.2) were added and the cells were incubated for 15 min on ice. Afterwards, the supernatant was collected and the cells were washed with an ice-cold glycine buffer (300 µL, 1 M, pH = 2.2). Both fractions were unified. After lysis of the cells with NaOH (300 µL, 1 M) for 15 min, the respective wells were washed with NaOH (300 µL, 1 M) and both fractions were unified. The radioactivity of the supernatant, the acid wash and the lysed fractions were quantified using a *γ*-counter.

### Stability studies in Human Serum

The ¹⁷⁷Lu-labeled CCK-2R ligands (1 nmol, approx. 5 MBq) were incubated for 72 ± 2 h in human serum (200 µL) at 37 °C. After incubation ice-cold EtOH (125 µL) and MeCN (375 µL) were added and the suspension was centrifuged for 2 min at 5,000 rpm. Then, the supernatant was transferred into ultracentrifugation vials and centrifuged for another 2 min at 5,000 rpm. After separating the precipitate from the solution, the stability of the ligands was determined via RP-HPLC chromatography.

### Determination of HSA binding

Human serum albumin (HSA) binding of the CCK-2R-targeted compounds was determined as reported by *Valko et al.* [31], using a Chiralpak HSA column (50 x 3 mm, 5 µm, H13H-2433, Daicel, Tokyo, Japan) at a constant flow rate of 0.5 mL/min at rt. Therefore, an aq. solution of NH₄OAc (pH = 6.9, 50 mM) as mobile phase A and isopropanol as mobile phase B were freshly prepared. In order to calibrate the column prior to the experiments, the retention times of nine reference substances with a HSA binding in the range of 13 to 99% were determined using a gradient of 100% A (0 to 3 min), followed by 80% A (3 to 40 min) [32]. Furthermore, all substances tested, were dissolved in a mixture (1/1, v/v) of A and B reaching a final concentration of 0.5 mg/mL. The HSA binding of all rhCCK ligands tested, was determined using the same gradient as for the calibration probes. The non-linear regression of the calibration experiments and the CCK-2R ligands **(****Fig. 29****)** was established using the OriginPro 2016G software (Northampton, United States).

### In vivo experiments

All animal experiments were conducted in accordance with general animal welfare regulations in Germany (German animal protection act, in the edition of the announcement, dated May 18^{th}, 2006, as amended by Article 280 of June 19^{th} 2020, approval no. ROB-55.2-1-2532.Vet_02-18-109 by the General Administration of Upper Bavaria) and the institutional guidelines for the care and use of animals. CB17-SCID mice of both genders (*Charles River Laboratories International Inc.,* Sulzfeld, Germany) were let to acclimate at the in-house animal facility at least for one week before inoculation was performed. To establish tumor xenografts, AR4-2J cells (5.0 × 10⁶ cells per 200 µL) were suspended in a 1/1 mixture (*v*/*v*) of Dulbecco modified Eagle medium / Nutrition Mixture F-12 with Glutamax-I (1/1) and Cultrex^{®} Basement Membrane Matrix Type 3 (*Trevigen,* Gaithersburg, MD, USA) and inoculated subcutaneously onto the right shoulder of 6-10 weeks old CB17-SCID mice (*Charles River,* Sulzfeld, Germany). Mice were used for experiments when tumors had grown to a volume of 150-300 mm³ (1-2 weeks after inoculation). Exclusion criteria for animals from an experiment were either a weight loss higher than 20%, a tumor size above 1,500 mm³, an ulceration of the tumor, a respiratory distress or a change of behavior. None of these criteria applied to any animal from the experiment. Neither randomization nor blinding was applied in the allocation of the experiments. Health status is SPF according to FELASA.

### Biodistribution

Approximately 1-5 MBq (100 pmol) of the radiolabeled minigastrin analogues were injected into the tail vein of AR4-2J tumor-bearing female CB17-SCID mice and sacrificed at 1 to 24 h post injection (n = 4-5). Selected organs were removed, weighted and measured in a *γ*-counter.

### Competition Studies

For competition studies, the radiolabeled compound (approx. 1-5 MBq, 100 pmol) was co-injected with [^{nat}Lu]Lu-DOTA-MGS5 (40 nmol) into the tail vein of AR4-2J tumor-bearing CB17-SCID mice and sacrificed at 1 to 24 h post injection (*n* = 2). Selected organs were removed, weighted and measured in a *γ*-counter.

### µSPECT/CT imaging

Imaging studies were performed at a MILabs VECTor⁴ small-animal SPECT/PET/OI/CT (MILabs, Utrecht, the Netherlands). Data were reconstructed using the MILabs-Rec software (version 10.02) and a pixel-based Similarity-Regulated Ordered Subsets Expectation Maximization (SROSEM) algorithm with a window-based scatter correction (20% below and 20% above the photopeak, respectively). Further data analysis was accomplished using the PMOD4.0 software (PMOD TECHNOLOGIES LLC, Zurich, Switzerland) at defined settings (voxel size CT: 80 µm, voxel size SPECT: 0.8 mm, 1.6 mm (FWHM) Gaussian blurring post processing filter, with calibration factor in kBq/mL and decay correction and no attenuation correction).

For SPECT studies mice were anesthetized with isoflurane and injected with 2-4 MBq (100 pmol) of the radiolabeled tracer into the tail vein. Static images were recorded 1, 4 and 24 h p.i. with an acquisition time of 45-60 min using the HE-GP-RM collimator and a step-wise multi-planar bed movement.

### Stability Studies

Stability studies were performed via injection of the ¹⁷⁷Lu-labeled peptide (approx. 35 MBq, 1 nmol) into the tail vein (*n* = 3) of anesthetized (2% isoflurane) CB17-SCID mice. At 30 min p.i. the mice were euthanized and a urine as well as a blood sample were taken. The urine sample was analyzed without further treatment. The blood sample was centrifuged at 5,000 rpm for 2 min. Afterwards, the serum was collected and ice-cold MeCN (1:1, *v*/*v)* as well as EtOH (1:2, *v*/*v)* were added. The sample was centrifuged at 5,000 rpm for 2 min once more and the supernatant was analyzed via RP-HPLC (10→30% in 5 min, 30→60% in 15 min, MeCN/H₂O + 0.1% TFA).

### Patient Studies

All patients gave written informed consent before undergoing [⁶⁸Ga]Ga-DOTA-CCK-66 PET/CT. The retrospective analysis of the imaging results was approved by the responsible ethics committee of the University of Munich. The anonymized analyses were carried out in accordance with the Declaration of Helsinki and its later amendments and the legal considerations of clinical guidelines.

The patients underwent [⁶⁸Ga]Ga-DOTA-CCK-66 whole body PET/CT using a Biograph mCT 40 (Siemens Healthineers, Erlangen, Germany) at 120 min after injection of 150-200 MBq of [⁶⁸Ga]Ga-DOTA-CCK-66. Whole body CT imaging was performed as auxiliary CT (120 kVp, 40 mAs). PET data sets were reconstructed using standard protocols and corrected for randoms, scatter, decay and attenuation (using whole body auxiliary CT).

### References

1. Wells, S.A., Jr.; Asa, S.L.; Dralle, H.; Elisei, R.; Evans, D.B.; Gagel, R.F.; Lee, N.; Machens, A.; Moley, J.F.; Pacini, F.; et al. Revised American Thyroid Association guidelines for the management of medullary thyroid carcinoma. Thyroid 2015, 25, 567-610, doi:10.1089/thy.2014.0335.
2. Society, A.C. Cancer Facts & Figures 2020. **2020,** *2020.*
3. Kebebew, E.; Kikuchi, S.; Duh, Q.-Y.; Clark, O.H. Long-term Results of Reoperation and Localizing Studies in Patients With Persistent or Recurrent Medullary Thyroid Cancer. Arch Surg. 2000, 135, 895-901.
4. Kebebew, E.; Greenspan, F.S.; Clark, O.H.; Woeber, K.A.; Grunwell, J. Extent of disease and practice patterns for medullary thyroid cancer. J Am Coll Surg 2005, 200, 890-896, doi:10.1016/j.jamcollsurg.2004.12.011.
5. Engelbach, M.; Görges, R.; Forst, T.; Pfützner, A.; Dawood, R.; Heerdt, S.; Kunt, T.; Bockisch, A.; Beyer, J. Improved diagnostic methods in the follow-up of medullary thyroid carcinoma by highly specific calcitonin measurements. J Clin Endocrinol Metab 2000, 85, 1890-1894.
6. Roman, S.; Lin, R.; Sosa, J.A. Prognosis of medullary thyroid carcinoma: demographic, clinical, and pathologic predictors of survival in 1252 cases. Cancer 2006, 107, 2134-2142, doi:10.1002/cncr.22244.
7. Schlumberger, M.; Carlomagno, F.; Baudin, E.; Bidart, J.M.; Santoro, M. New therapeutic approaches to treat medullary thyroid carcinoma. Nat Clin Pract Endocrinol Metab 2008, 4, 22-32, doi:10.1038/ncpendmet0717.
8. Raue, F. German medullary thyroid carcinoma/multiple endocrine neoplasia registry. German MTC/MEN Study Group. Medullary Thyroid Carcinoma/Multiple Endocrine Neoplasia Type 2. Langenbecks Arch Surg 1998, 383, 334-336.
9. Hennessy, J.F.; Wells, S.A.J.; Ontjes, D.A.; Cooper, C.W. A comparison of pentagastrin injection and calcium infusion as provocative agents for the detection of medullary carcinoma of the thyroid. J Clin Endocrinol Metab 1974, 39, 487-495.
10. Wells, S.A.J.; Chi, D.D.; Toshima, K.; Dehner, L.P.; Coffin, C.M.; Dowton, S.B.; Ivanovich, J.L.; DeBenedetti, M.K.; Dilley, W.G.; Moley, J.F. Predictive DNA testing and prophylactic thyroidectomy in patients at risk for multiple endocrine neoplasia type 2A. Ann Surg 1994, 220, 237-247.
11. Wells, S.A.J.; Baylin, S.B.; Linehan, W.M.; Farrell, R.E.; Cox, E.B.; Cooper, C.W. Provocative agents and the diagnosis of medullary carcinoma of the thyroid gland. Ann Surg 1978, 188, 139-141.
12. Béhé, M.; Behr, T.M. Cholecystokinin-B (CCK-B)/gastrin receptor targeting peptides for staging and therapy of medullary thyroid cancer and other CCK-B receptor expressing malignancies. Biopolymers 2002, 66, 399-418.
13. Israel, O.; Pellet, O.; Biassoni, L.; De Palma, D.; Estrada-Lobato, E.; Gnanasegaran, G.; Kuwert, T.; la Fougere, C.; Mariani, G.; Massalha, S.; et al. Two decades of SPECT/CT - the coming of age of a technology: An updated review of literature evidence. Eur J Nucl Med Mol Imaging 2019, 46, 1990-2012, doi:10.1007/s00259-019-04404-6.
14. Fani, M.; Maecke, H.R.; Okarvi, S.M. Radiolabeled peptides: valuable tools for the detection and treatment of cancer. Theranostics 2012, 2, 481-501, doi:10.7150/thno.4024.
15. Giovanella, L.; Treglia, G.; lakovou, I.; Mihailovic, J.; Verburg, F.A.; Luster, M. EANM practice guideline for PET/CT imaging in medullary thyroid carcinoma. Eur J Nucl Med Mol Imaging 2020, 47, 61-77, doi:10.1007/s00259-019-04458-6.
16. Treglia, G.; Castaldi, P.; Villani, M.F.; Perotti, G.; de Waure, C.; Filice, A.; Ambrosini, V.; Cremonini, N.; Santimaria, M.; Versari, A.; et al. Comparison of 18F-DOPA, 18F-FDG and 68Ga-somatostatin analogue PET/CT in patients with recurrent medullary thyroid carcinoma. Eur J Nucl Med Mol Imaging 2012, 39, 569-580, doi:10.1007/s00259-011-2031-6.
17. Brammen, L.; Niederle, M.B.; Riss, P.; Scheuba, C.; Selberherr, A.; Karanikas, G.; Bodner, G.; Koperek, O.; Niederle, B. Medullary Thyroid Carcinoma: Do Ultrasonography and F-DOPA-PET-CT Influence the Initial Surgical Strategy? Ann Surg Oncol 2018, 25, 3919-3927, doi:10.1245/s10434-018-6829-3.
18. Treglia, G.; Cocciolillo, F.; Di Nardo, F.; Poscia, A.; de Waure, C.; Giordano, A.; Rufini, V. Detection rate of recurrent medullary thyroid carcinoma using fluorine-18 dihydroxyphenylalanine positron emission tomography: a meta-analysis. Acad Radiol 2012, 19, 1290-1299, doi:10.1016/j.acra.2012.05.008.
19. Stamatakos, M.; Paraskeva, P.; Stefanaki, C.; Katsaronis, P.; Lazaris, A.; Safioleas, K.; Kontzoglou, K. Medullary thyroid carcinoma: The third most common thyroid cancer reviewed. Oncol Lett 2011, 2, 49-53, doi:10.3892/ol.2010.223.
20. Hadoux, J.; Schlumberger, M. Chemotherapy and tyrosine-kinase inhibitors for medullary thyroid cancer. Best Practice & Research Clinical Endocrinology & Metabolism 2017, 31, 335-347, doi:10.1016/j.beem.2017.04.009.
21. Hazard, J.B. The C cells (parafollicular cells) of the thyroid gland and medullary thyroid carcinoma. A review. Am J Pathol 1977, 88, 213-250.
22. Ljungberg, O. Medullary carcinoma of the human thyroid gland. Autoradiographic localization of radioiodine. Acta Pathol Microbiol Scand 1966, 68, 476-480.
23. Fussey, J.M.; Vaidya, B.; Kim, D.; Clark, J.; Ellard, S.; Smith, J.A. The role of molecular genetics in the clinical management of sporadic medullary thyroid carcinoma: A systematic review. Clin Endocrinol (Oxf) 2019, doi:10.1111/cen.14060.
24. Resteghini, C.; Cavalieri, S.; Galbiati, D.; Granata, R.; Alfieri, S.; Bergamini, C.; Bossi, P.; Licitra, L.; Locati, L.D. Management of tyrosine kinase inhibitors (TKI) side effects in differentiated and medullary thyroid cancer patients. Best Pract Res Clin Endocrinol Metab 2017, 31, 349-361, doi:10.1016/j.beem.2017.04.012.
25. Behr, T.M.; Gratz, S.; Markus, P.M.; Dunn, R.M.; Hüfner, M.; Becker, H.; Becker, W. Enhanced bilateral somatostatin receptor expression in mediastinal lymph nodes ("chimney sign") in occult metastatic medullary thyroid cancer: a typical site of tumour manifestation? Eur J Nucl Med 1997, 24, 184-191.
26. Reubi, J.C.; Schaer, J.C.; Waser, B. Cholecystokinin(CCK)-A and CCK-B/gastrin receptors in human tumors. Cancer Res. 1997, 57, 1377-1386.
27. Gotthardt, M.; Behe, M.P.; Beuter, D.; Battmann, A.; Bauhofer, A.; Schurrat, T.; Schipper, M.; Pollum, H.; Oyen, W.J.; Behr, T.M. Improved tumour detection by gastrin receptor scintigraphy in patients with metastasised medullary thyroid carcinoma. Eur J Nucl Med Mol Imaging 2006, 33, 1273-1279, doi:10.1007/s00259-006-0157-8.
28. Roosenburg, S.; Laverman, P.; van Delft, F.L.; Boerman, O.C. Radiolabeled CCK/gastrin peptides for imaging and therapy of CCK2 receptor-expressing tumors. Amino Acids 2011, 41, 1049-1058, doi:10.1007/s00726-010-0501-y.
29. Fani, M.; Peitl, P.K.; Velikyan, I. Current Status of Radiopharmaceuticals for the Theranostics of Neuroendocrine Neoplasms. Pharmaceuticals (Basel) 2017, 10, doi:10.3390/ph10010030.
30. Klingler, M.; Summer, D.; Rangger, C.; Haubner, R.; Foster, J.; Sosabowski, J.; Decristoforo, C.; Virgolini, I.; von Guggenberg, E. DOTA-MGS5, a New Cholecystokinin-2 Receptor-Targeting Peptide Analog with an Optimized Targeting Profile for Theranostic Use. J Nucl Med 2019, 60, 1010-1016, doi:10.2967/jnumed.118.221283.
31. Valko, K.; Nunhuck, S.; Bevan, C.; Abraham, M.H.; Reynolds, D.P. Fast gradient HPLC method to determine compounds binding to human serum albumin. Relationships with octanol/water and immobilized artificial membrane lipophilicity. J Pharm Sci 2003, 92, 2236-2248, doi:10.1002/jps.10494.
32. Yamazaki, K.; Kanaoka, M. Computational prediction of the plasma protein-binding percent of diverse pharmaceutical compounds. J Pharm Sci 2004, 93, 1480-1494, doi:10.1002/jps.20059.
33. Behr, T.M.; Jenner, N.; Radetzky, S.; Béhe, M.; Gratz, S.; Yücekent, S.; Raue, F.; Becker, W. Targeting of cholecystokinin-B/gastrin receptors in vivo: preclinical and initial clinical evaluation of the diagnostic and therapeutic potential of radiolabelled gastrin. Eur J Nucl Med 1998, 25, 424-430, doi:10.1007/s002590050241.
34. Klingler, M.; Rangger, C.; Summer, D.; Kaeopookum, P.; Decristoforo, C.; von Guggenberg, E. Cholecystokinin-2 Receptor Targeting with Novel C-terminally Stabilized HYNIC-Minigastrin Analogs Radiolabeled with Technetium-99m. Pharmaceuticals (Basel) 2019, 12, doi:10.3390/ph12010013.
35. Rottenburger, C.; Nicolas, G.P.; McDougall, L.; Kaul, F.; Cachovan, M.; Vija, A.H.; Schibli, R.; Geistlich, S.; Schumann, A.; Rau, T.; et al. Cholecystokinin 2 Receptor Agonist (177)Lu-PP-F11N for Radionuclide Therapy of Medullary Thyroid Carcinoma: Results of the Lumed Phase 0a Study. J Nucl Med 2020, 61, 520-526, doi:10.2967/jnumed.119.233031.
36. Kolenc-Peitl, P.; Tamma, M.; Kroselj, M.; Braun, F.; Waser, B.; Reubi, J.C.; Sollner Dolenc, M.; Maecke, H.R.; Mansi, R. Stereochemistry of amino acid spacers determines the pharmacokinetics of (111)In-DOTA-minigastrin analogues for targeting the CCK2/gastrin receptor. Bioconjug Chem 2015, 26, 1113-1119, doi:10.1021/acs.bioconjchem.5b00187.

The invention furthermore comprises the following items:
1. A tetrapeptide binding to Cholecystokinin 2 receptor (CCK-2R), wherein the tetrapeptide is represented by formula (I)

   Xaa1-Xaa2-Xaa3-Xaa4 (formula (I))

   or salt thereof, wherein
   Xaa1 is Trp, (β-(3-benzothienyl)-alanine, Trp, wherein one or more of the H atoms of the 1*H*-indol-3-yl are replaced by a substituent that is for each H atom independently selected from a C₁ to C₃ alkyl, -OH, -SH, -F, and -Cl, Phe, 1-Nal, 2-Nal, Tyr or p-Amino-Phe,
   Xaa2 is an N-methyl amino acid, wherein the N-methyl group is at the alpha carbon, and preferably an N-methyl amino acid containing an aliphatic side chain, wherein the N-methyl group is at the alpha carbon,
   Xaa3 is an amino acid with an acidic side chain at neutral pH, and
   Xaa4 is an amino acid with an aromatic side chain or an amide thereof.
2. The tetrapeptide of item 1, wherein Xaa2 is (N-Me)Nle, (N-Me)Met, (N-Me)lle, (N-Me)Leu, (N-Me)Val, (N-Me)Gly, (N-Me)Ala, (N-Me)Glu, is preferably (N-Me)Nle or (N-Me)Met and is most preferably (N-Me)Nle.
3. The tetrapeptide of item 1 or 2, wherein Xaa3 is Asp, isoaspartate, Glu or isoglutamine and is preferably Asp.
4. The tetrapeptide of any one of items 1 to 3, wherein Xaa4 is 1-Nal, Phe, Trp, Tyr, 2-Nal, p-Amino-Phe or an amide of any one of the foregoing, is preferably 1-Nal, Phe, Tyr or an amide of any one of the foregoing, and is most preferably 1-Nal or Tyr or an amide of 1-Nal or Tyr.
5. A conjugate comprising within a single molecule:
   (a) the tetrapeptide of any one of claims 1 to 4, and
   (b) one or more chelating groups capable of complexing a nonradioactive or radioactive cation or one or more chelating groups containing a chelated nonradioactive or radioactive cation.
6. The conjugate of item 5, wherein the chelating group is DOTA or DOTAGA with or without a chelated nonradioactive or radioactive cation, wherein DOTA and DOTAGA are preferably bound with one of its carboxylic groups via an amide bond to the remainder of the conjugate.
7. The conjugate of items 5 or 6 further comprising within the single molecule:
   (c) a silicon-based fluoride acceptor moiety R^{SiFA} selected from:
   (i) a silicon-based fluoride acceptor moiety which comprises a silicon atom and a fluorine atom, wherein the fluorine atom is linked via a covalent bond directly to the silicon atom, and which moiety can be labeled with ¹⁸F by isotopic exchange of ¹⁹F by ¹⁸F or which is labeled with ¹⁸F;
   (ii) a silicon-based fluoride acceptor moiety which comprises a silicon atom and a hydroxy group, wherein the hydroxy group is linked via a covalent bond directly to the silicon atom, and which moiety can be labeled with ¹⁸F by nucleophilic substitution of OH by ¹⁸F; and
   (iii) a silicon-based fluoride acceptor moiety which comprises a silicon atom and a hydrogen atom, wherein the hydrogen atom is linked via a covalent bond directly to the silicon atom, and which moiety can be labeled with ¹⁸F by nucleophilic substitution of H by ¹⁸F.
8. The conjugate compound in accordance with item 7, wherein the silicon-based fluoride acceptor moiety R^{SiFA}
   contains a group of the following formula (S-1): 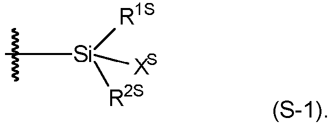 wherein
   X^{S} is F, OH or H;
   R^{1S} and R^{2S} are independently a linear or branched C₃ to C₁₀ alkyl group.
9. The conjugate compound in accordance with item 7, wherein the silicon-based fluoride acceptor moiety R^{SiFA} has a structure as represented by any of formulas (S-3) to (S-7): 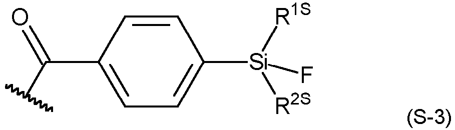 wherein
   R^{1S} and R^{2S} are independently from each other a linear or branched C3 to C10 alkyl group;. 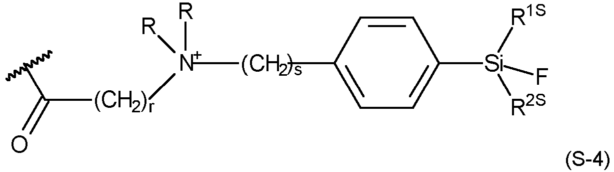
   wherein r is 1, 2 or 3, s in -(CH₂)ₛ- is an integer of 1 to 6,
   the groups R are, independently, H or C1 to C6 alkyl, and
   R^{1S} and R^{2S} are independently from each other a linear or branched C3 to C10 alkyl group; 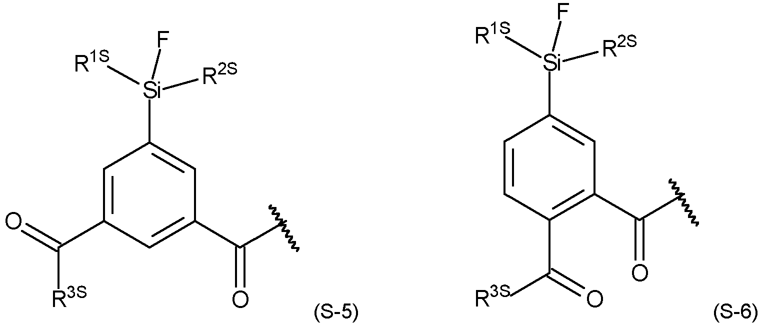 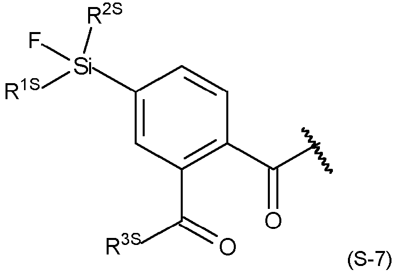
   wherein:
   R^{1S} and R^{2S} are independently a linear or branched C₃ to C₁₀ alkyl group; and
   R^{3S} is selected from
      (i) -OH or -O⁻,
      (ii) a sugar moiety or an amino sugar moiety,
      (iii) an amino acid moiety or an oligopeptide moiety,
      (iv) a PEG moiety;
   and from combinations of two or more of (ii), (iii) and (iv).
10. The conjugate of any one of items 5 to 9 that is represented by formula (III) 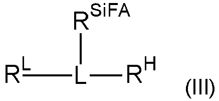 wherein:
   - R^{L}: is one or more chelating groups as defined in any one of claim 6 to 9 that may contain a chelated nonradioactive or radioactive cation;
   - L: is a linking moiety;
   - R^{H}: is the tetrapeptide of any one of claims 1 to 5, and
   - R^{SiFA}: is present or absent, and, if present, is a silicon-based fluoride acceptor moiety as defined in any one of claims 10 to 12.
11. The conjugate of item 10, wherein the linking moiety comprises D/L-diaminopropionic acid (D/L-Dap), D/L-diaminobutyric acid (D/L-Dab), D/L-ornithine (D/L-Orn) or D/L-lysine (D/L-Lys), wherein, if present, the SiFA is conjugated to D/L-Dap, D/L-Dab, D/L-Orn or D/L-Lys using the - NH₂ groups contained in these amino acids to provide a coupling group -NH- wherein the bond to one hydrogen atom in the -NH₂ group is replaced by a bond to the SiFA,
   wherein D/L-Dap, D/L-Dab, D/L-Orn or D/L-Lys are preferably placed such in the linking moiety that D/L-Dap, D/L-Dab, D/L- D/L-Orn or Lys are directly connected to R^{L} and/or R^{SiFA}.
12. The conjugate of item 10 or 11, wherein the linking moiety comprises polyethylenglycol (PEG), preferably (PEG)₃₋₁₁ optionally together with poly-L/D-hydroxyproline (L/D-Hyp), preferably (Hyp)₁₋₈, and/or
   α- or γ-glu, preferably γ-glu,
      and
   wherein the linker most preferably comprises
   y-glu-(Hyp)₆-y-glu-(PEG)₃ or y-glu-(PEG)₄-y-glu-(PEG)₃.
13. The conjugate of any one of items 5 or 9, wherein the conjugate is represented by
   DOTA-γ-glu-(PEG)₃-Trp-(N-Me)Nle-Asp-1-Nal-NH₂,
   DOTA-dap(SiFA)-γ-glu-(PEG)₇-γ-glu-(PEG)₃-Trp-(N-Me)Nle-Asp-1-Nal-NH₂,
   DOTA-dap(SiFA)-y-glu-(Hyp)₆-y-glu-(PEG)₃-Trp-(N-Me)Nle-Asp-1-Nal-NH₂
   DOTA-dap(SiFAlin)-y-glu-(PEG)₄-y-glu-(PEG)₃-Trp-(N-Me)Nle-Asp-1-Nal-NH₂,
   wherein SiFA represents a moiety of formula (S-3a) and SiFAlin represents a moiety of the formula (S-4a): 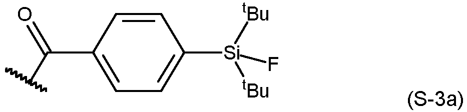 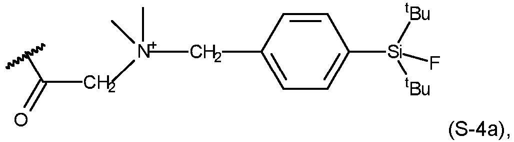 and
   wherein DOTA optionally contains a chelated nonradioactive or radioactive cation.
14. A conjugate compound binding to Cholecystokinin 2 receptor (CCK-2R) that is represented by formula (IV)

   R^{L}-dap(R^{SiFA})-R^{GABA}-L-R^{H} (IV)

   wherein:
   - R^{L}: is DOTA or DOTAGA, optionally containing a chelated nonradioactive or radioactive cation;
   - dap(R^{SiFA}): is a dap group carrying a silicon-based fluoride acceptor moiety R^{SiFA};
   - R^{GABA}: is present or absent, and, if present, is one or more γ-aminobutryic acid (GABA), preferably (GABA)₁₋₃;
   - L: is a linking moiety comprising at least 5, preferably at least 6 α- or γ-glu and most preferably 6 to 9 α- or γ-glu, whereby γ-glu is preferred over α-glu; and
   - R^{H}: is or comprises Ala-Tyr-Gly-Trp-Nle-Asp-Phe-NH_{2.}
15. The conjugate of item 14, wherein the conjugate is represented by
   DOTAGA-dap(SiFA)-(γ-glu)₆-Ala-Tyr-Gly-Trp-Nle-Asp-Phe-NH₂,
   DOTAGA-dap(SiFA)-(α-glu)₈- Ala-Tyr-Gly-Trp-Nle-Asp-Phe-NH₂,
   DOTA-dap(SiFA)-(γ-glu)₈- Ala-Tyr-Gly-Trp-Nle-Asp-Phe-NH₂,
   wherein SiFA represents a silicon-based fluoride acceptor moiety of formula (S-3a): 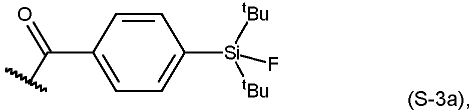 and
   wherein DOTA or DOTAGA optionally contains a chelated nonradioactive or radioactive cation.

## Claims

1. A conjugate compound binding to Cholecystokinin 2 receptor (CCK-2R) that is represented by formula (IV)
R^{L}-dap(R^{SiFA})-R^{GABA}-L-RH (IV)
wherein:
R^{L} is DOTA or DOTAGA, optionally containing a chelated nonradioactive or radioactive cation;
dap(R^{SiFA}) is a diaminopropionic acid (dap) group carrying a silicon-based fluoride acceptor moiety R^{SiFA};
R^{GABA} is present or absent, and, if present, is one or more γ-aminobutryic acid (GABA), preferably (GABA)₁₋₃;
L is a linking moiety comprising at least 5, preferably at least 6 α- or γ-glu and most preferably 6 to 9 α- or γ-glu, whereby γ-glu is preferred over α-glu; and
R^{H} is or comprises Ala-Tyr-Gly-Trp-Nle-Asp-Phe-NH₂

2. The conjugate of claim 1, wherein the radioactive or nonradioactive cation of the chelating group is selected from the cations of ⁴³Sc, ⁴⁴Sc, ⁴⁷Sc, ⁵¹Cr, ^{52m}Mn, ⁵⁵Co, ⁵⁷Co, ⁵⁸Co, ⁵²Fe, ⁵⁶Ni, ⁵⁷Ni, ⁶²Cu, ⁶⁴Cu, ⁶⁷Cu, ⁶⁶Ga, ⁶⁸Ga, ⁶⁷Ga, ⁸⁹Zr, ⁹⁰Y, ⁸⁶Y, ^{94m}Tc, ^{99m}Tc, ⁹⁷Ru, ¹⁰⁵Rh ¹⁰⁹Pd, ¹¹¹Ag,^{110m}In, ¹¹¹In, ¹¹³In, ^{114m}In, ^{117m}Sn, ¹²¹Sn, ¹²⁷Te, ¹⁴²Pr, ¹⁴³Pr, ¹⁴⁷Nd, ¹⁴⁹Gd, ¹⁴⁹Pm, ¹⁵¹Pm, ¹⁴⁹Tb, ¹⁵²Tb, ¹⁵⁵Tb, ¹⁵³Sm, ¹⁵⁶Eu, ¹⁵⁷Gd, ¹⁵⁵Tb, ¹⁶¹Tb, ¹⁶⁴Tb, ¹⁶¹Ho, ¹⁶⁶Ho, ¹⁵⁷Dy, ¹⁶⁵Dy, ¹⁶⁶Dy, ¹⁶⁰Er, ¹⁶⁵Er, ¹⁶⁹Er, ¹⁷¹Er, ¹⁶⁶Yb, ¹⁶⁹Yb, ¹⁷⁵Yb, ¹⁶⁷Tm, ¹⁷²Tm, ¹⁷⁷Lu, ¹⁸⁶Re, ^{186g}Re, ¹⁸⁸Re, ¹⁸⁸W, ¹⁹¹Pt, ^{195m}Pt, ¹⁹⁴Ir, ¹⁹⁷Hg, ¹⁹⁸Au, ¹⁹⁹Au, ²¹²Pb, ²⁰³Pb, ²¹¹At, ²¹²Bi, ²¹³Bi, ²²³Ra, ²²⁴Ra, ²²⁵Ac, ²²⁶Th and ²²⁷Th, and from cations of nonradioactive isotopes thereof, or is a cationic molecule comprising ¹⁸F or ¹⁹F, such as ¹⁸F-[AlF]²⁺, and is preferably selected from a cation of ⁶⁸Ga, ⁹⁰Y, or ¹⁷⁷Lu and from cations of nonradioactive isotopes of Ga, Y or Lu.

3. The conjugate of claim 1 or 2, wherein the silicon-based fluoride acceptor moiety R^{SiFA} selected from:
(i) a silicon-based fluoride acceptor moiety which comprises a silicon atom and a fluorine atom, wherein the fluorine atom is linked via a covalent bond directly to the silicon atom, and which moiety can be labeled with ¹⁸F by isotopic exchange of ¹⁹F by ¹⁸F or which is labeled with ¹⁸F;
(ii) a silicon-based fluoride acceptor moiety which comprises a silicon atom and a hydroxy group, wherein the hydroxy group is linked via a covalent bond directly to the silicon atom, and which moiety can be labeled with ¹⁸F by nucleophilic substitution of OH by ¹⁸F; and
(iii) a silicon-based fluoride acceptor moiety which comprises a silicon atom and a hydrogen atom, wherein the hydrogen atom is linked via a covalent bond directly to the silicon atom, and which moiety can be labeled with ¹⁸F by nucleophilic substitution of H by ¹⁸F.

4. The conjugate of claim 3, wherein the silicon-based fluoride acceptor moiety R^{SiFA} contains a group of the following formula (S-1): 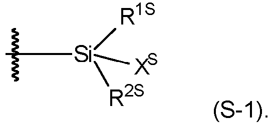 wherein
X^{S} is F, OH or H;
R^{1S} and R^{2S} are independently a linear or branched C₃ to C₁₀ alkyl group.

5. The conjugate of claim 3, wherein the silicon-based fluoride acceptor moiety R^{SiFA} has a structure as represented by any of formulas (S-3) to (S-7): 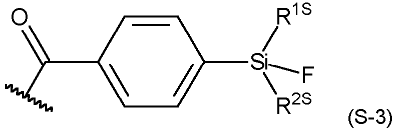 wherein
R^{1S} and R^{2S} are independently from each other a linear or branched C3 to C10 alkyl group;. 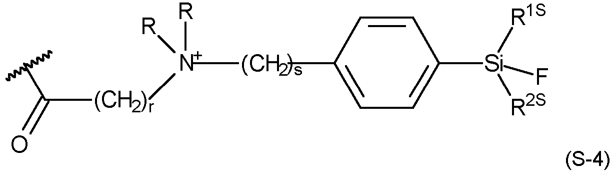
wherein r is 1, 2 or 3, s in -(CH₂)ₛ- is an integer of 1 to 6,
the groups R are, independently, H or C1 to C6 alkyl, and
R^{1S} and R^{2S} are independently from each other a linear or branched C3 to C10 alkyl group; 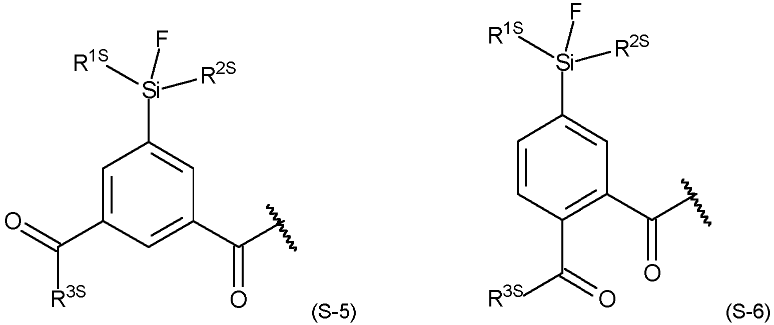 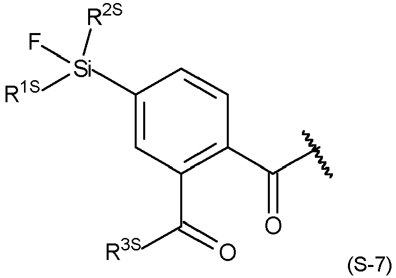 wherein:
R^{1S} and R^{2S} are independently a linear or branched C₃ to C₁₀ alkyl group; and
R^{3S} is selected from
(i) -OH or -O⁻,
(ii) a sugar moiety or an amino sugar moiety,
(iii) an amino acid moiety or an oligopeptide moiety,
(iv) a PEG moiety;
and from combinations of two or more of (ii), (iii) and (iv).

6. The conjugate of any one of claims 1 to 5, wherein the dap is D-diaminopropionic acid or L-diaminopropionic acid.

7. The conjugate of any one of claim 1 to 6, wherein the conjugate is represented by
DOTAGA-dap(SiFA)-(γ-glu)₆-Ala-Tyr-Gly-Trp-Nle-Asp-Phe-NH₂,
DOTAGA-dap(SiFA)-(α-glu)₈- Ala-Tyr-Gly-Trp-Nle-Asp-Phe-NH₂,
DOTA-dap(SiFA)-(γ-glu)₈- Ala-Tyr-Gly-Trp-Nle-Asp-Phe-NH₂,
wherein SiFA represents a silicon-based fluoride acceptor moiety of formula (S-3a): 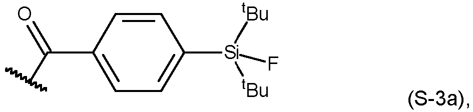 and
wherein DOTA or DOTAGA optionally contains a chelated nonradioactive or radioactive cation.

8. The conjugate of any one of claims 1 to 6, wherein the conjugate is 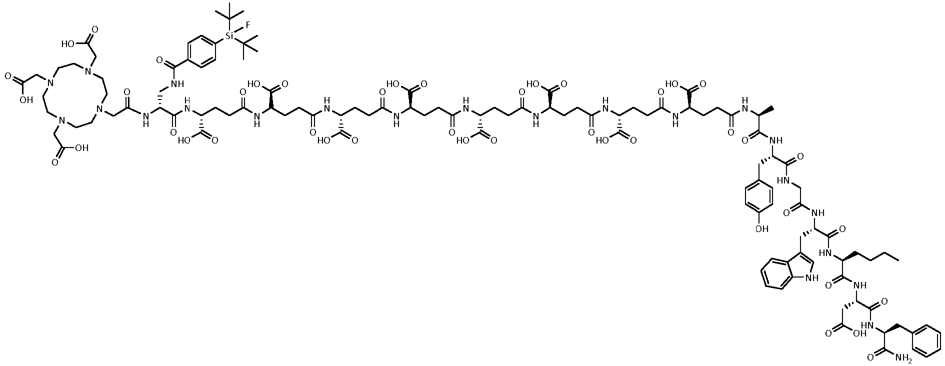 DOTA-dap(SiFA)-(γ-glu)₈-Ala-Tyr-Gly-Trp-Nle-Asp-Phe-NH₂.
